# EUROPÄISCHE PATENTSCHRIFT

(11) **EP 3 458 602 B1**
(45) Veröffentlichungstag und Bekanntmachung des Hinweises auf die Patenterteilung: **25.12.2024**
(21) Anmeldenummer: 17726566.7
(22) Anmeldetag: 15.05.2017
(51) Int. Cl.: C12Q 1/68

(54) **VERFAHREN ZUR ABSCHÄTZUNG DER PROGNOSE UND ZUR PRÄDIKTION DES ANSPRECHENS AUF EINE IMMUNTHERAPIE VON PATIENTEN MIT MALIGNEN ERKRANKUNGEN**
METHOD FOR ASSESSING A PROGNOSIS AND PREDICTING THE RESPONSE OF PATIENTS WITH MALIGNANT DISEASES TO IMMUNOTHERAPY
PROCÉDÉ POUR ÉVALUER LE PRONOSTIC ET POUR PRÉDIRE LA RÉPONSE À UNE IMMUNOTHÉRAPIE DE PATIENTS ATTEINTS DE MALADIES MALIGNES

(30) Priorität: 16.05.2016 DE 102016005947
(43) Veröffentlichungstag der Anmeldung: 27.03.2019
(73) Patentinhaber: Dietrich, Dimo, 10437 Berlin (DE)
(72) Erfinder: Dietrich, Dimo, 10437 Berlin (DE)
(74) Vertreter: Andresen, Heiko
(86) Internationale Anmeldenummer: PCT/EP2017/061612
(87) Internationale Veröffentlichungsnummer: WO 2017/198617

(56) Entgegenhaltungen:
- WO-A1-2010/086388
- WO-A1-2012/045888
- WO-A1-2015/077717
- WO-A2-2011/037936
- SONJA KLEFFEL ET AL: "Melanoma Cell-Intrinsic PD-1 Receptor Functions Promote Tumor Growth", CELL, vol. 162, no. 6, 1 September 2015 (2015-09-01), AMSTERDAM, NL, pages 1242 - 1256, XP055594329, ISSN: 0092-8674, DOI: 10.1016/j.cell.2015.08.052
- H YANG ET AL: "Expression of PD-L1, PD-L2, PD-1 and CTLA4 in myelodysplastic syndromes is enhanced by treatment with hypomethylating agents", LEUKEMIA, 25 November 2013 (2013-11-25), XP055109609, ISSN: 0887-6924, DOI: 10.1038/leu.2013.355
- VÉRONIQUE PHÉ ET AL: "Interest of methylated genes as biomarkers in urothelial cell carcinomas of the urinary tract", BJU INTERNATIONAL, vol. 104, no. 7, 1 October 2009 (2009-10-01), GB, pages 896 - 901, XP055398125, ISSN: 1464-4096, DOI: 10.1111/j.1464-410X.2009.08696.x
- DATABASE BIOSIS [online] BIOSCIENCES INFORMATION SERVICE, PHILADELPHIA, PA, US; November 2013 (2013-11-01), YANG HUI ET AL: "Expression Of Immune Checkpoints PD-L1, PD-L2, PD-1 and CTLA4 Predict For Prognosis and Resistance To Hypomethylating Agents (HMAs) In Myelodysplastic Syndromes (MDS)", XP055398012, Database accession no. PREV201400361750
- BLOOD, vol. 122, no. 21, November 2013 (2013-11-01), 55TH ANNUAL MEETING OF THE AMERICAN-SOCIETY-OF-HEMATOLOGY; NEW ORLEANS, LA, USA; DECEMBER 07 -10, 2013, pages 2767, ISSN: 0006-4971(print)
- W. ZOU ET AL: "PD-L1 (B7-H1) and PD-1 pathway blockade for cancer therapy: Mechanisms, response biomarkers, and combinations", SCIENCE TRANSLATIONAL MEDICINE, vol. 8, no. 328, 2 March 2016 (2016-03-02), pages 328rv4 - 328rv4, XP055397823, ISSN: 1946-6234, DOI: 10.1126/scitranslmed.aad7118

## Beschreibung

### Verweis auf frühere Anmeldungen

Diese Patentanmeldung beansprucht die Priorität der deutschen Patentanmeldung DE 10 2016 005 947.8, deren Offenbarungsgehalt hiermit durch Rückbezug aufgenommen wird.

### Sequenzprotokoll

Die Anmeldung beinhaltet ein elektronisches Sequenzprotokoll im txt-Format nach WIPO ST.25 Standard mit 485 Sequenzen als Teil der Beschreibung, auf dessen Inhalte hiermit vollinhaltlich Bezug genommen wird.

### Gebiet der Erfindung

Die Erfindung betrifft molekulardiagnostische Verfahren im Bereich der Onkologie, mit denen die Prognose eines Patienten mit einer malignen Erkrankung abgeschätzt beziehungsweise das Ansprechen eines Patienten mit einer malignen Erkrankung auf eine Immuntherapie vorhergesagt werden kann. Die Erfindung betrifft weiterhin molekulardiagnostische Verfahren, welche eine individualisierte Auswahl eines geeigneten Wirkstoffs zur immuntherapeutischen Behandlung dieser Patienten ermöglichen. Entsprechend betrifft die Erfindung auch die Verwendung solcher Verfahren zur Prognosebestimmung, Prädiktion oder individualisierten Wirkstoffauswahl sowie prognostische und/oder prädiktive Biomarker beziehungsweise Biomarker zur individualisierten Auswahl eines Wirkstoffs. Ferner betrifft die Erfindung Kits zur Durchführung der angegebenen Verfahren beziehungsweise für die angegebenen Verwendungen. Insbesondere beschränkt sich die Erfindung auf *in vitro* Verfahren und Verwendungen.

### Hintergrund der Erfindung

Die richtige Wahl der Behandlung eines Patienten ist ein zentrales Anliegen in der modernen Medizin. Für die Wahl der richtigen Behandlung ist zum einen eine möglichst präzise Abschätzung des wahrscheinlichsten Krankheitsverlaufs des Patienten unabhängig von einer potenziellen Therapie notwendig. Auf der Grundlage dieser Prognose kann der Kliniker entscheiden, ob beispielsweise bei einem voraussichtlich günstigen Verlauf eine eher konservative Behandlung oder bei einem voraussichtlich ungünstigen Verlauf eine eher radikale Behandlung vorgenommen werden sollte. Eine zuverlässige Abschätzung des Erkrankungsverlaufs würde demnach eine Optimierung der Therapie ermöglichen. Insbesondere könnten die Patienten von einer zielgerichteteren Therapie profitieren.

Die Abschätzung der Prognose eines Patienten mit einer malignen Erkrankung (Krebserkrankung) ist momentan nur unzureichend möglich. Üblicherweise wird die Prognose des Patienten anhand des Fortschreitungsgrads der malignen Erkrankung abgeschätzt. Hierzu werden Parameter wie zum Beispiel das Vorliegen von Metastasen, die Größe des Tumors und die Infiltration in Lymph- und Blutgefäße berücksichtigt. Diese Verfahren zu Feststellung des Tumorstadiums sind für jede Tumorentität unterschiedlich. Für einige wenige Tumoren gibt es dazu weitere prognostische Faktoren, wie zum Beispiel die Bestimmung des Wachstumsmusters nach Gleason bei Patienten mit Prostatakrebs. Insgesamt ist diese Bestimmung der Prognose von Patienten mit malignen Erkrankungen nur unzureichend zuverlässig und kaum standardisierbar.

Prognostische Biomarker haben das Potenzial, dieses Problem zu lösen. Aus US 2014/0011702 A1 ist bekannt, dass eine Methylierungsanalyse der Gene *DGKI, MGMT* und *SDPR* zur Prognose beziehungsweise Prädiktion bei Glioblastom-Patienten verwendet werden kann. US 2006/0121467 A1 beschreibt ein Verfahren, welches die Methylierung der Gene *STMN1, SFN, S100A2, TGFBR2, TP53, PTGS2, FGFR1, SYK, PITX2, GRIN2D, PSA, CGA, CYP2D6, MSMB, COX7A2L, VTN, PRKCD, ONECUT2, WBP11, DAG1, ERBB2, TFF1, TMEFF2, ESR1, RASSF1, PITX2, PSAT1* und *PCAF* für die Prädiktion des Therapieansprechens auf eine Hormontherapie bei Brustkrebs heranzieht. Aus US 2015/0051084 A1 ist bekannt, dass die Methylierung des Gens *HSPB1* bei der Bestimmung der Prognose von Prostatakrebspatienten herangezogen wird. In WO 2011/051414 A1 ist eine Verwendung der Methylierung der Gene *CLK3, MTMR4, NFE2L1, PERLD1, AKAP2, ANAPC1, ANKRD47, ATF7, ATP5G1, BCL2, C9orf3, COIL, CSF2, DMP1, E2F3, ELMO2, ERBB2, GABRG2, GPC5, HSPA6, KISS1, MARCH3, KIAA0100, MRO, MSX1, NCOA6, PAPOLA, PDGFRA, RPL23A, RUFY3, SOX3, TP53, TSPYL1, UBXD3* und *ZNF420* zur Prognose und Prädiktion des Ansprechens auf eine Platin-Therapie bei Patientinnen im Eierstockkrebs beschrieben.

Kleffel et al. (Cell 2015, 162, 1242-1256) zeigen, dass die PD-1:PD-L1-Achse in Melanomzellen für das Tumorwachstum verantwortlich ist und dass die Blockierung von PD-1 im Melanom zur klinischen Wirksamkeit der anti-PD-1 Therapie beitragen könnte.

Zum anderen ist eine verlässliche Vorhersage des Ansprechens eines Patienten auf eine Immuntherapie von hohem klinischen und wirtschaftlichen Nutzen. Einen Durchbruch in der Onkologie stellen aktuell Immuntherapien mit sogenannten Immun-Checkpoint-Inhibitoren dar, welche auch bei fortgeschrittenen Tumorerkrankungen herausragende Ergebnisse zeigen. Allerdings spricht nur ein relativ kleiner Anteil der Patienten auf diese Therapien an. Ein prädiktiver Biomarker, welcher das Ansprechen auf diese Therapien vorhersagen könnte, wäre demnach von besonderem klinischen Wert. Gegenwärtig sind immunhistologische Verfahren mit Antikörpern in der Erprobung, welche anhand eines Gewebeschnitts das Vorhandensein der entsprechenden Immun-Checkpoint-Proteine anzeigen sollten, um auf diese Weise das Therapieansprechen vorherzusagen. Allerdings zeigen diese Tests nur eine mäßige Zuverlässigkeit.

Nach wie vor wird die Therapie von Patienten mit malignen Erkrankungen in vielen Fällen nicht optimal gewählt, da sich der Verlauf der Erkrankung bei individuellen Patienten oft nur ungenau abschätzen lässt und dem Kliniker daher keine ausreichenden Anhaltspunkte für eine individuelle Auswahl oder Anpassung einer Therapie zur Verfügung stehen. Es fehlt demnach an robusten und wirtschaftlichen prognostischen und prädiktiven Verfahren auf der Grundlage von objektiv messbaren Parametern, mit denen eine akkurate Abschätzung des Krankheitsverlaufs und eine zuverlässige Vorhersage des Ansprechens auf eine Therapie von Patienten mit unterschiedlichen malignen Erkrankungen möglich ist.

### Zusammenfassung der Erfindung

Vor diesem Hintergrund stellt die vorliegende Erfindung in einem ersten Aspekt ein Verfahren zur zur Prädiktion des Ansprechens eines Patienten mit einer malignen Erkrankung auf eine Immuntherapie bereit, wobei die maligne Erkrankung eine Krebserkrankung ist. Das Verfahren ist dadurch gekennzeichnet, dass eine DNA-Methylierungsanalyse mindestens eines immunregulatorischen Gens von Zellen der malignen Erkrankung und/oder von mit den Zellen der malignen Erkrankung wechselwirkenden T-Lymphozyten durchgeführt wird, wobei das immunregulatorische Gen für einen Immun-Checkpoint ausgewählt aus B7-Proteinen und deren Rezeptoren codiert und ausgewählt ist aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2,* sowie beliebigen Kombinationen davon, wobei die Immuntherapie mindestens einen Wirkstoff umfasst, welcher ein Immun-Checkpoint-Inhibitor ist, der dazu ausgelegt ist, die immunregulatorische Wirkung des Immun-Checkpoints zu verändern, und das Ergebnis der DNA-Methylierungsanalyse mit einem Referenzwert verglichen wird, um das Ansprechen des Patienten auf die Immuntherapie zu bestimmen (Prädiktion).

Als Referenz wird gemäß einem zweiten Aspekt der Erfindung ein Verfahren zur individualisierten Auswahl eines Wirkstoffs zur immuntherapeutischen Behandlung eines Patienten mit einer malignen Erkrankung offenbart, wobei wiederum eine DNA-Methylierungsanalyse mindestens eines immunregulatorischen Gens von Zellen der malignen Erkrankung und/oder von mit den Zellen der malignen Erkrankung wechselwirkenden T-Lymphozyten durchgeführt und anhand des Ergebnisses der Wirkstoff ausgewählt wird.

Ein dritter Erfindungsaspekt betrifft die Verwendung einer DNA-Methylierungsanalyse mindestens eines immunregulatorischen Gens von Zellen einer malignen Erkrankung eines Patienten und/oder von mit den Zellen der malignen Erkrankung wechselwirkenden T-Lymphozyten, wobei die maligne Erkrankung eine Krebserkrankung ist, wobei das immunregulatorische Gen für einen Immun-Checkpoint ausgewählt aus B7-Proteinen und deren Rezeptoren codiert und ausgewählt ist aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2,* sowie beliebigen Kombinationen davon, zur Prädiktion des Ansprechens des Patienten auf eine Immuntherapie und/oder individualisierten Auswahl eines Wirkstoffs zur immuntherapeutischen Behandlung des Patienten, wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern, wobei der Wirkstoff ausgewählt wird, wenn die DNA-Methylierungsanalyse eine Expression des Immun-Checkpoints in den Zellen der malignen Erkrankung und/oder in den T-Lymphozyten anzeigt.

Ein vierter Aspekt der Erfindung betrifft die Verwendung von Anwesenheit, Abwesenheit oder Ausmaß einer Methylierung von mindestens einem CpG-Dinukleotid eines immunregulatorischen Gens von Zellen einer malignen Erkrankung und/oder von mit den Zellen der malignen Erkrankung wechselwirkenden T-Lymphozyten, wobei die maligne Erkrankung eine Krebserkrankung ist, wobei das immunregulatorische Gen für einen Immun-Checkpoint ausgewählt aus B7-Proteinen und deren Rezeptoren codiert und ausgewählt ist aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2,* sowie beliebigen Kombinationen davon, als prädiktiver Biomarker zur Vorhersage des Ansprechens der malignen Erkrankung auf eine Immuntherapie und/oder als Biomarker zur individualisierten Auswahl eines Wirkstoffs zur immuntherapeutischen Behandlung eines Patienten mit der malignen Erkrankung, wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern, wobei der Wirkstoff ausgewählt wird, wenn die Methylierung des mindestens einen CpG-Dinukleotids eine Expression des Immun-Checkpoints in den Zellen der malignen Erkrankung und/oder in den T-Lymphozyten anzeigt.

Letzlich stellt die Erfindung in einem fünften Aspekt eine Verwendung eines Kits zur Durchführung des Verfahrens des vorgenannten ersten Aspektes bereit. Das Kit enthältmindestens ein Oligonukleotid-Paar für die DNA-Methylierungsanalyse, wobei das Oligonukleotid-Paar dazu ausgelegt ist, an eine Sequenz des immunregulatorischen Gens in DNA von den Zellen der malignen Erkrankung und/oder von den T-Lymphozyten zu hybridisieren, nachdem in der DNA enthaltene Cytosine in Uracil oder eine andere Base mit einem von Cytosin unterscheidbaren Basenpaarungsverhalten und/oder Molekulargewicht umgewandelt wurden, um die Sequenz zu amplifizieren und/oder zu detektieren.

Bevorzugte Varianten dieser Aspekte ergeben sich aus der Beschreibung und den abhängigen Ansprüche.

### Definitionen und allgemeine Erläuterungen

In dieser Beschreibung werden verschiedene Dokumente zitiert, um einen allgemeinen technischen Hintergrund in Bezug auf die vorliegende Erfindung zu vermitteln. Auf die Offenbarung und Lehre dieser Dokumente wird in Ergänzung nachfolgender Beschreibung vollinhaltlich Bezug genommen, um Wiederholungen zu vermeiden.

Die nachfolgenden Definitionen und allgemeinen Erläuterungen sollen den fachkundigen Leser beim Verständnis, in der Auslegung und bei der Ausübung der vorliegenden Erfindung anleiten und unterstützen. Vorbehaltlich anderer Angaben sollen alle technischen und wissenschaftlichen Begriffe diejenige Bedeutung haben, welche dem üblichen Begriffsverständnis eines Durchschnittsfachmanns auf dem Gebiet der vorliegenden Erfindung entspricht.

Die verschiedenen Aspekte und Varianten der Erfindung involvieren Techniken und Methoden aus der molekularbiologischen Routinepraxis. Insbesondere gehört die DNA-Methylierungsanalyse zur Bestimmung der Methylierung eines CpG-Dinukleotids zu den Fachkenntnissen eines Molekularbiologen oder -genetikers. Zweckdienliche Laborhandbücher für diese Techniken und Methoden stehen dem Fachmann ohne Weiteres zur Verfügung, beispielsweise "Molecular Cloning, A Laboratory Manual" von M.R. Green und J. Sambrook, 4th Edition, 2012, Cold Spring Harbor Laboratory Press.

So, wie sie hier verwendet werden, schließen unbestimmte Artikel wie "ein" oder "eine" die Möglichkeit mit ein, dass auch zwei oder mehrere dieser Merkmale vorhanden sein können.

So, wie der Begriff hier verwendet wird, umfassen "maligne Erkrankungen" oder auch "bösartige Erkrankungen" solche Erkrankungen, die durch einen Krankheitsverlauf gekennzeichnet sind, der fortschreitend zerstörerisch ist und möglicherweise auch zum Tod des Patienten führen kann. Maligne Erkrankungen umfassen maligne Gewebeneubildungen, zum Beispiel Neoplasien oder Tumoren, wobei die Malignität durch unkontrolliertes, raumforderndes, verdrängendes, infiltratives und/oder invasives Wachstum gekennzeichnet sein kann. Maligne Tumoren sind in der Regel in der Lage, Tochtergeschwülste (Metastasen) zu bilden. Maligne Tumoren sind zum Beispiel Karzinome, Sarkome, Melanome, Gliome, Blastome, Seminome und Teratome. Maligne Erkrankungen umfassen auch hämatologische maligne Erkrankungen, das heißt bösartige Erkrankungen, die das Blutsystem oder das blutbildende System betreffen, beispielsweise Leukämien, Lymphome, myeloproliferative Erkrankungen und myelodysplastische Syndrome. Leukämien umfassen eine Gruppe von malignen Erkrankungen, bei denen unreife hämatopoetische Zellen sich bösartig verändert haben, sich übermäßig stark vermehren und zu einer Anhäufung von Zellen im peripheren Blut führen. Lymphome umfassen Erkrankungen, bei denen Zellen des lymphatischen Systems entartet sind. Myeloproliferative Erkrankungen umfassen eine Gruppe von Erkrankungen, bei denen eine oder mehrere blutbildende Zellreihen stark vermehrt auftreten. Myelodysplastische Syndromen umfassen eine klonale Expansion von Vorläuferzellen aller blutbildenden Zellreihen, wobei eine chronisch verlaufende Differenzierungsstörung der blutbildenden Stammzellen zugrunde liegt.

So, wie der Begriff hier verwendet wird, bezeichnet "Prognose" die Aussage über den Gesundheitszustand eines Patienten mit einer malignen Erkrankung oder die Veränderung des Gesundheitszustands des Patienten in der Zukunft. Dies kann sowohl den Gesundheitszustand von Patienten in Abwesenheit einer therapeutischen Maßnahme umfassen, als auch den Gesundheitszustand von Patienten, welche bereits eine Therapie erhalten oder erhalten haben. Die Prognose im Sinne der vorliegenden Erfindung umfasst auch die Aussage über den Gesundheitszustand oder die Veränderung des Gesundheitszustands, wenn der Patient in der Zukunft eine Therapie erhält. Die Therapie kann jeweils palliativ, kurativ, neoadjuvant oder adjuvant sein. Insbesondere umfasst "Prognose" die Aussage über den Eintritt eines oder mehrerer der verschiedenen Gesundheitzustände: Tod, Leben, Auftreten eines Rezidivs, Auftreten von Lymphknotenmetastasen, Auftreten von Fernmetastasen, Progression (Fortschreiten) der malignen Erkrankung, Regression (Zurückbildung) der malignen Erkrankung, Unveränderung der malignen Erkrankung, Anstieg oder Abnahme eines Parameters, der spezifisch für die maligne Erkrankung ist.

Als "Prädiktion" wird hier eine Vorhersage des Ansprechverhaltens einer malignen Erkrankung auf eine bestimmte Therapie verstanden. Das Ansprechen auf eine Therapie kann dadurch gekennzeichnet sein, dass das Ausmaß der malignen Erkrankung bei Anwendung der Therapie abnehmend, gleichbleibend, oder verlangsamt zunehmend ist. Das Ausbleiben des Ansprechens kann dadurch gekennzeichnet sein, dass das Ausmaß der malignen Erkrankung gleichbleibend, zunehmend, oder beschleunigt zunehmend ist. Als Vergleich kann jeweils das Ausmaß der malignen Erkrankung vor Anwendung der Therapie dienen oder ein Patient, welcher die Therapie nicht erhält. Das Ausmaß der Erkrankung kann durch die Anzahl der malignen Zellen beziehungsweise die Größe des malignen Tumors charakterisiert sein. Insbesondere kann ein Ansprechen auf eine Therapie durch eine Verzögerung des Eintritts des Todes, des Auftretens eines Rezidivs, des Auftretens von Lymphknotenmetastasen, des Auftretens von Fernmetastasen, der Progression der malignen Erkrankung, und/oder des Anstiegs eines sonstigen Parameters, welcher spezifisch für die maligne Erkrankung ist, gekennzeichnet sein.

Insbesondere bezeichnen Prognose und Prädiktion deduktive Schritte in Zusammenhang mit einem vorausgehenden *in vitro* Verfahren, sodass kein erfindungswesentlicher technischer Schritt am menschlichen oder tierischen Körper stattfindet.

Als "Gen" wird hier ein Abschnitt auf der DNA bezeichnet, der regulatorische, transkribierte und/oder funktionelle Sequenzregionen umfasst und somit die Grundinformationen zur Herstellung einer biologisch aktiven RNA enthält. Insbesondere umfasst ein Gen diejenigen Elemente, wie beispielsweise Promotor, Transkriptionsfaktor-Bindestellen, CpG-Inseln, offenes Chromatin, Enhancer und Silencer, CTCF-Bindestellen, welche bei der Transkription des Gens eine regulatorische Funktion erfüllen.

Die Nomenklatur für die Bezeichnung von Genen und deren Nukleotiden erfolgt entsprechend der Empfehlung des "Human Genome Organisation Gene Nomenclature Committee" (HGNC) mit Stand vom 30. April 2016. Ein Genstamm wird beispielsweise mit kursiven lateinischen Großbuchstaben bezeichnet (z.B. *PDCD1, CD274*)*.*

Die hier beschriebenen Gene sind über die "GenBank" des National Institute of Health, USA, mit Stand vom 30. April 2016 öffentlich verfügbar (Benson D.A. et al., Nucleic Acids Research, 2013, 41, D36-42).

Wenn in der nachfolgenden Beschreibung Bezug auf bestimmte DNA-Sequenzen (SEQ ID NOs) genommen wird, schließt dies immer auch Sequenzvarianten mit mindestens 95%, mindestens 96%, mindestens 97%, mindestens 98% oder mindestens 99% Sequenzidentität mit der genannten DNA-Sequenz mit ein. Die Sequenzidentität von zwei Nukleinsäuresequenzen kann zum Beispiel mit dem Algorithmus ClustalW bestimmt werden (Thompson et al., Nucleic Acids Research, 1994, 22, 4673-4680) .

Ein "CpG-Dinukleotid" ist ein DNA-Motiv, welches in allgemein gültiger Leserichtung von 5' nach 3' die Nukleosidfolge Cytidin-Phosphat-Guanosin aufweist. Guanosin besteht aus der Nukleinbase Guanin und dem Zucker β-D-Ribose. Cytidin besteht aus der Nukleinbase Cytosin und dem Zucker β-D-Ribose.

Unter "DNA-Methylierung" versteht man die biochemische oder chemische Kopplung von Methylgruppen an bestimmte Nukleotide der DNA. Im Rahmen dieser Erfindung bezieht sich "DNA-Methylierung" auf das Vorliegen einer Methylgruppe am fünften Kohlenstoffatom eines Cytosins (5-Methylcytosin), welches sich im CpG-Dinukleotidkontext befindet.

Eine "DNA-Methylierungsanalyse" im Sinne der vorliegenden Erfindung umfasst demnach die Bestimmung des Methylierungszustands eines CpG-Dinukleotids oder mehrerer CpG-Dinukleotide aus einem bestimmten Sequenzkontext. In verschiedenen Varianten der Erfindung wird unter "DNA-Methylierungsanalyse" die Feststellung verstanden, ob das Cytosin in dem oder den CpG-Dinukleotid(en) methyliert vorliegt. Die DNA-Methylierungsanalyse kann eine einzelne Kopie des CpG-Dinukleotids umfassen. Die DNA-Methylierungsanalyse kann auch eine Vielzahl von Kopien des CpG-Dinukleotids umfassen, beispielsweise wenn die DNA einer Vielzahl von Zellen vorliegt. In diesem Fall kann die DNA-Methylierungsanalyse einen Methylierungsstatus beziehungsweise einen Methylierungswert des CpG-Dinukleotids liefern, das heißt einen Querschnittswert, welcher das Ausmaß der Methylierung in der Vielzahl von Kopien des CpG-Dinukleotids ausdrückt.

Ein "Immun-Checkpoint" ist ein Protein, welches die Immunantwort moduliert, also entweder immunsupprimmierend beziehungsweise anti-inflammatorisch (entzündungshemmend) oder immunstimulierend beziehungsweise pro-inflammatorisch (entzündungsfördernd) ist. Immun-Checkpoints dienen der Überwachung der korrekten Funktion der Immunantwort, indem beispielsweise die Stärke und Intensität einer antigenspezifischen T-Zell-Aktivierung oder T-Zell-Effektorfunktion ko-stimulatorisch (positiv regulierend) oder ko-inhibitorisch (negativ regulierend) geregelt wird. Bei manchen malignen Erkrankungen sind zum Beispiel anti-inflammatorische beziehungsweise ko-inhibitorische Immun-Checkpoints im Zuge einer Immunevasion hochreguliert.

Im Sinne der vorliegenden Erfindung umfasst "mit den Zellen der malignen Erkrankung wechselwirkende T-Lymphozyten" solche T-Lymphozyten, die mit Zellen der malignen Erkrankung über eine Liganden-Rezeptor-Bindung spezifisch in Kontakt stehen beziehungsweise in Kontakt treten können. Der Ligand kann sich auf der Oberfläche von Zellen der malignen Erkrankung befinden, zum Beispiel ein MHC:Peptid-Komplex beziehungsweise ein MHC II:Antigen-Komplex. Der Rezeptor kann sich auf der Oberfläche der T-Lymphozyten befinden, zum Beispiel ein T-Zell-Rezeptor (TCR). Alternativ kann sich der Ligand auf der Oberfläche der T-Lymphozyten und der Rezeptor auf der Oberfläche von Zellen der malignen Erkrankung befinden. "Mit den Zellen der malignen Erkrankung wechselwirkende T-Lymphozyten" umfasst demnach auch solche T-Lymphozyten, die durch den Kontakt mit antigenpräsentierenden Zellen in die Lage versetzt (aktiviert) wurden, dass sie spezifisch mit Zellen der malignen Erkrankung über eine der vorgenannten Liganden-Rezeptor-Bindungen wechselwirken können, ohne selbst zuvor mit dem entsprechenden Antigen in Kontakt gekommen zu sein. Eine antigenpräsentierende Zelle kann beispielsweise eine dendritische Zelle oder eine Makrophage sein. Die Aktivierung erfolgt beispielsweise über eine Liganden-Rezeptor-Bindung zwischen T-Lymphozyten und einer antigenpräsentierenden Zelle, welche ein aus einer Zelle der malignen Erkrankung stammendes Antigen präsentiert. Der Ligand, zum Beispiel ein MHC II:Antigen-Komplex, befindet sich auf der Oberfläche der antigenpräsentierenden Zellen und der T-Zell-Rezeptor (TCR) befindet sich auf der Oberfläche des T-Lymphozyten. Eine weitere Form der Wechselwirkung zwischen T-Lymphozyten und Zellen der malignen Erkrankung im Sinne der Erfindung beinhaltet, dass von den Zellen der malignen Erkrankung gebildetes Adenosin durch einen Rezeptor auf der Oberfläche der T-Lymphozyten gebunden wird. Es ist auch möglich, dass das Adenosin durch T-Lymphozyten gebildet wird und sich der Rezeptor auf der Oberfläche von Zellen der malignen Erkrankung befindet.

"Immuntherapie" beziehungsweise "immuntherapeutische Behandlung" wird hier als Sammelbegriff für alle Behandlungsansätze verwendet, mit denen die Aktivität des Immunsystems beeinflusst werden soll. Eine Immuntherapie kann darauf gerichtet sein, die Wirkung des Immunsystems zu stärken oder sie abzuschwächen. In bestimmten Varianten der Erfindung umfasst eine Immuntherapie eine Behandlung mit Wirkstoffen, um die organismuseigene Immunreaktion gegen die maligne Erkrankung zu verstärken. Solche Wirkstoffe umfassen Immun-Checkpoint-Inhibitoren wie zum Beispiel monoklonale Antikörper, welche spezifisch an Immun-Checkpoints binden und auf diese Weise deren (insbesondere anti-inflammatorische) Signalgebung verhindern. Eine andere Möglichkeit sind Wirkstoffe, welche bereits die Expression der Immun-Checkpoints unterbinden, beispielsweise durch RNA-Interferenz oder CRISPR-Interferenz. Die Wirkstoffe können aber auch Immun-Checkpoint-Agonisten sein, welche die Signalgebung von (insbesondere pro-inflammatorischen) Immun-Checkpoints verstärken.

So, wie der Begriff hier verwendet wird, bedeutet "inhibieren" eines Immun-Checkpoints ein Verlangsamen, Hemmen oder Verhindern einer oder mehrerer durch den Immun-Checkpoint vermittelter Reaktionen chemischer, biologischer und/oder physikalischer Natur.

"Biomarker" sind charakteristische Indikatoren oder/und biologische Merkmale, die objektiv gemessen werden können und Rückschluss über den Status eines normalen biologischen oder eines krankhaften Prozesses in einem Organismus, beziehungsweise die Antwort eines normalen oder krankhaften Prozesses auf eine Intervention, beispielsweise eine Operation, eine Bestrahlung oder eine medikamentöse Behandlung, zulassen. Biomarker sind häufig (bio-)chemische Substanzen, wie zum Beispiel Proteine, Hormone, Metaboliten, Zucker und Nukleinsäuren, sowie Modifikationen davon.

Als "palliative Therapie" oder Palliativtherapie bezeichnet man eine medizinische Behandlung, die nicht auf die Heilung einer Erkrankung abzielt, sondern darauf, die Symptome zu lindern oder sonstige nachteilige Folgen zu reduzieren (Palliation). Sie stehen damit im Gegensatz zu den kurativen Therapien, die auf eine Heilung abzielen. Die Maßnahmen der Palliativmedizin haben oft das Ziel, bei fortschreitenden unheilbaren Erkrankungen den Verlauf zu verlangsamen und Symptome wie Übelkeit, Schmerz oder (reaktive) Depressionen zu reduzieren.

Eine "kurative Therapie" ist eine Therapie, welche auf eine vollständige Wiederherstellung der Gesundheit eines Patienten abzielt und so auch gleichzeitig eine Verschlechterung verhindert. Der Begriff wird insbesondere auch angewendet, wenn eine vollständige Heilung nicht absehbar ist (geringe prozentuale Chance).

Als "neoadjuvante Therapie" wird eine Therapie bezeichnet, die zur Reduktion der Tumormasse vor einem geplanten operativen Eingriff durchgeführt wird. Sie wird oft durchgeführt, wenn ein Tumor primär nicht operabel ist. Durch die neoadjuvante Therapie kann eine Verkleinerung des Tumors erreicht werden mit dem Erfolg, dass eine chirurgische Tumorentfernung doch noch möglich wird. Sie ist in diesem Fall häufig der einzige Weg einer kurativen Therapie eines malignen Tumors.

Als "adjuvante Therapie" bezeichnet man eine ergänzende oder unterstützende Therapiemaßnahme, die nach vollständiger Entfernung aller erkennbaren Tumoranteile angewandt wird, um mögliche, bisher aber noch nicht nachweisbare Tumorabsiedlungen (Mikrometastasen) zu bekämpfen und dadurch die langfristigen Heilungsaussichten zu verbessern.

Sowohl die vorstehende allgemeine Beschreibung als auch die nachfolgende detaillierte Beschreibung sind als Beispiel aufzufassen und sollen zur Erläuterung der beanspruchten Erfindung dienen. Weitere Vorteile und Merkmale der Erfindung sind aus der nachstehenden Beschreibung, den Zeichnungen und den Patentansprüchen ersichtlich. Auch wenn die Erfindung anhand ihrer bevorzugten Ausführungsformen erläutert wird, können viele weitere Variationen vorgenommen werden, ohne über den Umfang der vorliegenden Erfindung hinauszugehen. Daher ist es vorgesehen, dass die beiliegenden Patentansprüche Variationen und Kombinationen von Merkmalen abdecken, die im tatsächlichen Umfang der Erfindung enthalten sind, auch wenn diese nicht ausdrücklich in den Ansprüchen abgebildet sind.

### Kurze Beschreibung der Figuren

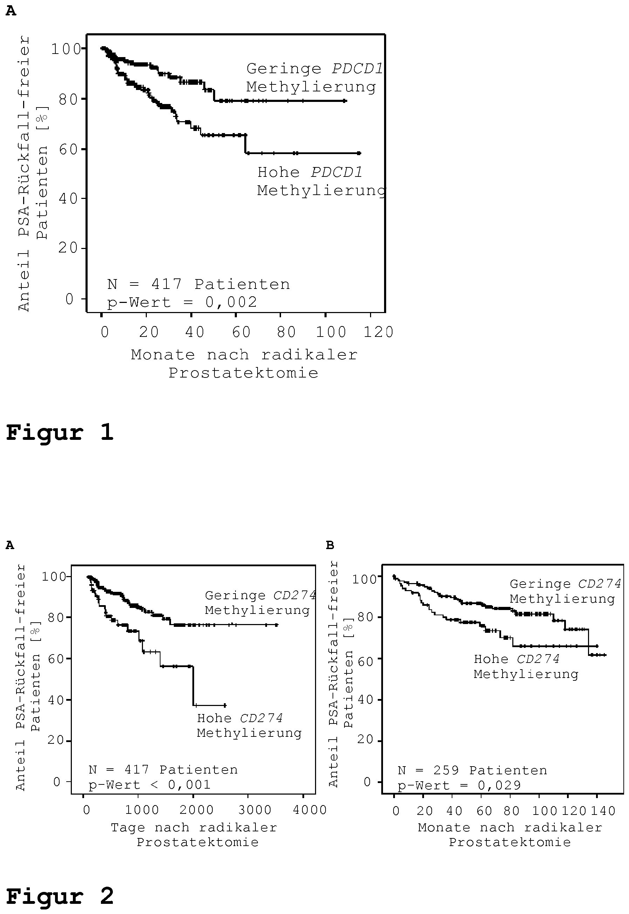

Figur 1 zeigt die Kaplan-Meier Analyse des rückfallfreien Überlebens von 417 Prostatakrebspatienten nach radikaler Prostatektomie. Die Patienten sind anhand der erfindungsgemäßen DNA-Methylierungsanalyse des *PDCD1* Genlokus im Tumor stratifiziert.
Figur 2 zeigt die Kaplan-Meier Analyse des rückfallfreien Überlebens von 417 (A) beziehungsweise einer unabhängigen Kohorte von 259 (B) Prostatakrebspatienten nach radikaler Prostatektomie. Die Patienten sind anhand der erfindungsgemäßen DNA-Methylierungsanalyse des *CD274* Genlokus im Tumor stratifiziert. Die DNA-Methylierungsanalyse erfolgte mittels der Infinium HumanMethylation450 BeadChip Technologie (A) beziehungsweise mittels quantitativer methylierungsspezifischer Echtzeit-PCR (B).

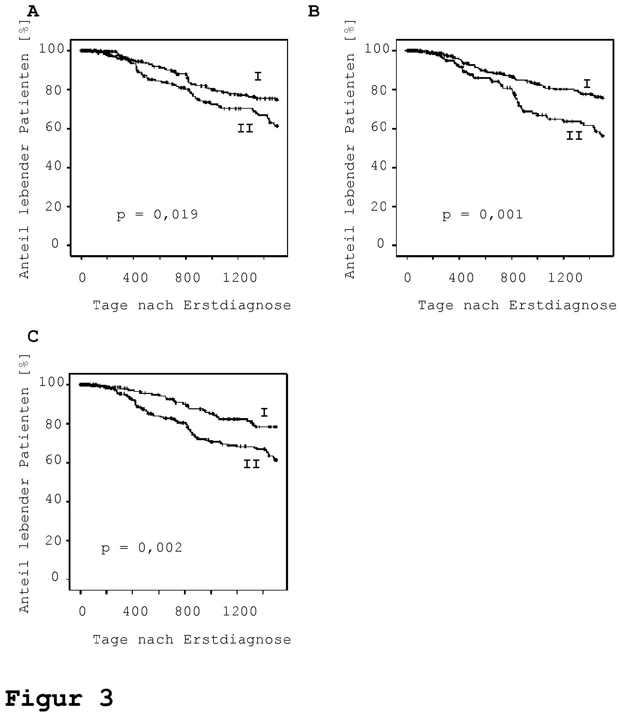

Figur 3 zeigt die Kaplan-Meier Analyse des Gesamtüberlebens von 470 Patienten mit malignen Melanomen, welche anhand der erfindungsgemäßen DNA-Methylierungsanalyse der immunregulatorischen Gene *PDCD1* (A), *CD274* (B) und *PDCD1LG2* (C) im Tumor stratifiziert wurden. Gruppe I: Patienten mit DNA-Methylierung im Tumor unterhalb des Schwellenwerts. Gruppe II: Patienten mit DNA-Methylierung im Tumor oberhalb des Schwellenwerts. Als Schwellenwerte für die Gruppierung der Patienten wurden folgende Methylierungswerte festgesetzt: 12,84% für *PDCD1LG2;* 13,11% für *CD274* und 20,54% für *PDCD1.*

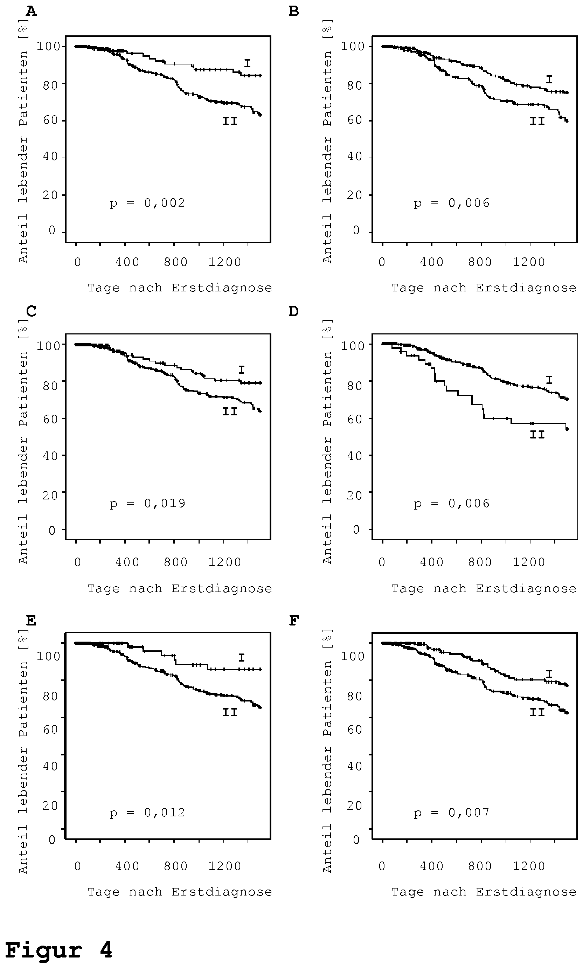

Figur 4 zeigt die Kaplan-Meier Analyse des Gesamtüberlebens von 470 Patienten mit malignen Melanomen, welche anhand der erfindungsgemäßen DNA-Methylierungsanalyse der immunregulatorischen Gene *TIGIT* (A), *TNFRSF9* (B), *LAG3* (C), *BTLA* (D), *CTLA4* (E) und *CD40* (F) stratifiziert wurden. Gruppe I: Patienten mit Methylierung im Tumor unterhalb des Schwellenwerts. Gruppe II: Patienten mit Methylierung im Tumor oberhalb des Schwellenwerts. Als Schwellenwerte für die Gruppierung der Patienten wurden folgende Methylierungswerte festgesetzt: 86,41% für *TIGIT;* 57,80% für *TNFRSF9;* 66,94% für *LAG3;* 91,31% für *BTLA;* 13,10% für *CTLA4* und 17,16% für *CD40.*

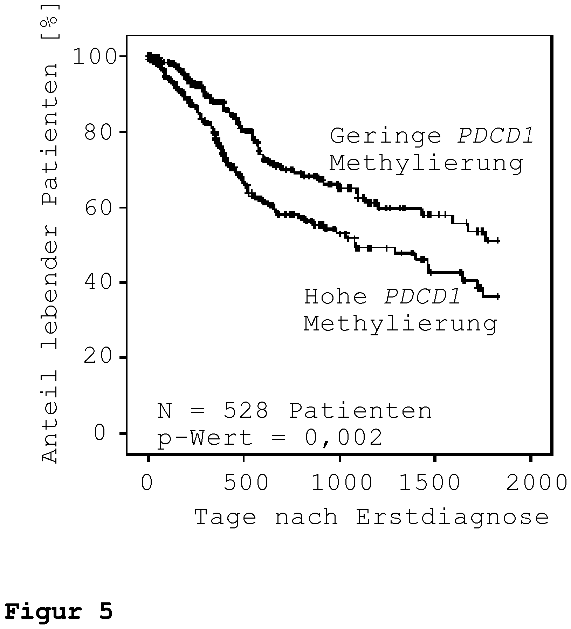

Figur 5 zeigt die Kaplan-Meier Analyse des Gesamtüberlebens von 528 Patienten mit Plattenepithelkarzinomen des Kopf- und Halsbereichs, welche anhand der erfindungsgemäßen DNA-Methylierungsanalyse des *PDCD1* Genlokus stratifiziert wurden. Die Gruppierung der Patienten erfolgte anhand des Medians der Methylierung aller Patienten, wobei einer Gruppe die Patienten mit Methylierung über dem Median und der anderen Gruppe die Patienten mit Methylierung unter dem Median zugeordnet wurden.

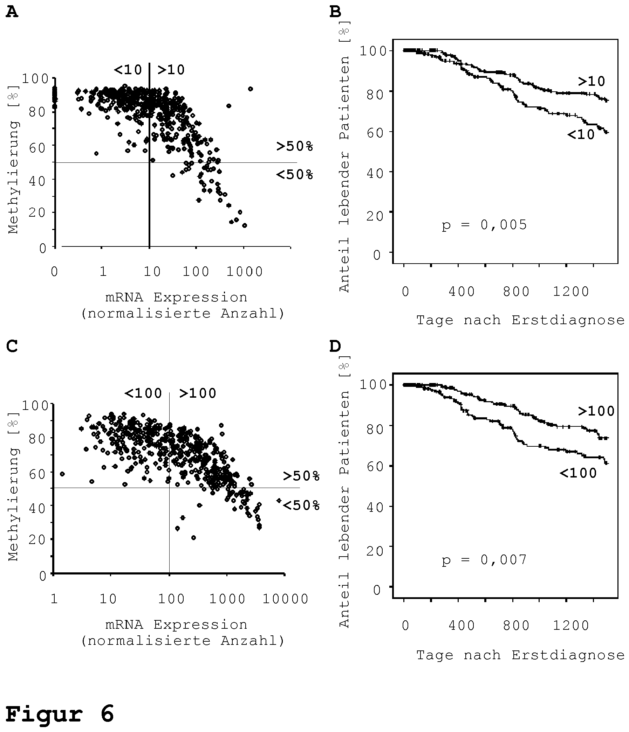

Figur 6 zeigt für die Untersuchung von 470 Patienten mit malignen Melanomen die erfindungsgemäße Korrelation von DNA-Methylierungsanalyse und mRNA-Expressionsanalyse der Gene *BTLA* (A) und *CD27* (C) sowie die Ergebnisse der Kaplan-Meier Analyse des Gesamtüberlebens der anhand der mRNA-Expression der Gene *BTLA* (B) und *CD27* (D) stratifizierten Patienten.

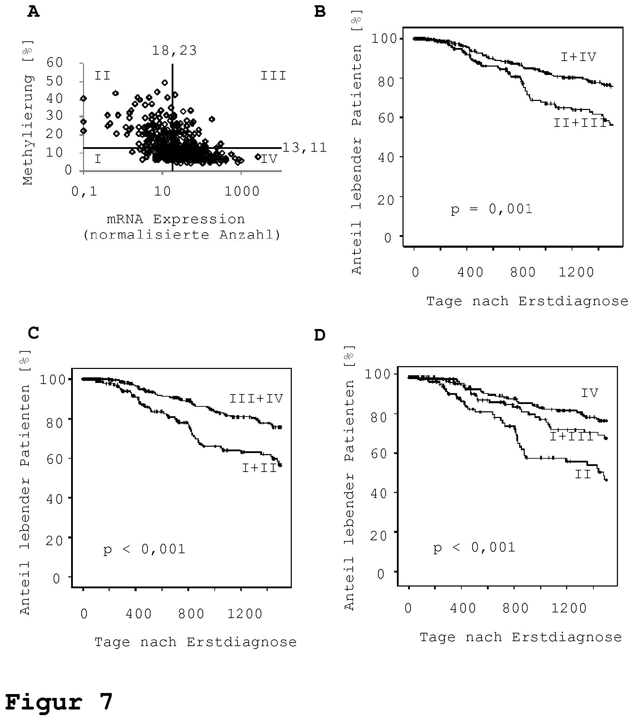

Figur 7 zeigt für die Untersuchung von 470 Patienten mit malignen Melanomen die erfindungsgemäße Korrelation von mRNA-Expressionsanalyse und DNA-Methylierungsanalyse des immunregulatorischen Gens *CD274* (A) sowie die Kaplan-Meier Analyse des Gesamtüberlebens der anhand der erfindungsgemäßen DNA-Methylierungsanalyse (B), anhand der mRNA-Expressionsanalyse (C) und anhand der Kombination aus mRNA-Expressionsanalyse und DNA-Methylierungsanalyse (D) stratifizierten Patienten.

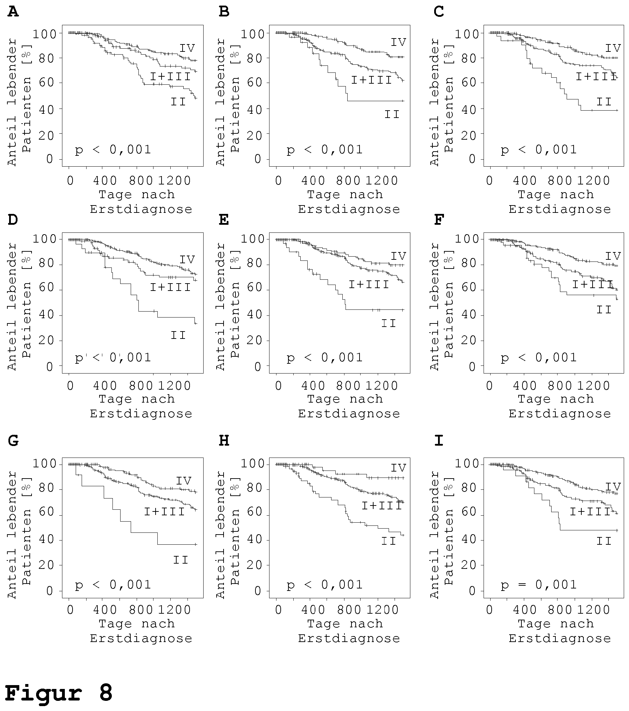

Figur 8 zeigt die Kaplan-Meier Analyse des Gesamtüberlebens von 470 Patienten mit malignen Melanomen, stratifiziert anhand der erfindungsgemäßen DNA-Methylierungsanalyse und der mRNA-Expressionsanalyse der immunregulatorischen Gene *CD274* (A) (wie in Figur 7), *PDCD1LG2* (B), *PDCD1* (C), *BTLA* (D), *LAG3* (E), *TIGIT* (F), *CD40* (G), *CTLA4* (H) und *TNFRSF9* (I). Gruppe II (Hochrisikogruppe): hohe DNA-Methylierung und niedrige mRNA-Expression; Gruppe IV (Niedrigrisikogruppe): niedrige Methylierung und hohe mRNA-Expression; Gruppe I+III (Gruppe mittleren Risikos): hohe mRNA-Expression und hohe DNA-Methylierung oder niedrige mRNA-Expression und niedrige DNA-Methylierung.

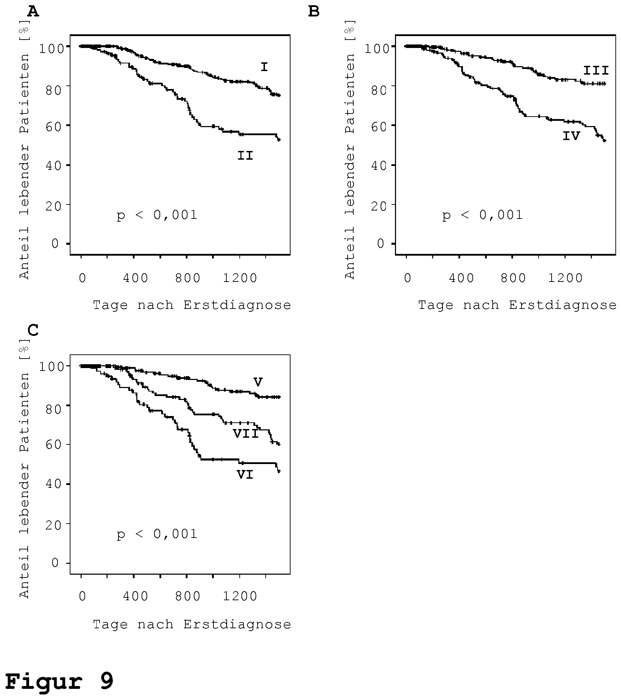

Figur 9 zeigt die Kaplan-Meier Analyse des Gesamtüberlebens von 470 Patienten mit malignen Melanomen, erfindungsgemäß stratifiziert anhand der mRNA-Expressionsanalyse (A), der DNA-Methylierungsanalyse (B) und der kombinierten mRNA-Expressionsanalyse und DNA-Methylierungsanalyse (C) der neun immunregulatorischen Gene *CD274, PDCD1, PDCD1LG2, TIGIT, TNFRSF9, LAG3, BTLA, CTLA4* und *CD40.* Gruppe I: Patienten mit erhöhter mRNA-Expression bei mindestens acht der neun Gene; Gruppe II: Patienten mit erhöhter mRNA-Expression bei weniger als acht der neun Gene; Gruppe III: Patienten mit erhöhter DNA-Methylierung bei maximal vier der neun Gene; Gruppe IV: Patienten mit erhöhter DNA-Methylierung bei mindestens fünf der neun Gene; Gruppe V: Patienten, die sowohl der Gruppe I als auch III zugehören; Gruppe VI: Patienten, die sowohl der Gruppe II und der Gruppe IV zugehörig sind; Gruppe VII: Patienten, die entweder den Gruppen I und IV oder den Gruppen II und III zugehörig sind.

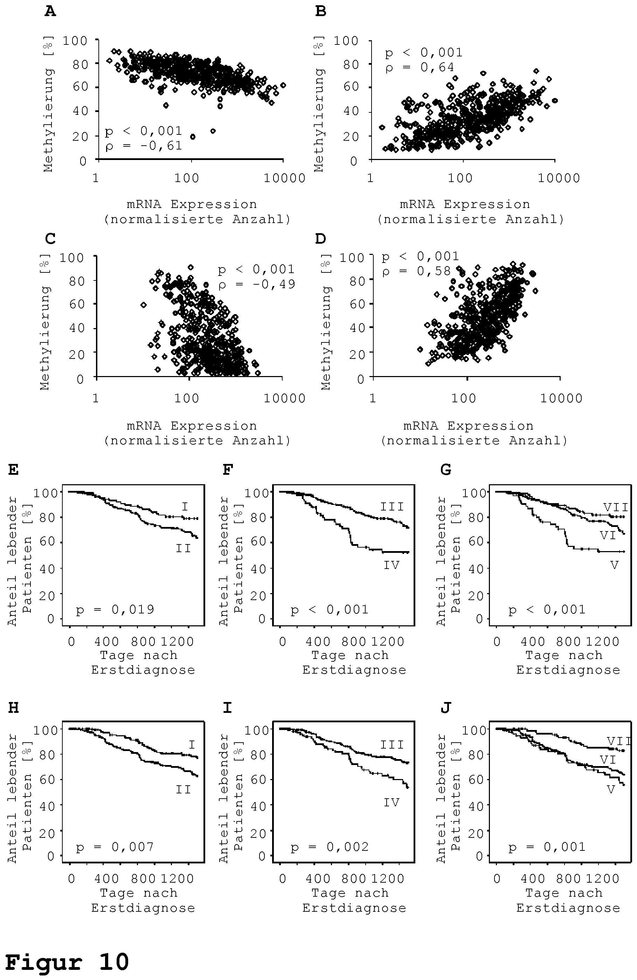

Figur 10 zeigt für das immunregulatorische Gen *LAG3* die Korrelation der mRNA-Expression mit der DNA-Methylierung des Promotorbereichs (A) und des intragenischen Bereichs (B); für das immunregulatorische Gen *CD40* die Korrelation der mRNA-Expression mit der DNA-Methylierung des Promotorbereichs (C) und des intragenischen Bereichs (D); sowie die Kaplan-Meier Analyse des Gesamtüberlebens von 470 Patienten mit malignen Melanomen, erfindungsgemäß stratifiziert anhand der DNA-Methylierungsanalyse des Promotors (E, H), des intragenischen Bereichs (F, I) und der kombinierten Analyse beider Bereiche (G, J) der immunregulatorischen Gene *LAG3* (E, F, G) und *CD40* (H, I, J). Gruppe I und IV: DNA-Methylierung unterhalb des Schwellenwerts; Gruppe II und III: DNA-Methylierung oberhalb des Schwellenwerts; Gruppe V: Patienten aus Gruppe II und IV; Gruppe VI: Patienten aus Gruppe II und III oder I und IV; Gruppe VII: Patienten aus Gruppe I und III.

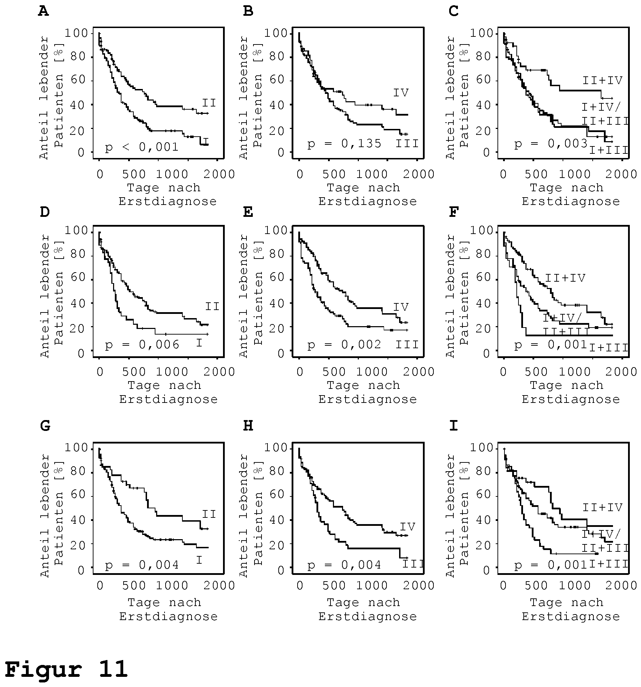

Figur 11 zeigt die Kaplan-Meier Analyse des Gesamtüberlebens von 182 Patienten mit akuter myeloischer Leukämie, wobei die Patienten erfindungsgemäß anhand der DNA-Methylierungsanalyse (A, D, G), der mRNA-Expressionsanalyse (B, E, H) und der Kombination von mRNA-Expressionsanalyse und DNA-Methylierungsanalyse (C, F, I) der immunregulatorischen Gene *CD274* (A, B, C), *PDCD1* (D, E, F) und *PDCD1LG2* (G, H, I) stratifiziert wurden. Gruppe I: DNA-Methylierung unterhalb des Schwellenwertes; Gruppe II: DNA-Methylierung oberhalb des Schwellenwertes; Gruppe III: mRNA-Expression oberhalb des Schwellenwertes; Gruppe IV: mRNA-Expression unterhalb des Schwellenwertes. Schwellenwerte: 17,00% für *CD274* DNA-Methylierung; 56,95% für *PDCD1* DNA-Methylierung; 62,82% für *PDCD1LG2* DNA-Methylierung; 4,7319 für *CD274* mRNA-Expression; 23,7933 für *PDCD1* mRNA-Expression und 12,9895 für *PDCD1LG2* mRNA-Expression.

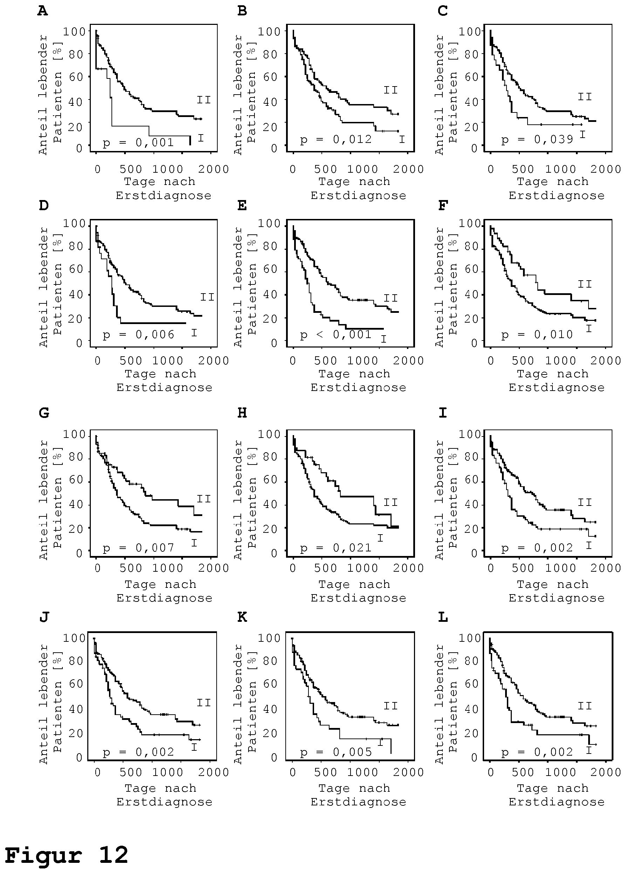

Figur 12 zeigt die Kaplan-Meier Analyse des Gesamtüberlebens von 182 Patienten mit akuter myeloischer Leukämie, wobei die Patienten erfindungsgemäß anhand der DNA-Methylierungsanalyse der immunregulatorischen Gene *TNFRSF9* (A), *TIGIT* (B), *BTLA* (C), *HAVCR2* (D), *CD80* (E), *CTLA4* (F), *ICOS* (G), *C10orf54* (H), *HHLA2* (I), *CD160* (J), *KIR2DL4* (K) und *KIR3DL1* (L) stratifiziert wurden. Die Dichotomisierung erfolgte anhand von optimierten Schwellenwerten. Gruppe I: DNA-Methylierung unterhalb des Schwellenwerts; Gruppe II: DNA-Methylierung oberhalb des Schwellenwerts.

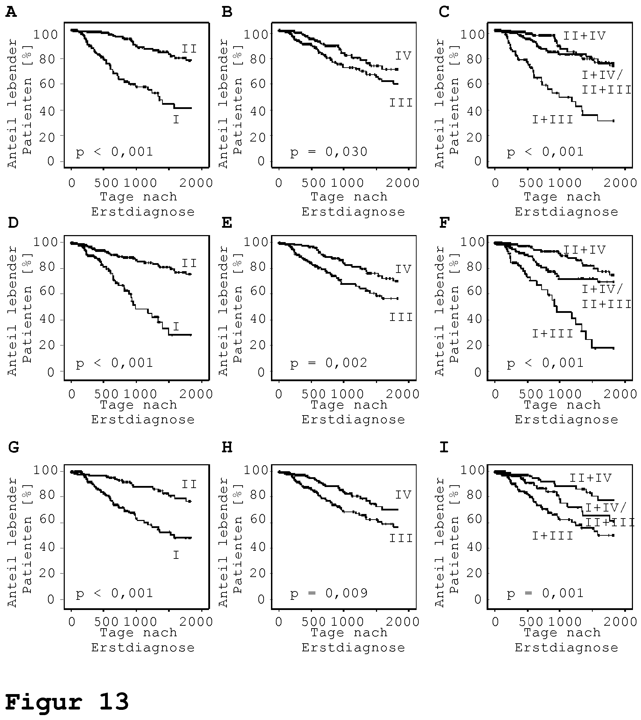

Figur 13 zeigt die Kaplan-Meier Analyse des Gesamtüberlebens von 510 Patienten mit niedriggradigen Gliomen, erfindungsgemäß stratifiziert anhand der DNA-Methylierungsanalyse (A, D, G), der mRNA-Expressionsanalyse (B, E, H) und der Kombination von DNA-Methylierungsanalyse und mRNA-Expressionsanalyse (C, F, I) der immunregulatorischen Gene *CD274* (A, B, C), *PDCD1* (D, E, F) und *PDCD1LG2* (G, H, I). Die folgenden Schwellenwerte wurden für die Dichotomisierung der DNA-Methylierung herangezogen: 32,00% für *CD274;* 33,79% für *PDCD1* und 59,89% für *PDCD1LG2.* Die Dichotomisierung der mRNA-Expression erfolgte anhand des Medians der mRNA-Expression des jeweiligen Gens aus allen Tumoren der Patientengruppe. Gruppe I: DNA-Methylierung unterhalb des Schwellenwerts; Gruppe II: DNA-Methylierung oberhalb des Schwellenwerts; Gruppe III: mRNA-Expression oberhalb des Medians; Gruppe IV: mRNA-Expression unterhalb des Medians; Gruppe I+III: Patienten aus Gruppe I und III; Gruppe II+IV: Patienten aus Gruppe II und IV; Gruppe I+IV/II+III: Patienten aus Gruppe II und III oder I und IV.

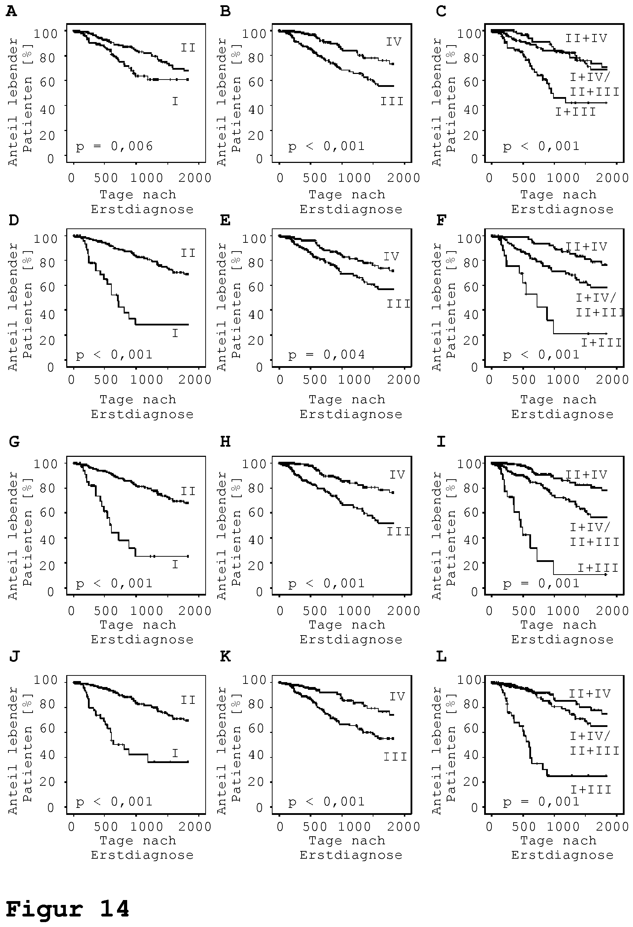

Figur 14 zeigt die Kaplan-Meier Analyse des Gesamtüberlebens von 510 Patienten mit niedriggradigen Gliomen, erfindungsgemäß stratifiziert anhand der DNA-Methylierungsanalyse (A, D, G, J), der mRNA-Expressionsanalyse (B, E, H, K) und der kombinierten DNA-Methylierungsanalyse und mRNA-Expressionsanalyse (C, F, I, L) der immunregulatorischen Gene *CD80* (A, B, C), *CTLA4* (D, E, F), *ICOS* (G, H, I), *CD276* (J, K, L). Für die Dichotomisierung anhand der DNA-Methylierung wurden folgende Schwellenwerte gewählt: 90,76% (*CD80*); 86,43% *(CTLA4);* 84,75% (*ICOS*) und 30,08% (*CD276*). Die Dichotomisierung der mRNA-Expression erfolgte anhand des Medians der mRNA-Expression des jeweiligen Gens aus allen Tumoren der Patientengruppe. Gruppe I: DNA-Methylierung unterhalb des Schwellenwerts; Gruppe II: DNA-Methylierung oberhalb des Schwellenwerts; Gruppe III: mRNA-Expression oberhalb des Medians; Gruppe IV: mRNA-Expression unterhalb des Medians; Gruppe I+III: Patienten aus Gruppe I und III; Gruppe II+IV: Patienten aus Gruppe II und IV; Gruppe I+IV/II+III: Patienten aus Gruppe II und III oder I und IV.

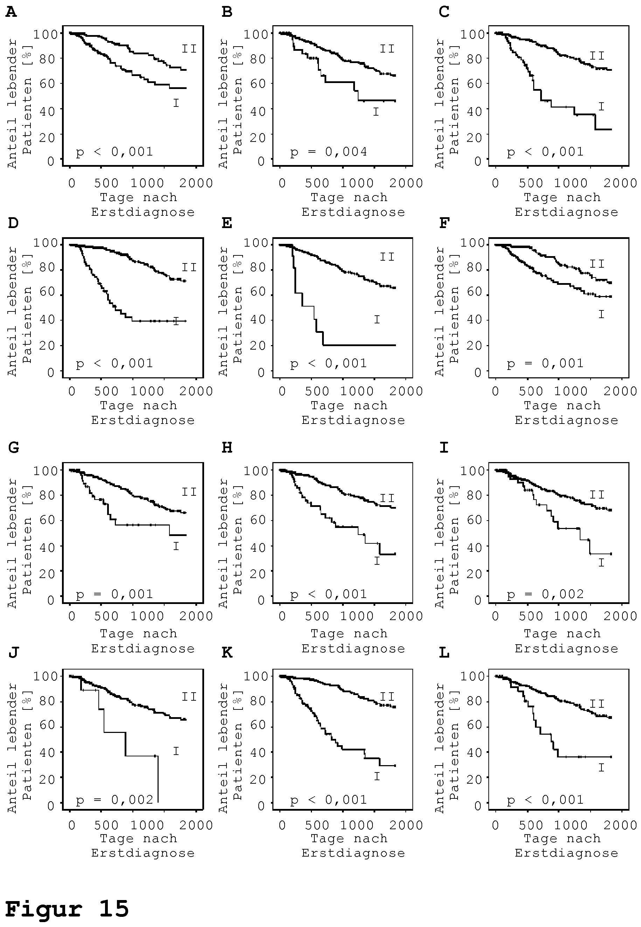

Figur 15 zeigt die Kaplan-Meier Analyse des Gesamtüberlebens von 510 Patienten mit niedriggradigen Gliomen, erfindungsgemäß stratifiziert anhand der DNA-Methylierungsanalyse der Gene *TNFRSF25* (A), *TNFRSF9* (B), *CD40* (C), *TIGIT* (D), *BTLA* (E), *HAVCR2* (F), *C10orf54* (G), *HHLA2* (H), *LAG3* (I), *CD160* (J), *KIR2DL4* (K) und *KIR3DL1* (L). Die Dichotomisierung erfolgte anhand folgender Schwellenwerte: 61,88% für *TNFRSF25;* 79,24% für *TNFRSF9;* 43,24% für *CD40;* 80,66% für *TIGIT;* 84,64% für *BTLA;* 2,495% für *HAVCR2;* 66,52% für *C10orf54;* 77,14% für *HHLA2;* 74,34% für *LAG3;* 92,89% für *CD160;* 90,85% für *KIR2DL4* und 52,28% für *KIR3DL1.* Gruppe I: Methylierung unterhalb des Schwellenwerts; Gruppe II: Methylierung oberhalb des Schwellenwerts.

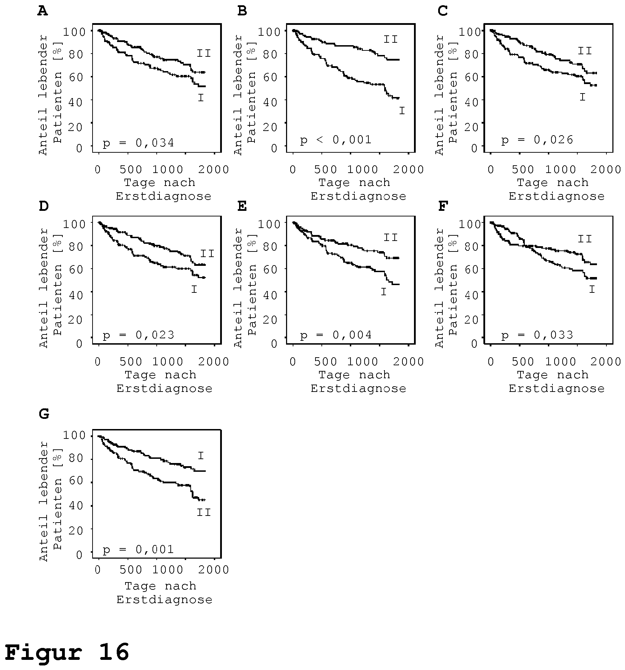

Figur 16 zeigt die Kaplan-Meier Analyse des Gesamtüberlebens von 318 Patienten mit klarzelligen Nierenzellkarzinomen, erfindungsgemäß stratifiziert anhand der DNA-Methylierungsanalyse der immunregulatorischen Gene *TIGIT* (A), *TNFRSF9* (B), *CD274* (C), *CD80* (D), *CTLA4* (E), *CD276* (F) und *HHLA2* (G). Die Dichotomisierung erfolgte anhand des Medians der DNA-Methylierung des jeweiligen Gens aus den Tumoren aller Patienten. Gruppe I: Methylierung unterhalb des Medians; Gruppe II: Methylierung oberhalb des Medians.

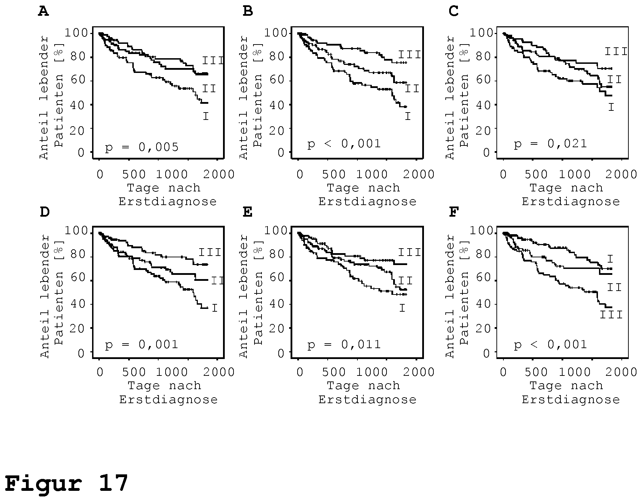

Figur 17 zeigt die Kaplan-Meier Analyse des Gesamtüberlebens von 318 Patienten mit klarzelligen Nierenzellkarzinomen, stratifiziert anhand der erfindungsgemäßen Kombination von DNA-Methylierungsanalyse und mRNA-Expressionsanalyse der immunregulatorischen Gene *TIGIT* (A), *TNFRSF9* (B), *CD80* (C), *CTLA4* (D), *CD276* (E) und *HHLA2* (F). Die Dichotomisierung erfolgte anhand des Medians der DNA-Methylierung beziehungsweise der mRNA-Expression des jeweiligen Gens aus den Tumoren aller Patienten. Gruppe I: DNA-Methylierung unterhalb und mRNA-Expression oberhalb des Medians; Gruppe III: DNA-Methylierung oberhalb und mRNA-Expression unterhalb des Medians; Gruppe II: DNA-Methylierung oberhalb und mRNA-Expression oberhalb des Medians oder DNA-Methylierung unterhalb und mRNA-Expression unterhalb des Medians.

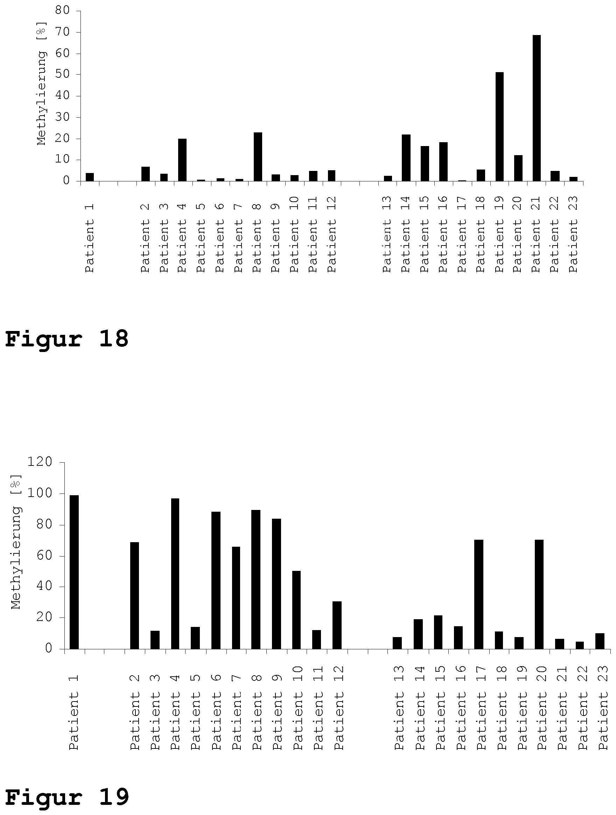

Figur 18 zeigt die erfindungsgemäße DNA-Methylierungsanalyse des immunregulatorischen Gens *CD274* zur Bestimmung des Ansprechens von Patienten auf eine Immuntherapie mit dem Wirkstoff Pembrolizumab, welcher die immunregulatorische Wirkung des durch das Gen *PDCD1* codierten Immuncheckpoints inhibiert. Gezeigt sind die Methylierungswerte (%) des Gens in Metastasen von 23 Patienten mit malignen Melanomen, die vor dem Beginn der Immuntherapie chirurgisch entfernt wurden.
Figur 19 zeigt die erfindungsgemäße DNA-Methylierungsanalyse des immunregulatorischen Gens *PDCD1* zu Bestimmung des Ansprechens von Patienten auf eine Immuntherapie mit dem Wirkstoff Pembrolizumab, welcher die immunregulatorische Wirkung des durch das Gen *PDCD1* codierten Immuncheckpoints inhibiert. Gezeigt sind die Methylierungswerte (%) des Gens in Metastasen von 23 Patienten mit malignen Melanomen, die vor dem Beginn der Immuntherapie chirurgisch entfernt wurden.

### Kurze Beschreibung der Sequenzen

SEQ ID NO:1 bis SEQ ID NO:485 wie jeweils unter der numerischen Kennzahl <213> beziehungsweise <223> des Sequenzprotokolls angegeben.

### Beschreibung der Erfindung

Der erste Aspekt der vorliegenden Erfindung betrifft ein Verfahren zur Prädiktion des Ansprechens eines Patienten mit einer malignen Erkrankung auf eine Immuntherapie, wobei die maligne Erkrankung eine Krebserkrankung ist. Dabei wird eine DNA-Methylierungsanalyse mindestens eines immunregulatorischen Gens von Zellen der malignen Erkrankung und/oder von mit den Zellen der malignen Erkrankung wechselwirkenden T-Lymphozyten durchgeführt. Das immunregulatorische Gen codiert hierin für einen Immun-Checkpoint ausgewählt aus B7-Proteinen und deren Rezeptoren, das heißt Rezeptoren, welche ein B7-Protein als Liganden binden, und ist ausgewählt aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2.* Es ist auch möglich, DNA-Methylierungsanalysen von mehreren immunregulatorischen Genen durchzuführen, welche beliebige Kombinationen dieser Immun-Checkpoints umfassen können. Vorzugsweise werden in diesem Fall DNA-Methylierungsanalysen von mehreren immunregulatorischen Genen durchgeführt, welche an derselben Liganden-Rezeptor-Bindung beteiligt sind. Die Immuntherapie umfasst mindestens einen Wirkstoff, welcher ein Immun-Checkpoint-Inhibitor ist, der dazu ausgelegt ist, die immunregulatorische Wirkung des Immun-Checkpoints zu verändern. Das Ergebnis der DNA-Methylierungsanalyse wird mit einem Referenzwert verglichen, um das Ansprechen des Patienten auf die Immuntherapie zu bestimmen (Prädiktion).

Der vorliegenden Erfindung ging die Erkenntnis voraus, dass maligne Erkrankungen komplexe genetische und epigenetische Veränderungen aufweisen und folglich sehr individuell ausgeprägt sein können. Selbst maligne Erkrankungen des gleichen Organs und im gleichen Erkrankungsstadium können daher zu einer unterschiedlichen Prognose des Patienten führen. Der Erfinder hat im Rahmen seiner Forschungsarbeit erkannt, dass es immunologische Subtypen von malignen Erkrankungen gibt, welche besser oder schlechter von dem körpereigenen Immunsystem in Schach gehalten werden, so dass regelmäßig ein günstigerer oder ungünstigerer Krankheitsverlauf resultiert. Diese Eigenschaft konnte darauf zurückgeführt werden, dass diese Subtypen besondere Signaturen von DNA-Methylierung und mRNA-Expression immunregulatorischer Gene aufweisen.

Entsprechend den Nachforschungen des Erfinders sind bei Patienten mit günstigem Verlauf die Zellen der malignen Erkrankungen beziehungsweise die mit den Zellen der malignen Erkrankung wechselwirkenden T-Lymphozyten oder auch beide Zelltypen unter anderem durch die charakteristische Expression von einem oder mehreren B7-Proteinen und deren Rezeptoren, einem oder mehreren MHC:Peptid-Komplex bindenden Co-Rezeptoren, einem oder mehreren von TNFRSF9, CD40, TNFRSF4, TNFRSF18 und CD27, einem oder mehreren von TIGIT, BTLA HAVCR2, BTNL2, CD48 und/oder dem Adenosin-bindenden Adenosin 2A-Rezeptor gekennzeichnet. Der Erfinder konnte in diesem Zusammenhang erstmals nachweisen, dass die Expression dieser immunreglatorischen Gene durch Methylierung reguliert wird. Auf diese Weise lässt sich durch eine entsprechende DNA-Methylierungsanalyse ein immunologischer Phänotyp beschreiben, welcher der durch die Methylierung der immunregulatorischen Gene definiert und objektiv messbar ist.

Im Rahmen der Erfindung konnte festgestellt werden, dass die Methylierung der erfindungsgemäßen immunregulatorischen Gene signifikant mit der Prognose der malignen Erkrankung des Patienten korreliert. Eine wesentliche und außerordentlich überraschende Erkenntnis des Erfinders ist, dass es sich dabei um ein generelles Phänomen von malignen Erkrankungen handelt, welches nicht auf bestimmte Entitäten beschränkt ist. Damit stellt die vorliegende Erfindung erstmals prognostische Tests bereit, welche auf der Grundlage einer DNA-Methylierungsanalyse derselben Gene eine universelle Eignung für die Vorhersage des Krankheitsverlaufs einer Vielzahl verschiedener maligner Erkrankungstypen aufweisen, wohingegen Methylierungsanalysen der bisher als Biomarker bekannten Gene jeweils nur eine Prognose in Bezug auf einzelne oder wenige Entitäten erlaubten. Diesbezüglich wird auch auf die nachfolgenden Ausführungsbeispiele verwiesen.

Ein weiteres Alleinstellungsmerkmal und besonderer Vorteil der Erfindung ist, dass die vom Erfinder identifizierten immunregulatorischen Gene, deren DNA-Methylierung prognostisch für den Verlauf einer malignen Erkrankung ist, gleichzeitig auch für Immun-Checkpoints codieren, welche Schlüsselziele von Immuntherapien darstellen. Das Wissen um den Grad der An- oder Abwesenheit der entsprechenden Genprodukte ist somit für die Planung einer effektiven immuntherapeutischen Behandlung hochrelevant. Da gezeigt werden konnte, dass die DNA-Methylierung der immunregulatorischen Gene direkt mit der Expression der entsprechenden Immun-Checkpoints korreliert ist, ermöglicht das erfindungsgemäße Verfahren alternativ oder zusätzlich zur Abschätzung der Prognose auch eine Vorhersage über das Ansprechen des Patienten auf eine entsprechende Immuntherapie. Beispielsweise ist das Ansprechen demnach wahrscheinlich, wenn die DNA-Methylierungsanalyse eine Expression des Immun-Checkpoints anzeigt. Somit kann erstmals eine gleichzeitige Bestimmung der Prognose des Patienten und die Prädiktion des Ansprechens auf eine Immuntherapie innerhalb desselben Tests durchgeführt werden, wohingegen konventionelle Verfahren auf der Grundlage herkömmlicher Markergene bisher entweder nur eine Prognose oder nur eine Prädiktion ermöglichten.

Die DNA-Methylierungsanalyse kann im Grunde genommen mit allen gängigen Methoden durchgeführt werden, welche dem Fachmann aus der einschlägigen Literatur bekannt sind. Ein geeignetes Verfahren umfasst beispielsweise die folgenden Schritte: A) Bereitstellen von DNA der malignen Zellen beziehungsweise der T-Lymphozyten; B) Umwandeln zumindest eines Teils der in der DNA enthaltenen Cytosine in Uracil oder eine andere Base mit einem von Cytosin unterscheidbaren Basenpaarungsverhalten und/oder Molekulargewicht; C) Untersuchen der DNA-Methylierung des immunregulatorischen Gens mit der aus Schritt B) erhaltenen DNA.

Die zu analysierende DNA aus Schritt A) kann aus verschiedenen Quellen stammen, beispielsweise von Zellen der malignen Erkrankung oder infiltrierenden T-Lymphozyten aus operativ oder bioptisch entnommenem Gewebe. Die Zellen können auch von Abstrichen und aus Aspiraten wie zum Beispiel Spülflüssigkeiten, Feinnadelaspiraten oder Sputum stammen. Die DNA kann auch aus Blut, Blutserum und Blutplasma stammen, beispielsweise in Form von frei zirkulierender DNA, exosomaler DNA, oder in Form von frei zirkulierenden Zellen, aus denen die DNA gewonnen wird. Die DNA kann auch aus weiteren Körperflüssigkeiten wie zum Beispiel Urin, Pleuraergüssen oder Aszites stammen, beispielsweise in Form von freier DNA oder in Form von Zellen, aus denen die DNA gewonnen wird. Die DNA kann auch aus nicht-konservierten (frischen) Zellen, Geweben und Körperflüssigkeiten, sowie aus fixierten Zellen, Geweben und Körperflüssigkeiten gewonnen werden. Die Fixierung der Zellen, Gewebe und Körperflüssigkeiten kann durch präzipitierende Fixative wie zum Beispiel Ethanol und andere Alkohole oder durch quervernetzende Fixative wie zum Beispiel Formaldehyd erreicht werden. Die DNA kann auch aus beliebigen Kombinationen dieser Quellen stammen. Es kann sich auch um extrahierte DNA aus den vorgenannten Quellen handeln. Es ist auch möglich, die DNA anzureichern, zum Beispiel durch Fällung oder Extraktion. Dies kann sich zum Beispiel bei frei zirkulierender DNA aus den genannten Körperflüssigkeiten anbieten. Es ist auch möglich, die Zellen anzureichern, zum Beispiel durch Größenfiltration oder über Magnetpartikel, welche Antikörper auf der Oberfläche tragen, deren Antigene sich auf der Oberfläche der anzureichernden Zellen befinden. Dies kann sich zum Beispiel bei frei zirkulierenden Zellen der malignen Erkrankung oder T-Lymphozyten aus den genannten Körperflüssigkeiten anbieten. Weitere geeignete Quellen für die zu analysierende DNA sind oder Homogenisate von Frischgeweben und Lysate von fixierten Geweben.

In einer bevorzugten Variante umfasst die DNA frei zirkulierende DNA, DNA aus Exosomen und/oder DNA aus frei zirkulierenden Zellen aus einer Körperflüssigkeit, so genannte Flüssigbiopsien oder "liquid biopsies". Flüssigbiopsien stellen zurzeit ein zentrales Gebiet der onkologischen Forschung dar. Es wird dabei nicht das verdächtige Gewebe selbst, also zum Beispiel ein Tumorgewebe, sondern eine Probe einer Körperflüssigkeit analysiert, beispielsweise eine Blutprobe. In dieser Probe können verschiedene Substanzen untersucht werden, die aus dem Tumor stammen, da frei zirkulierende genomische DNA, exosomale DNA, beziehungsweise frei zirkulierende Zellen vom Tumor in die Blutbahn freigesetzt werden. Mit Vorteil wird das erfindungsgemäße Verfahren zur Analyse von Flüssigbiopsien verwendet, wenn der Tumor oder eine Metastase nicht biopsiert werden kann beziehungsweise wenn eine Biopsie für den Patienten im späten Tumorstadium eine zu große Gefahr darstellen würde. Es zeichnet die vorliegende Erfindung aus, dass die DNA-Methylierung der immunregulatorischen Gene sehr gut in Körperflüssigkeiten messbar ist, wohingegen ein herkömmlicher Expressionsnachweis der immunregulatorischen Gene anhand von mRNA oder Immunohistochemie nur schwer oder gar nicht möglich ist.

Die Umwandlung der DNA in Schritt B) kann im Prinzip mit allen im Stand der Technik für diesen Zweck bekannten und geeigneten Methoden erfolgen. Typischerweise handelt es sich um eine chemische oder enzymatische Umwandlung, beispielsweise durch Kontaktieren der DNA mit Bisulfit, zum Beispiel Natriumbisulfit oder Ammoniumbisulfit. Erforderlichenfalls kann nach der Umwandlung in Schritt B) und vor der Untersuchung der DNA-Methylierung in Schritt C) ein Aufreinigen der DNA erfolgen. Geeignete Aufreinigungsmethoden und Protokolle sind dem Fachmann bekannt und können zum Beispiel eine DNA-Extraktion, eine Fällung oder eine polymergestützte Anreicherung (polymermediated enrichment) umfassen.

Die DNA-Methylierungsanalyse kann ein Bestimmen von Anwesenheit, Abwesenheit oder Ausmaß einer Methylierung von mindestens einem in dem immunregulatorischen Gen enthaltenen CpG-Dinukleotid umfassen. Es können auch mehrere CpG-Dinukleotide des immunregulatorischen Gens untersucht werden. Diese können sich auch auf verschiedene zu untersuchende Teilbereiche des immunregulatorischen Gens verteilen. In bevorzugten Varianten wird die Methylierung von mindestens je einem CpG-Dinukleotid von mindestens zwei verschiedenen immunregulatorischen Genen bestimmt. Auf diese Weise löst die Erfindung das Problem, dass CpG-Dinukleotide in der DNA einer malignen Erkrankung mitunter heterogen methyliert vorliegen können. Falsch-negative und falsch-positive Messergebnisse werden folglich vermieden, sodass eine besonders zuverlässige und darüber hinaus besonders differenzierte Prognose und/oder Prädiktion erreicht wird, wie aus den nachfolgenden Ausführungsbeispielen ersichtlich ist.

Die Untersuchung der DNA-Methylierung des immunregulatorischen Gens in Schritt C) unterliegt dabei keinen besonderen Beschränkungen. Geeignete Methoden kann ein Fachmann unschwer auf der Grundlage dieser Offenbarung bestimmen. Diesbezüglich wird auch auf die oben genannten Laborhandbücher verwiesen. In einer bevorzugten Variante wird zunächst eine Polymerasekettenreaktion (PCR) mit Oligonukleotiden, sogenannten Primern, durchgeführt, welche dazu ausgelegt ist, einen Abschnitt der umgewandelten DNA zu amplifizieren, welcher das mindestens eine zu analysierende CpG-Dinukleotid umfasst. Anschließend erfolgt vorzugsweise eine Sequenzierung zumindest eines Teils des Amplifikats, zum Beispiel eine Sanger-Sequenzierung, Pyrosequenzierung, massenspektrometrische Sequenzierung oder eine Sequenzierung der zweiten oder dritten Generation, welche auch als "Massive Parallel Sequencing", "Next Generation Sequencing" (NGS) oder als Nanoporensequenzierung bezeichnet werden. Es ist auch möglich, im Anschluss an die PCR eine Hybridisierung mit mutationsspezifischen Oligonukleotiden (Sonden) durchzuführen, beispielsweise in Form eines DNA-Microarrays. Die Methylierung kann auch durch quantitative Echtzeit-PCR (quantitative real-time PCR, qPCR), gegebenenfalls gefolgt von einer Schmelzkurvenanalyse, bestimmt werden. Insbesondere kann die quantitative Echtzeit-PCR mit methylierungsspezifischen Primern wie in WO 1997/046705 A1 und/oder methylierungsspezifischen Blockeroligonukleotiden wie in WO 2002/072880 A2 durchgeführt werden. In einer bevorzugten Variante werden methylierungsspezifische Detektionssonden verwendet.

In wiederum anderen bevorzugten Varianten kann eine PCR entfallen, beispielsweise bei einem "Whole Genome Shotgun Bisulfite Sequencing" (WGSBS) oder einer direkten Nanoporensequenzierung. Bei der WGSBS wird die DNA fragmentiert, anschließend werden Adapter an die DNA Fragmente ligiert. Über die Adapter ist im Anschluss eine Amplifikation und Sequenzierung möglich. Es ist auch möglich bei der WGSBS den Schritt der Fragmentierung wegzulassen, da die DNA zum Beispiel durch die Umwandlung durch Bisulfit-Behandlung bereits fragmentiert vorliegen kann. Protokolle für die Durchführung einer WGSBS sind dem Fachmann leicht zugänglich (Johnson, M. D. et al., Curr. Protoc. Mol. Biol., 2012, 99, 21.23.1-21.23.28; Lister, R. et al., Nature, 2009, 462, 315-322; Berman, B. P. et al., Nat. Genet., 2011, 44, 40-46) .

In einer anderen bevorzugten Variante kann vor der PCR Amplifikation eine Hybridisierung mit spezifischen Oligonukleotiden (Sonden) erfolgen, die im Falle des Bindens ligiert werden und anschließend mittels PCR amplifiziert werden. Geeignete Methoden und Protokolle, wie zum Beispiel eine "multiplex ligation dependent probe amplification" (MLPA) stehen dem Fachmann ohne Probleme zur Verfügung, zum Beispiel in "PCR Mutation Detection Protocols" von B. D. M. Theophilus und R. Rapley, 2nd Edition, 2011, Springer.

In einer weiteren bevorzugten Variante wird die Methylierungsanalyse mittels des Infinium HumanMethylation450 BeadChip durchgeführt. Geeignete Protokolle finden sich beispielsweise im Kapitel "Determination of DNA Methylation Levels Using Illumina HumanMethylation450 BeadChips" von M.A. Carless, welches im Buch "Chromatin Protocols" von S.P. Chellappan, Band 1288, 2015, der Buchserie "Methods in Molecular Biology", Springer Science+Business Media New York, zu finden ist. Weitere geeignete Protokolle sind aus den nachfolgenden Ausführungsbeispielen ersichtlich.

Die DNA-Methylierungsanalyse wird vorzugsweise unter Bedingungen durchgeführt, welche eine quantitative Bestimmung der Methylierung des mindestens einen CpG-Dinukleotids erlauben. Die quantitative Bestimmung kann auch den Methylierungszustand mehrerer CpG-Dinukleotide in einem oder mehreren Genen umfassen. Anschließend kann eine Mittlung der erhaltenen Quantitäten erfolgen, zum Beispiel um einen besonders robusten Wert zu erhalten. Auf diese Weise wird eine besonders hohe Robustheit und Präzision des Verfahrens erreicht. Eine quantitative Methylierungsanalyse ist beispielsweise dafür geeignet, eine relative Methylierung zu bestimmen, indem die Anzahl der methylierten Kopien eines Lokus zu der Gesamtzahl der Kopien desselben Lokus in ein Verhältnis gesetzt wird.

Das Ergebnis der DNA-Methylierungsanalyse beziehungsweise die Methylierung des immunregulatorischen Gens kann anschließend beispielsweise mit einem Referenzwert verglichen werden, um die Prognose und/oder das wahrscheinliche Ansprechen des Patienten auf die Immuntherapie zu bestimmen. Das Festlegen von geeigneten Referenzwerten gehört zur labormedizinischen Routine. Eine Möglichkeit ist, bei einer Gruppe von Patienten mit einer vergleichbaren malignen Erkrankung, insbesondere der gleichen Entität, deren Prognose und/oder Ansprechverhalten auf eine Immuntherapie im Rahmen einer retrospektiven Analyse bereits bekannt ist, eine DNA-Methylierungsanalyse des immunregulatorischen Gens durchzuführen. Auf dieser Grundlage kann dann die DNA-Methylierung des immunregulatorischen Gens mit der Prognose und/oder dem wahrscheinlichen Ansprechen auf die Immuntherapie korreliert werden. Beispielsweise wird die retrospektiv analysierte Gruppe in zwei oder mehrere Untergruppen eingeteilt, deren Prognose oder Ansprechverhalten sich anhand der DNA-Methylierung des immunregulatorischen Gens unterscheiden lässt. Ein geeigneter Referenzwert ist dann beispielsweise ein Wert der DNA-Methylierung, welcher zwei Untergruppen voneinander trennt, nachfolgend auch als Schwellenwert bezeichnet. Je nachdem, ob beim erfindungsgemäßen Verfahren das Ergebnis der DNA-Methylierungsanalyse über oder unter dem Schwellenwert liegt, kann folglich ein individueller Patient einer der Gruppen mit bekannter Prognose beziehungsweise bekanntem Therapieansprechen zugeordnet werden.

Es ist auch möglich, das Ergebnis der DNA-Methylierungsanalyse mit einer mRNA-Expression des immunregulatorischen Gens zu korrellieren. Ist die Expression dieses Gens beispielsweise ein Indikator für die Prognose des Patienten, dann kann so über die Bestimmung der mRNA-Expression anhand der DNA-Methylierung dieses Gens die Prognose des Patienten bestimmt werden. Da die Expression der erfindungsgemäßen immunregulatorischen Gene auch den Angriffspunkt für die immuntherapeutische Behandlung liefert, kann auf diese Weise auch das Ansprechen auf eine entsprechende Immuntherapie zuverlässig vorausgesagt werden, welche den vom immunregulatorischen Gen codierten Immun-Checkpoint als Angriffspunkt nutzt.

Es zeichnet die vorliegende Erfindung besonders aus, dass sich die DNA-Methylierungsanalyse der erfindungsgemäßen immunregulatorischen Gene in einer für den Fachmann nicht vorhergesehenen Weise universell für eine Prognose des Krankheitsverlaufs beziehungsweise die Vorhersage des Ansprechverhaltens auf eine Immuntherapie bei vielen verschiedenen Tumorentitäten eignet. Die maligne Erkrankung kann insbesondere ein Karzinom, ein Melanom, ein Sarkom, ein Gliom, ein Lymphom und/oder eine Leukämie umfassen. Das Karzinom kann zum Beispiel ein Adenokarzinom, ein Plattenepithelkarzinom, ein kleinzelliges Karzinom, ein neuroendokrines Karzinom, ein Nierenzellkarzinom, ein Urothelkarzinom, ein hepatozelluläres Karzinom, ein Analkarzinom, ein Bronchialkarzinom, ein Endometriumkarzinom, ein cholangiozelluläres Karzinom, ein hepatozelluläres Karzinom, ein Hodenkarzinom, ein kolorektales Karzinom, ein Karzinom des Kopf- und Halsbereichs, ein Karzinom des Ösophagus, ein Magenkarzinom, ein Mammakarzinom, ein Nierenkarzinom, ein Ovarialkarzinom, ein Pankreaskarzinom, ein Prostatakarzinom, ein Schilddrüsenkarzinom und/oder ein Zervixkarzinom umfassen. Ein Sarkom kann beispielsweise ein Angiosarkom, ein Chondrosarkom, ein Ewing-Sarkom, ein Fibrosarkom, ein Kaposi-Sarkom, ein Liposarkom, ein Leiomyosarkom, ein malignes fibröses Histiozytom, ein neurogenes Sarkom, ein Osteosarkom oder ein Rhabdomyosarkom sein. Eine Leukämie kann beispielsweise eine akute myeloische Leukämie (AML), eine akute lymphatische Leukämie (ALL), eine chronische lymphatische Leukämie (CLL), oder eine chronische myeloische Leukämie (CML) sein. Ein Lymphom kann ein Hodgkin-Lymphom oder Non-Hodgkin-Lymphom sein. Ein Non-Hodgkin-Lymphom kann ein B-Zell-Lymphom oder ein T-Zell-Lymphom sein. Entsprechende Nachweise finden sich in den Ausführungsbeispielen.

In einer weiteren Variante des Verfahrens wird zusätzlich zu der DNA-Methylierungsanalyse eine mRNA-Expressionsanalyse des immunregulatorischen Gens durchgeführt. Diese kann zum Beispiel eine Bestimmung des mRNA-Expressionsniveaus von mindestens einer Transkriptvariante des immunregulatorischen Gens oder von mindestens einer Transkriptvariante von mindestens zwei verschiedenen immunregulatorischen Genen umfassen. Vorzugsweise erfolgt auch die mRNA-Expressionsanalyse unter Bedingungen, welche eine quantitative Bestimmung des mRNA-Expressionsniveaus erlauben.

Geeignete Protokolle für die mRNA-Expressionsanalyse sind dem Fachmann hinlänglich bekannt und umfassen üblicherweise die Schritte a) Bereitstellen von mRNA der malignen Zellen beziehungsweise der T-Lymphozyten; b) Umschreiben der mRNA in cDNA; c) Nachweisen von cDNA des immunregulatorischen Gens. Es ist aber auch möglich, die mRNA direkt ohne vorhergehendes Umschreiben in cDNA zu analysieren. Insbesondere bietet sich hierfür die Nanostringtechnologie an. Die mRNA-Expressionsanalyse kann auch mittels RNA-Seq (RNA Sequencing), oder auch *whole transcriptome shotgun sequencing* (WTSS), durchgeführt werden. Hierbei wird können Next-Generation Sequencing (NGS) Methoden verwendet werden um die Anwesenheit oder und/oder die Quantität einer mRNA zu bestimmen. Beispielsweise kann eine Anzahl von mRNA Molekülen einer bestimmten Sequenz bestimmt werden und zu der Anzahl aller mRNA Moleküle ins Verhältnis gesetzt werden, um eine normalisierte Anzahl (*normalized count*) zu bestimmen, so wie es in den Ausführungsbeispielen angewendet wurde. Geeignete RNA-Seq Techniken und Komplettlösungen sind beispielsweise von der Firma Illumina, San Diego, Ca, USA, erhältlich.

Überraschenderweise führt die erfindungsgemäße Kombination von DNA-Methylierungsanalyse und mRNA-Expressionsanalyse zu einer zusätzlich verbesserten Abschätzung der Prognose, in der Hinsicht, dass eine noch genauere Stratifikation von Prognosegruppen möglich wird. Die Kombination der Analysen kann derart erfolgen, dass für einen Patienten zunächst sowohl anhand der DNA-Methylierungsanalyse als auch anhand der mRNA-Expressionsanalyse eines immunregulatorischen Gens jeweils eine Prognosegruppe bestimmt wird, beispielsweise anhand oben beschriebener Referenzwerte. Anschließend können diese Prognosegruppen zusammengeführt werden, so dass sich weitere Subgruppen für die Prognose ergeben. Eine Subgruppe mit schlechter Prognose kann zum Beispiel gebildet werden, wenn sowohl nach der mRNA-Expressionsanalyse und auch nach der DNA-Methylierungsanalyse des immunregulatorischen Gens jeweils eine schlechte Prognose anzeigen. Eine Subgruppe mit guter Prognose kann beispielsweise gebildet werden, wenn der Patient sowohl anhand der mRNA-Expressionsanalyse als auch anhand der DNA-Methylierungsanalyse des immunregulatorischen Gens eine gute Prognose hat. Zwei intermediäre Prognosegruppen umfassen zum Beispiel diejenigen Patienten, für welche entweder die DNA-Methylierungsanalyse oder die mRNA-Expressionsanalyse des immunregulatorischen Gens eine gute Prognose und die jeweils andere Analyse eine schlechte Prognose anzeigt. Eine noch differenziertere Stratifizierung ist möglich, wenn mehrere immunregulatorische Gene analysiert werden, anhand derer eine multifaktorielle Einteilung in Prognosegruppen vorgenommen werden kann. Auf dieser Grundlage kann mithilfe vorliegender Erfindung die klinische Entscheidungsfindung in bisher noch nicht da gewesener Weise anhand objektiv messbarer Parameter noch genauer und noch zuverlässiger auf den individuellen Patienten abgestimmt werden.

Die veränderte DNA-Methylierung von Zellen einer malignen Erkrankung betrifft eine Vielzahl von CpG-Dinukleotiden innerhalb und in der Umgebung eines Gens. Für die erfindungsgemäße Verfahrensausführung sind insbesondere CpG-Dinukleotide in Promotoren und in der für das Transkript des immunregulatorischen Gens kodierenden Sequenz in den Zellen der malignen Erkrankung beziehungsweise den T-Lymphozyten geeignet. Allerdings kann auch die Methylierung von CpG-Dinukleotiden außerhalb des Genkörpers der immunregulatorischen Gene in den Zellen der malignen Erkrankung oder der T-Lymphozyten verändert und für das erfindungsgemäße Verfahren geeignet sein. Bevorzugte Bereiche für die erfindungsgemäße DNA-Methylierungsanalyse umfassen regulatorische Genbereiche, insbesondere Transkriptionsfaktorbindestellen, Promotoren, CpG-Inseln, Silencer, Enhancer, CTCF-Bindestellen sowie Kombinationen davon. Enhancer können beispielsweise vom Gen entfernt als distale Enhancer vorliegen. Enhancer können auch in der Nähe des Gens liegen und werden dann proximale Enhancer benannt. Regulatorische Genbereiche sind dem Fachmann gut bekannt und beispielsweise in "Gene Control" von D. S. Latchman, 2. Edition, 2015, Garyland Science, Taylor & Francis Group, LLC, beschrieben. Für die erfindungsgemäße Verfahrensausführung sind beispielsweise auch diejenigen CpG-Dinukleotide geeignet, deren Methylierungszustand mit der transkriptionellen Aktivität beziehungsweise der Expression eines Gens korreliert. Die transkriptionelle Aktivität ist beispielsweise anhand einer veränderten Chromatinstruktur ersichtlich. Sogenanntes "offenes Chromatin" kann mit einer hohen transkriptionellen Aktivität eines Gens einhergehen wie beispielsweise in "Genetik" von W. Janning und E. Kunst, 2004, Georg Thieme Verlag, Stuttgart und New York, beschrieben ist. Bereiche "offenen Chromatins" sind daher für die erfindungsgemäße DNA-Methylierungsanalyse geeignet.

Die Bestimmung regulatorischer Genelemente ist für den Fachmann ohne Weiteres anhand geeigneter Datenbanken möglich. Beispielsweise sind in der Datenbank "Ensembl" solche regulatorischen Elemente annotiert, wie beispielsweise in "The Ensembl Regulatory Build" von D. R. Zerbino, S. P. Wilder, N. Johnson, T. Juettemann und P. R. Flicek, 2015, Genome Biology, Ausgabe 16, doi:10.1186/s13059-015-0621-5 beschrieben.

Eine geeignete Primärsequenz des humanen Genoms, anhand derer geeignete und bevorzugte Bereiche und Sequenzen der immunregulatorischen Gene für die erfindungsgemäße DNA-Methylierungsanalyse bestimmt werden können, ist beispielsweise die humane Genomversion des Genome Reference Consortium Genome Reference Consortium Human Build 38 (GRCh38) beziehungsweise Reference Consortium Human Build 38 patch release 7 (GRCh38.p7) mit Stand vom 21. März 2016. Auf Bereiche des Genoms wird nachfolgend in der Schreibweise "Chromosomennummer:Position der ersten Base des Bereichs-Position der letzten Base des Bereichs" Bezug genommen, also beispielsweise "2:241849881-241858908" für den Bereich von Base 241849881 bis Base 241858908 von Chromosom 2.

In einer Variante ist das immunregulatorische Gen ausgewählt aus den für B7-Proteine und deren Rezeptor codierenden Genen *CD274, PDCD1LG2* und *PDCD1.* In einer weiteren Variante ist das immunregulatorische Gen ausgewählt aus den für das B7-Protein und dessen Rezeptor codierenden Genen *CD80* und *CTLA4.* In noch einer weiteren Variante ist das immunregulatorische Gen ausgewählt aus dem für den Rezeptor eines B7-Proteins codierenden Gen *ICOS* und den für die B7-Proteine codierenden Genen *CD276, C10orf54* und *HHLA2.* In noch einer weiteren Variante ist das immunregulatorische Gen ausgewählt aus den für die MHC:Peptid-Komplex bindenden Co-Rezeptoren codierenden Genen *LAG3, CD160, KIR2DL4, KIR3DL1.* Es sind auch beliebigen Kombinationen der immunregulatorischen Gene aus diesen Varianten möglich.

Das Gen *PDCD1* beziehungsweise *Programmed Cell Death 1* ist auch unter den synonymen Bezeichnungen *CD279, PD1, hPD-1, SLEB2, PD-1, hSLE1* und *hPD-1* bekannt. *PDCD1* ist ein immunregulatorisches Gen und codiert für ein Protein, welches ein Immun-Checkpoint im Sinne der Erfindung ist. Das Protein PDCD1 ist ein Rezeptor und kann beispielsweise die beiden zu den B7-Proteinen gehörenden Liganden binden, die durch die Gene *CD274* und *PDCD1LG2* codiert sind. *PDCD1* gehört zu der Immunglobulin-Superfamilie. Bevorzugte Bereiche für die DNA-Methylierungsanalyse von *PDCD1* sind in Bereichen, die für die Transkripte codieren (2:241849881-241858908, SEQ ID NO:17), Regionen mit offenem Chromatin (2:241849051-241853001, SEQ ID NO:18 und 2:241861820-241862593, SEQ ID NO:19), dem Enhancer (2:241852997-241855201, SEQ ID NO:2), einer CTCF-Bindestelle (2:241859081-241860074, SEQ ID NO:3) und/oder Promotoren (2:241856912-241861429, SEQ ID NO:29 und 2:241862929-241865230, SEQ ID NO:30) enthalten. Weitere bevorzugte Bereiche für die DNA-Methylierungsanalyse von *PDCD1* sind aus denjenigen ausgewählt, welche für diesen Zweck in den Ausführungsbeispielen 1, 3, 5, 7, 8, 10, 12 und 17 beschrieben sind. Für die erfindungsgemäße mRNA-Expressionsanalyse eignet sich der Nachweis zumindest eines Teils der Sequenz von Transkripten, deren cDNA zumindest teilweise SEQ ID NO:73, SEQ ID NO:74 und/oder SEQ ID NO:75 entspricht. Die Vorhersage des Ansprechens auf eine Immuntherapie umfasst insbesondere Wirkstoffe, welche geeignet sind, den durch *PDCD1* codierten Rezeptor und/oder dessen Liganden, wobei die Liganden insbesondere durch die immunregulatorischen Gene *PDCD1LG2* und *CD274* codiert sind, zu inhibieren. Beispiele sind Nivolumab (Handelsname: Opdivo, Hersteller: Bristol-Myers Squibb), Pembrolizumab (Handelsname: Keytruda; Hersteller: Merck/ MSD Sharp & Dohme), Pidilizumab (CT-011, Hersteller: CureTech Ltd.), MGD013 (Macrogenics), AMP-224 (Hersteller: GlaxoSmithKline), MEDI0680 (AMP-514, Hersteller: MedImmune LLC), AUNP-12 (Hersteller: Aurigene Discovery Technologies Ltd.), BMS935559 (MDX1105, Hersteller: Bristol-Myers Squibb), CA-170 (Hersteller: Curis Inc.), MPDL3280A (Hersteller: Roche), MEDI4736 (Hersteller: AstraZeneca), Avelumab (MSB0010718C, Hersteller: Pfizer) und rHIgM12B7 (B7-DC cross-linking antibody rHIgM12B7, Mayo Clinic).

Das Gen *CD274* beziehungsweise *CD274 molecule* ist auch unter den synonymen Bezeichnungen *PDCD1L1, B7-H, B7-H1, PDCD1LG1, PD-L1, PDL1* und *B7H1* bekannt. *CD274* ist ein immunregulatorisches Gen und codiert für ein B7-Protein, welches ein Immun-Checkpoint im Sinne der Erfindung ist. *CD274* codiert für ein Protein, welches ein Ligand des durch *PDCD1* codierten Rezeptors ist. *CD274* gehört zu der Immunglobulin-Superfamilie. Bevorzugte Bereiche für die DNA-Methylierungsanalyse von *CD274* sind in dem Bereich, der für die Transkripte codiert (9:5450503-5470566, SEQ ID NO:1) sowie den Promotoren (9:5445402-5456799, SEQ ID NO:76 und 9:5458041-5461360, SEQ ID NO:77), Enhancern (9:5457122-5457702, SEQ ID NO:78; 9:5463574-5468340, SEQ ID NO:79; 9:5440647-5441785, SEQ ID NO:80 und 9:5472191-5473149, SEQ ID NO:81) und/oder CTCF-Bindestellen (9:5440970-5441435, SEQ ID NO:82 und 9:5446325-5446870, SEQ ID NO:83) enthalten. Weiterhin bevorzugte Bereiche für die DNA-Methylierungsanalyse umfassen den kodierenden Bereich von *CD274,* welcher einen alternativen Promotor und eine CTCF-Bindestelle enthält (9:5451072-5481072, SEQ ID NO:371), den alternativen Promotor (9:5451072-5461819, SEQ ID NO:375), den regulatorischen Bereich vor dem Promotor (9:5433159-5449887, SEQ ID NO:370), den Bereich vor dem Promotor, welcher eine CTCF-Bindestelle enthält (9:5439290-5449887, SEQ ID NO:376), den codierenden Bereich hinter dem Promotor (9:5451072-5470566, SEQ ID NO:372), sowie die Region hinter dem codierenden Bereich, welche eine CTCF-Bindestelle und einen Enhancer umfasst (9:5470566-5496357, SEQ ID NO:373). Weitere bevorzugte Bereiche für die DNA-Methylierungsanalyse von *CD274* sind aus denjenigen ausgewählt, welche für diesen Zweck in den Ausführungsbeispielen 2, 3, 7, 8, 10, 12, 15 und 17 beschrieben sind. Für die erfindungsgemäße mRNA-Expressionsanalyse eignet sich der Nachweis zumindest eines Teils der Sequenz von Transkripten, deren cDNA zumindest teilweise SEQ ID NO:28, SEQ ID NO:101, SEQ ID NO:102, SEQ ID NO:103 und/oder SEQ ID NO:104 entspricht. Die Vorhersage des Ansprechens auf eine Immuntherapie umfasst insbesondere Wirkstoffe, welche das durch *CD274* codierte B7-Protein und/oder den entsprechenden durch *PDCD1* codierten Rezeptor und/oder den durch *PDCD1LG2* codierten Liganden, welcher ebenfalls an den *PDCD1* codierten Rezeptor bindet, inhibieren. Beispiele sind Nivolumab, Pembrolizumab, Pidilizumab, AMP-224, AMP-514, AUNP-12, BMS935559, CA-170 (Curis), Atezolizumab (MPDL3280A, Roche, Genentech), MGD013 (Macrogenics), MEDI4736, CA-170 (Curis, Inc.) und Avelumab.

Das Gen *PDCD1LG2* beziehungsweise *programmed cell death 1 ligand 2* ist auch unter den synonymen Bezeichnungen *Btdc, PDL2, CD273, PDCD1L2, B7-DC, bA574F11.2, PD-L2* und *B7DC* bekannt. *PDCD1LG2* ist ein immunregulatorisches Gen und codiert für ein B7-Protein, das ein Immun-Checkpoint im Sinne der Erfindung ist. *PDCD1LG2* codiert für ein B7-Protein, welches ein Ligand des durch *PDCD1* codierten Rezeptors ist. *PDCD1LG2* gehört zu der Immunglobulin-Superfamilie. Bevorzugte Bereiche für die DNA-Methylierungsanalyse von *PDCD1LG2* sind in dem Bereich, der für das Transkript codiert (9:5510570-5571254) sowie den Promotoren (9:5507688-5523442, SEQ ID NO:84; 9:5491444-5503289, SEQ ID NO:85; 9:5528150-5534251, SEQ ID NO:86 und 9:5547972-5571492, SEQ ID NO:87) und/oder dem Enhancer (9:5479110-5491616, SEQ ID NO:88; 9:5522642-5528253, SEQ ID NO:89; 9:5534822-5547690, SEQ ID NO:90; 9:5572730-5580962, SEQ ID NO:91) enthalten, sowie der Bereich vor der kodierenden Sequenz (9:5496357-5510570, SEQ ID NO:374). Besonders bevorzugt umfasst die DNA-Methylierungsanalyse eines oder mehrere der CpG-Dinukleotide, welche sich in dem Sequenzbereich 9:5433159-5603325 befinden, der für die Transkripte der benachbarten Gene *CD274* und *PDCD1LG2* codiert und Enhancer und Promotoren dieser Gene umfasst, insbesondere der Bereich zwischen den kodierenden Bereichen von *CD274* und *PDCD1LG2* (9:5470566-5510570; SEQ ID NO:485). Weitere bevorzugte Bereiche für die Methylierungsanalyse von *PDCD1LG2* sind aus denjenigen ausgewählt, welche für diesen Zweck in den Ausführungsbeispielen 3, 5, 7, 8, 10 und 13 beschrieben sind. Für die erfindungsgemäße mRNA-Expressionsanalyse eignet sich der Nachweis zumindest eines Teils der Sequenz vom Transkript, dessen cDNA zumindest teilweise SEQ ID NO:105 entspricht. Die Vorhersage des Ansprechens auf eine Immuntherapie umfasst insbesondere Wirkstoffe, welche das durch *PDCD1LG2* codierte B7-Protein und/oder den entsprechenden durch *PDCD1* codierten Rezeptor, inhibieren. Geeignete Inhibitoren sind beispielsweise Nivolumab, Pembrolizumab, Pidilizumab, AMP-224, AMP-514, AUNP-12, CA-170 (Curis, Inc.) und rHIgM12B7.

Das Gen *ICOS* beziehungsweise *inducible T-cell co-stimulator* ist auch unter den synonymen Bezeichnungen *CD278, CVID1* und *AILIM* bekannt. *ICOS* ist ein immunregulatorisches Gen und codiert für ein Protein, welches ein Immun-Checkpoint im Sinne der Erfindung ist. ICOS ist ein Rezeptor und kann beispielsweise den zu den B7-Proteinen gehörenden Liganden binden, der durch das Gen *ICOSLG* codiert ist. *ICOSLG* beziehungsweise *inducible T-cell co-stimulator ligand* ist ein immunregulatorisches Gen und codiert für einen Liganden, der ein Immun-Checkpoint im Sinne der Erfindung ist. *ICOSLG* wird auch als *ICOSL, B7RP1, LICOS, B7RP-1, KIAA0653, GL50, ICOS-L, CD275, B7H2* und *B7-H2* bezeichnet. *ICOS* und *ICOSLG* gehören zu der Immunglobulin-Superfamilie. Bevorzugte Bereiche für die DNA-Methylierungsanalyse von *ICOS* sind in dem Bereich, der für das Transkript codiert (2:203936748-203961577, SEQ ID NO:92) sowie den Promotoren (2:203934590-203941036, SEQ ID NO:93 und 2:203948548-203953636, SEQ ID NO:94) und/oder dem Enhancer (2:203931099-203937863, SEQ ID NO:95 und 2:203940518-203949061, SEQ ID NO:96) enthalten. Bevorzugte Bereiche für die DNA-Methylierungsanalyse von *ICOSLG* sind in dem Bereich, der für die Transkripte codiert (21:44222991-44240966, SEQ ID NO:97) sowie einer CpG-Insel (21:44240132-44243380, SEQ ID NO:98), dem Promotor (21:44238919-44247988, SEQ ID NO:99) und/oder einer Enhancerregion (21:44214476-44227512, SEQ ID NO:100) enthalten. Weitere bevorzugte Bereiche für die DNA-Methylierungsanalyse von *ICOS* und *ICOSLG* sind aus denjenigen ausgewählt, welche für diesen Zweck in den Ausführungsbeispielen 11, 13 und 17 beschrieben sind. Für die erfindungsgemäße mRNA-Expressionsanalyse von *ICOS* beziehungsweise von *ICOSLG* eignet sich der Nachweis zumindest eines Teils der Sequenz von Transkripten, deren cDNA zumindest teilweise SEQ ID NO:106, SEQ ID NO:107, SEQ ID NO:108, SEQ ID NO:109, SEQ ID NO:110 und/oder SEQ ID NO:111 entspricht. Eine bevorzugte Verfahrensausführung umfasst die Anwendung der DNA-Methylierungsanalyse des *ICOS* und/oder des *ICOSLG* Gens zur Bestimmung des Ansprechens auf einen Wirkstoff, welcher geeignet ist, die immunregulatorische Wirkung von *ICOS* und/oder *ICOSLG* zu verändern, insbesondere den durch *ICOS* codierten Rezeptor zu aktivieren. Ein geeigneter Aktivator ist beispielsweise JTX-2011 (Jounce Therapeutics).

Das Gen *CTLA4* beziehungsweise *cytotoxic T-lymphocyte-associated protein 4* ist auch unter den synonymen Bezeichnungen *CELIAC3, GRD4, CTLA-4, IDDM12, CD, CD28, GSE, CD152* und *ALPS5* bekannt. *CTLA4* ist ein immunregulatorisches Gen und codiert für ein Protein, welches ein Immun-Checkpoint im Sinne der Erfindung ist. *CTLA4* codiert für einen Rezeptor, der beispielsweise den zu den B7-Proteinen gehörenden Liganden binden kann, welcher durch das Gen *CD80* codiert ist. *CD80* beziehungsweise *CD80 molecule* ist ein immunregulatorisches Gen und codiert für einen Liganden, der ein Immun-Checkpoint im Sinne der Erfindung ist. *CD80* ist beispielsweise auch *B7, CD28LG1, B7.1, B7-1, LAB7, BB1 und CD28LG* benannt. *CTLA4* und *CD80* gehören zu der Immunglobulin-Superfamilie. Bevorzugte Bereiche für die Methylierungsanalyse von *CTLA4* sind in dem Bereich, der für die Transkripte codiert (2:203867786-203873960, SEQ ID NO:160) sowie den Promotoren (2:203866174-203868926, SEQ ID NO:162 und 2:203869477-203874095, SEQ ID NO:164), dem Enhancer (2:203874152-203875266, SEQ ID NO:165; 2:203876672-203878051, SEQ ID NO:166 und 2:203879313-203881585, SEQ ID NO:167) und/oder der Region zwischen *CTLA4* und dem benachbarten immunregulatorischen Gen *ICOS* (2:203872383-203939876) enthalten. Weitere bevorzugte Bereiche für die DNA-Methylierungsanalyse von *CTLA4* sind aus denjenigen ausgewählt, welche für diesen Zweck in den Ausführungsbeispielen 4, 7, 8, 11, 13, 15 und 16 beschrieben sind. Bevorzugte Bereiche für die DNA-Methylierungsanalyse von *CD80* sind beispielsweise in dem Bereich, der für die Transkripte codiert (3:119524293-119559602) sowie den Promotoren (3:119554042-119563668, SEQ ID NO:163 und 3:119568227-119573274, SEQ ID NO:177) und/oder dem Enhancer (3:119563379-119568778, SEQ ID NO:178; 3:119538188-119543511, SEQ ID NO:179 und 3:119545840-119554325, SEQ ID NO:180) enthalten. Weitere bevorzugte Bereiche für die Methylierungsanalyse von *CD80* sind aus denjenigen ausgewählt, welche für diesen Zweck in den Ausführungsbeispielen 11, 13, 15 und 16 beschrieben sind. Für die erfindungsgemäße mRNA-Expressionsanalyse von *CTLA4* beziehungsweise von *CD80* eignet sich der Nachweis zumindest eines Teils der Sequenz von Transkripten, deren cDNA zumindest teilweise SEQ ID NO:168, SEQ ID NO:169, SEQ ID NO:170, SEQ ID NO:171, SEQ ID NO:172 und SEQ ID NO:173 beziehungsweise SEQ ID NO:174, SEQ ID NO:175 und/oder SEQ ID NO:176 entspricht. Eine bevorzugte Verfahrensausführung beinhaltet die Anwendung der DNA-Methylierungsanalyse des *CTLA4* und/oder des *CD80* Gens zur Vorhersage des Ansprechens auf Wirkstoffe, welche geeignet sind, die immunregulatorische Wirkung von *CTLA4* und/oder *CD80* zu verändern, insbesondere zu inhibieren. Geeignete Wirkstoffe sind beispielsweise Ipilimumab (MDX-010; Handelsname Yervoy, Bristol-Myers Squibb), Tremelimumab (Ticilimumab, CP-675,206, Pfizer, MedImmune, Astra Zeneca).

Weitere für die erfindungsgemäße Verfahrensausführung bevorzugte immunregulatorischen Gene, welche für B7-Proteine codieren, sind die Gene *CD276, C10orf54, HHLA2, NCR3LG1, CD86* und *VTCN1.* Die Gene *CD276, C10orf54, HHLA2, NCR3LG1, CD86* und *VTCN1* codieren Immun-Checkpoints im Sinne der Erfindung, welche ebenfalls zu der Immunglobulin-Superfamilie gehören. Das Gen *CD276* beziehungsweise *CD276 molecule* wird auch als *B7-H3, B7H3, 4Ig-B7-H3* und *B7RP-2* bezeichnet. Bevorzugte Bereiche für die DNA-Methylierungsanalyse von *CD276* sind beispielsweise in dem Bereich, welcher für die Transkripte codiert (15:73683966-73714518, SEQ ID NO:181) sowie Promotoren (15:73679515-73692168, SEQ ID NO:182 und 15:73693817-73698829, SEQ ID NO:184), einer CpG-Insel (15:73683810-73685004, SEQ ID NO:183), einer CTCF- und Transkriptionsfaktorenbindestelle (15:73699041-73706032, SEQ ID NO:185) und Enhancern (15:73674927-73679619, SEQ ID NO:186 und 15:73710442-73723236, SEQ ID NO:187) enthalten. Weitere bevorzugte Bereiche für die Methylierungsanalyse von *CD276* sind aus denjenigen ausgewählt, welche für diesen Zweck in den Ausführungsbeispielen 13, 15, 16 und 17 beschrieben sind. Für die erfindungsgemäße mRNA-Expressionsanalyse von *CD276* eignet sich der Nachweis zumindest eines Teils der Sequenz von Transkripten, deren cDNA zumindest teilweise den Sequenzen SEQ ID NO:241, SEQ ID NO:242, SEQ ID NO:243, SEQ ID NO:244, SEQ ID NO:245, SEQ ID NO:246, SEQ ID NO:247, SEQ ID NO:248, SEQ ID NO:249, SEQ ID NO:250, SEQ ID NO:251, SEQ ID NO:251 und/oder SEQ ID NO:253 entspricht. Eine bevorzugte Verfahrensausführung umfasst die Anwendung der DNA-Methylierungsanalyse des *CD276* Gens zur Bestimmung des Ansprechens auf einen Wirkstoff, der geeignet ist die immunregulatorische Wirkung von *CD276* zu verändern, insbesondere zu inhibieren. Geeignete Wirkstoffe sind beispielsweise Enoblituzumab (MGA271, Mocrogenics) und MGD009 (Macrogenics).

Das Gen *C10orf54* beziehungsweise *chromosome 10 open reading frame* 54 wird auch als *B7H5, GI24, PP2135, VISTA, B7-H5, DD1alpha* und *SISP1* bezeichnet. Bevorzugte Bereiche für die DNA-Methylierungsanalyse von *C10orf54* sind beispielsweise in dem Bereich, der für die Transkripte codiert (10:71747559-71773498, SEQ ID NO:188) sowie Promotoren (10:71759805-71784691, SEQ ID NO:189 und 10:71723534-71753287, SEQ ID NO:191) und/oder einer CTCF-Bindestelle (10:71738936-71750940, SEQ ID NO:190) enthalten. Weitere bevorzugte Bereiche für die DNA-Methylierungsanalyse von *C10orf54* sind aus denjenigen ausgewählt, welche für diesen Zweck in den Ausführungsbeispielen 11, 14 und 17 beschrieben sind. Für die erfindungsgemäße mRNA-Expressionsanalyse von *C10orf54* eignet sich der Nachweis zumindest eines Teils der Sequenz von Transkripten, deren cDNA zumindest teilweise den Sequenzen SEQ ID NO:192, SEQ ID NO:193 und/oder SEQ ID NO:194 entspricht. Eine bevorzugte Verfahrensausführung umfasst die Anwendung der DNA-Methylierungsanalyse des *C10orf54* Gens zur Bestimmung des Ansprechens auf einen Wirkstoff, welcher geeignet ist, die immunregulatorische Wirkung von *C10orf54* zu verändern, insbesondere zu inhibieren. Geeignete Inhibitoren sind beispielsweise CA-170 (Curis) und JNJ-61610588 (Janssen Biotech, Inc.).

Das Gen *HHLA2* beziehungsweise *HERV-H LTR-associating 2* ist auch als *B7-H7, B7y, B7H7* und *B7-H5* bekannt. Bevorzugte Bereiche für die DNA-Methylierungsanalyse von *HHLA2* sind beispielsweise in dem Bereich, der für die Transkripte codiert (3:108296490-108378285) sowie einem regulatorischen Bereich mit Enhancern, Promotor und CTCF-Bindestellen (3:108291804-108313328, SEQ ID NO:36) und zwei weiteren Promotoren (3:108310655-108313476, SEQ ID NO:195 und 3:108341108-108352706, SEQ ID NO:196) enthalten. Weitere bevorzugte Bereiche für die DNA-Methylierungsanalyse von *HHLA2* sind aus denjenigen ausgewählt, welche für diesen Zweck in den Ausführungsbeispielen 11, 14, 15 und 16 beschrieben sind. Für die erfindungsgemäße mRNA-Expressionsanalyse von *HHLA2* eignet sich der Nachweis zumindest eines Teils der Sequenz von Transkripten, deren cDNA zumindest teilweise den Sequenzen SEQ ID NO:197, SEQ ID NO:198, SEQ ID NO:199, SEQ ID NO:200, SEQ ID NO:201, SEQ ID NO:202, SEQ ID NO:203, SEQ ID NO:204, SEQ ID NO:205, SEQ ID NO:206 und/oder SEQ ID NO:207 entspricht. Eine bevorzugte Verfahrensausführung umfasst die Anwendung der DNA-Methylierungsanalyse des *HHLA2* Gens zur Bestimmung des Ansprechens auf einen Wirkstoff, welcher geeignet ist, die immunregulatorische Wirkung von *HHLA2* zu verändern, insbesondere zu inhibieren.

Das Gen *NCR3LG1* beziehungsweise *natural killer cell cytotoxicity receptor 3 ligand 1* ist auch als *B7H6, DKFZp686024166* und *B7-H6* bekannt. *NCR3LG1* ist ein immunregulatorisches Gen, dessen Produkt zu den B7-Proteinen gehört und ein erfindungsgemäßer Immun-Checkpoint ist. Bevorzugte Bereiche für die DNA-Methylierungsanalyse von *NCR3LG1* sind beispielsweise in dem Bereich, der für die Transkripte codiert (11:17351726-17377341, SEQ ID NO:208) sowie dem Promotor (11:17347524-17359073, SEQ ID NO:209) und/oder einer CpG-Insel (11:17351068-17354459, SEQ ID NO:210) enthalten. Für die erfindungsgemäße mRNA-Expressionsanalyse von *NCR3LG1* eignet sich der Nachweis zumindest eines Teils der Sequenz von Transkripten, deren cDNA zumindest teilweise SEQ ID NO:254 und/oder SEQ ID NO:255 entspricht. Eine bevorzugte Verfahrensausführung umfasst die Anwendung der DNA-Methylierungsanalyse des *NCR3LG1* Gens zur Bestimmung des Ansprechens auf einen Wirkstoff, welcher geeignet ist, die immunregulatorische Wirkung von *NCR3LG1* und/oder den durch *NCR3* codierten Rezeptor zu verändern.

Das Gen *CD86* beziehungsweise *CD86 molecule* ist auch als *B7-2, B70, LAB72, B7.2* und *CD28LG2* bekannt. Der durch *CD86* codierte Ligand gehört zu den B7-Proteinen und ist ein erfindungsgemäßer Immun-Checkpoint, welcher beispielsweise an die durch *CD28* und *CTLA4* codierten Rezeptoren binden kann. Bevorzugte Bereiche für die DNA-Methylierungsanalyse von *CD86* sind beispielsweise in dem Bereich, welcher für die Transkripte codiert (3:122055366-122121139), den Promotoren (3:122071817-122084231, SEQ ID NO:211; 3:122053987-122061537, SEQ ID NO:212; 3:122081939-122095873, SEQ ID NO:213; 3:122096457-122110191, SEQ ID NO:214) und/oder dem Enhancer (3:122119408-122125888, SEQ ID NO:215) enthalten. Weitere bevorzugte Bereiche für die Methylierungsanalyse von *CD86* sind aus denjenigen ausgewählt, welche für diesen Zweck in dem Ausführungsbeispiel 17 beschrieben sind. Für die erfindungsgemäße mRNA-Expressionsanalyse von *CD86* eignet sich der Nachweis zumindest eines Teils der Sequenz von Transkripten, deren cDNA zumindest teilweise SEQ ID NO:256, SEQ ID NO:257, SEQ ID NO:258, SEQ ID NO:259, SEQ ID NO:260, SEQ ID NO:261, SEQ ID NO:262, SEQ ID NO:263 und/oder SEQ ID NO:264 entspricht. Eine bevorzugte Verfahrensausführung umfasst die Anwendung der DNA-Methylierungsanalyse des *CD86* Gens zur Bestimmung des Ansprechens auf einen Wirkstoff, welcher geeignet ist, die immunregulatorische Wirkung des durch *CD86* codierten Liganden und/oder die durch *CTLA4* und/oder *CD28* codierten Rezeptoren zu inhibieren. Geeignete Wirkstoffe sind beispielsweise Ipilimumab (MDX-010; Handelsname Yervoy, Bristol-Myers Squibb), Tremelimumab (ticilimumab, CP-675,206, Pfizer, MedImmune, Astra Zeneca).

Das Gen *VTCN1* beziehungsweise *V-set domain containing T-cell activation inhibitor 1* ist auch als *B7-H4, B7H4, B7S1, B7X, B7h.5, PRO1291* und *VCTN1* bekannt. Das durch *VTCN1* codierte Protein gehört zu den B7-Proteinen und ist ein erfindungsgemäßer Immun-Checkpoint. Bevorzugte Bereiche für die DNA-Methylierungsanalyse von *VTCN1* sind beispielsweise in dem Bereich, welcher für die Transkripte codiert (1:117143587-117210960) und/oder den Promotor (1:117193851-117226364) enthält. Für die erfindungsgemäße mRNA-Expressionsanalyse von *VTCN1* eignet sich der Nachweis zumindest eines Teils der Sequenz von Transkripten, deren cDNA zumindest teilweise SEQ ID NO:377, SEQ ID NO:378 und/oder SEQ ID NO:379 entspricht. Eine bevorzugte Verfahrensausführung umfasst die Anwendung der DNA-Methylierungsanalyse des *VTCN1* Gens zur Bestimmung des Ansprechens auf einen Wirkstoff, welcher geeignet ist, die immunregulatorische Wirkung des durch *VTCN1* codierten Liganden und/oder seines Rezeptors zu inhibieren.

Die immunregulatorischen Gene *KIR2DL4, KIR3DL1, KIR3DL3, LAG3* und *CD160* codieren für Proteine, welche MHC:Peptid-Komplex bindenden Co-Rezeptoren sind. *KIR2DL4, KIR3DL1, KIR3DL3, LAG3* und *CD160* sind Immun-Checkpoints im Sinne der Erfindung und gehören zur Immunglobulin-Superfamilie. Die KIR-Gene *KIR2DL4, KIR3DL1* und *KIR3DL3* codieren für *killer cell immunoglobulin-like receptors* (kurz KIR oder KIR-Rezeptoren). Das Gen *KIR2DL4* beziehungsweise *killer cell immunoglobulin-like receptor, two domains, long cytoplasmic tail,* 4 ist beispielsweise auch als *KIR103, KIR103AS, G9P, CD158D, 15.212, KIR-103AS* und *103AS* bekannt. Bevorzugte Bereiche für die DNA-Methylierungsanalyse von *KIR2DL4* sind beispielsweise im Bereich, welcher für die Transkripte codiert (19:54803535-54814517, SEQ ID NO:216), in den Promotoren (19:54799627-54807083, SEQ ID NO:217; 19:54791651-54800236, SEQ ID NO:229) und/oder zwei Enhancern (19:54803283-54804114, SEQ ID NO:218 und 19:54812823-54819942, SEQ ID NO:219) enthalten. Weitere bevorzugte Bereiche für die Methylierungsanalyse von *KIR2DL4* sind aus denjenigen ausgewählt, welche für diesen Zweck in den Ausführungsbeispielen 11, 14 und 17 beschrieben sind. Für die erfindungsgemäße mRNA-Expressionsanalyse von *KIR2DL4* eignet sich der Nachweis zumindest eines Teils der Sequenz von Transkripten, deren cDNA zumindest teilweise SEQ ID NO:220, SEQ ID NO:221, SEQ ID NO:222, SEQ ID NO:223, SEQ ID NO:224, SEQ ID NO:225, SEQ ID NO:226, SEQ ID NO:227 und/oder SEQ ID NO:228 entspricht. Eine bevorzugte Verfahrensausführung umfasst die Anwendung der DNA-Methylierungsanalyse des *KIR2DL4* Gens zur Bestimmung des Ansprechens auf einen Wirkstoff, welcher geeignet ist, die immunregulatorische Wirkung des durch *KIR2DL4* codierten Rezeptors zu inhibieren. Ein geeigneter Wirkstoff ist beispielsweise Lirilumab (Bristol-Myers Squibb).

Das Gen *KIR3DL1* beziehungsweise *killer cell immunoglobulin-like receptor, three domains, long cytoplasmic tail,* 1 ist auch als *NKB1, CD158E, cl-11, cl-2, CD158e2, NKAT3, AMB11, NKB1B, KIR, KIR3DL2, CD158E1, CD158e1*/*2, NKAT-3, nkat3* und *KIR3DL1*/*S1* bekannt. Bevorzugte Bereiche für die DNA-Methylierungsanalyse von *KIR3DL1* sind beispielsweise im Bereich, der für die Transkripte codiert (19:54816468-54866993) sowie dem Promotor (19:54808180-54831092, SEQ ID NO:230) und/oder zwei Enhancern (19:54812749-54821037, SEQ ID NO:231 und 19:54835785-54863422, SEQ ID NO:232) enthalten. Weitere bevorzugte Bereiche für die DNA-Methylierungsanalyse von *KIR3DL1* sind aus denjenigen ausgewählt, welche für diesen Zweck in den Ausführungsbeispielen 11 und 14 beschrieben sind. Für die erfindungsgemäße mRNA-Expressionsanalyse von *KIR3DL1* eignet sich der Nachweis zumindest eines Teils der Sequenz von Transkripten, deren cDNA zumindest teilweise SEQ ID NO:233, SEQ ID NO:234, SEQ ID NO:235 und/oder SEQ ID NO:236 entspricht. Eine bevorzugte Verfahrensausführung umfasst die Anwendung der Methylierungsanalyse des *KIR3DL1* Gens zur Bestimmung des Ansprechens auf einen Wirkstoff, welcher geeignet ist, die immunregulatorische Wirkung des durch *KIR3DL1* codierten Rezeptors zu inhibieren, beispielsweise Lirilumab (auch bekannt als IPH2102/BMS-986015 von Bristol-Myers Squibb und Innate Pharma) und IPH4102 (Innate Pharma).

Das Gen *KIR3DL3* beziehungsweise *killer cell immunoglobulin-like receptor, three domains, long cytoplasmic tail, 3* ist auch als *KIR2DS2, CD158z, KIR3DL7, KIR44, CD158Z* und *KIRC1* bekannt. Bevorzugte Bereiche für die DNA-Methylierungsanalyse von *KIR3DL3* sind im Bereich, der für die Transkripte codiert (19:54724479-54736536, SEQ ID NO:237) sowie in dem Promotor (19:54723156-54728908, SEQ ID NO:238) und/oder zwei Enhancern (19:54723473-54725230, SEQ ID NO:239 und 19:54735023-54744608, SEQ ID NO:240) enthalten. Für die erfindungsgemäße mRNA-Expressionsanalyse von *KIR3DL3* eignet sich der Nachweis zumindest eines Teils der Sequenz von Transkripten, deren cDNA zumindest teilweise SEQ ID NO:265 entspricht. Eine bevorzugte Verfahrensausführung umfasst die Anwendung der DNA-Methylierungsanalyse des *KIR3DL3* Gens zur Bestimmung des Ansprechens auf einen Wirkstoff, welcher geeignet ist, die immunregulatorische Wirkung des durch *KIR3DL3* codierten Rezeptors zu inhibieren, beispielsweise Lirilumab (auch bekannt als IPH2102/BMS-986015 von Bristol-Myers Squibb und Innate Pharma).

Das Gen *LAG3* beziehungsweise *lymphocyte-activation gene 3* ist beispielsweise auch *FDC* und *CD223* benannt. Bevorzugte Bereiche für die Methylierungsanalyse von *LAG3* sind beispielsweise der Bereich, der für die Transkripte codiert (12:6772512-6778455, SEQ ID NO:161) sowie der Promotor (12:6770333-6774801, SEQ ID NO:349), ein alternativer Promotor mit CTCF-Bindestelle (12:6777768-6781320, SEQ ID NO:350) sowie ein Enhancer (12:6774685-6778062, SEQ ID NO:351). Weitere bevorzugte Bereiche für die DNA-Methylierungsanalyse von *LAG3* sind aus denjenigen ausgewählt, welche für diesen Zweck in den Ausführungsbeispielen 4, 7, 8, 9 und 14 beschrieben sind. Für die erfindungsgemäße Bestimmung der mRNA von *LAG3* werden beispielsweise die Sequenzen oder Teilbereiche der Sequenzen SEQ ID NO:352, SEQ ID NO:353, SEQ ID NO:354 und SEQ ID NO:355 bevorzugt. Eine bevorzugte Verfahrensausführung ist die Anwendung der Methylierungsanalyse des *LAG3* Gens zur Bestimmung des Ansprechens auf einen Inhibitor, der geeignet ist die immunregulatorische Wirkung des durch *LAG3* codierten Rezeptors zu inhibieren. Geeignete Inhibitoren sind beispielsweise *MGD013 (Macrogenics)* und *BMS-986016 (Bristol-Myers Squibb).*

Das Gen *CD160* beziehungsweise *CD160 molecule* wird auch als *BY55, NK28* und *NK1* bezeichnet. Bevorzugte Bereiche für die DNA-Methylierungsanalyse von *CD160* sind beispielsweise in dem Bereich, der für die Transkripte codiert (1:145719471-145739288, SEQ ID NO:361) sowie dem Promotor (1:145718367-145722818, SEQ ID NO:362), einem alternativen Promotor (1:145735798-145741393, SEQ ID NO:364), einer CTCF-Bindestelle (1:145720204-145722255, SEQ ID NO:363) und/oder zwei Enhancern (1:145727546-145735069, SEQ ID NO:365 und 1:145708511-145719786, SEQ ID NO:366) enthalten. Weitere bevorzugte Bereiche für die DNA-Methylierungsanalyse von *CD160* sind aus denjenigen ausgewählt, welche für diesen Zweck in den Ausführungsbeispielen 11, 14 und 17 beschrieben sind. Für die erfindungsgemäße mRNA-Expressionsanalyse von *CD160* eignet sich der Nachweis zumindest eines Teils der Sequenz von Transkripten, deren cDNA zumindest teilweise den SEQ ID NO:356, SEQ ID NO:357, SEQ ID NO:358, SEQ ID NO:359 und/oder SEQ ID NO:360 entspricht. Eine bevorzugte Verfahrensausführung umfasst die Anwendung der DNA-Methylierungsanalyse des *CD160* Gens zur Bestimmung des Ansprechens auf einen Wirkstoff, welcher geeignet ist, die immunregulatorische Wirkung des durch *CD160* codierten Rezeptors zu verändern, insbesondere zu inhibieren.

Die immunregulatorischen Gene *TIGIT, BTLA, HAVCR2, BTNL2* und *CD48* codieren für die Proteine TIGIT, BTLA, HAVCR2, BTLN2 und CD48, welche zu den Mitgliedern der Immunglobulin-Superfamilie gehören und Immun-Checkpoints im Sinne der Erfindung sind.

Das Gen *TIGIT* beziehungsweise *T-cell immunoreceptor with Ig and ITIM domains* ist beispielsweise auch *DKFZp667A205, VSIG9, VSTM3, FLJ39873* und *WUCAM* bekannt. Bevorzugte Bereiche für die DNA-Methylierungsanalyse von *TIGIT* sind beispielsweise von dem Bereich, der für die Transkripte codiert (3:114276913-114310288) sowie drei regulatorischen Bereichen mit Enhancern und CTCF-Bindestellen (3:114273873-114278448, SEQ ID NO:266; 3:114306113-114321848, SEQ ID NO:267; 3:114288458-114302904, SEQ ID NO:268) umfasst. Weitere bevorzugte Bereiche für die DNA-Methylierungsanalyse von *TIGIT* sind aus denjenigen ausgewählt, welche für diesen Zweck in den Ausführungsbeispielen 4, 7, 8, 11, 14, 15, 16 und 17 beschrieben sind. Für die erfindungsgemäße mRNA-Expressionsanalyse von *TIGIT* eignet sich der Nachweis zumindest eines Teils der Sequenz von Transkripten, deren cDNA zumindest teilweise SEQ ID NO:269, SEQ ID NO:270, SEQ ID NO:271, SEQ ID NO:272, SEQ ID NO:273, SEQ ID NO:274 und/oder SEQ ID NO:275 entspricht. Eine bevorzugte Verfahrensausführung beinhaltet die Anwendung der Methylierungsanalyse des *TIGIT* Gens zur Bestimmung des Ansprechens auf einen Wirkstoff, welcher geeignet ist, die immunregulatorische Wirkung von *TIGIT* zu verändern, insbesondere zu inhibieren.

Das Gen *BTLA* beziehungsweise *B and T lymphocyte associated* ist auch als *CD272* und *BTLA1* bekannt. Bevorzugte Bereiche für die DNA-Methylierungsanalyse von *BTLA* sind beispielsweise in dem Bereich, der für die Transkripte codiert (3:112463968-112499561) sowie dem Promotor (3:112494210-112503447, SEQ ID NO:276), einem alternativen Promotor (3:112460682-112487674, SEQ ID NO:279) und/oder dem Enhancer (3:112501618-112513121, SEQ ID NO:277; 3:112486680-112495554, SEQ ID NO:278; 3:112451208-112463106, SEQ ID NO:280; 3:112508297-112522457, SEQ ID NO:281) enthalten. Weitere bevorzugte Bereiche für die DNA-Methylierungsanalyse von *BTLA* sind aus denjenigen ausgewählt, welche für diesen Zweck in den Ausführungsbeispielen 4, 6, 7, 8, 11 und 14 beschrieben sind. Für die erfindungsgemäße mRNA-Expressionsanalyse von *BTLA* eignet sich der Nachweis zumindest eines Teils der Sequenz von Transkripten, deren cDNA zumindest teilweise SEQ ID NO:282, SEQ ID NO:283, SEQ ID NO:284 und/oder SEQ ID NO:285 entspricht. Eine bevorzugte Verfahrensausführung umfasst die Anwendung der DNA-Methylierungsanalyse des *BTLA* Gens zur Bestimmung des Ansprechens auf einen Wirkstoff, welcher geeignet ist, die immunregulatorische Wirkung von *BTLA* zu verändern, insbesondere zu inhibieren.

Das Gen *HAVCR2* beziehungsweise *hepatitis A virus cellular receptor 2* wird auch als *TIMD-3, CD366, Tim-3, TIMD3, FLJ14428, TIM3, HAVcr-2* und *KIM-3* bezeichnet. Bevorzugte Bereiche für die DNA-Methylierungsanalyse von *HAVCR2* sind vom Bereich, der für die Transkripte codiert (5:157085832-157142869) sowie dem Promotor (5:157138356-157147783, SEQ ID NO:286), einem alternativen Promotor (5:157097106-157121624, SEQ ID NO:287) sowie drei Enhancern (5:157144698-157161450, SEQ ID NO:288; 5:157116666-157139926, SEQ ID NO:289 und 5:157080879-157096259, SEQ ID NO:290) umfasst. Weitere bevorzugte Bereiche für die DNA-Methylierungsanalyse von *HAVCR2* sind aus denjenigen ausgewählt, welche für diesen Zweck in den Ausführungsbeispielen 11 und 14 beschrieben sind. Für die erfindungsgemäße mRNA-Expressionsanalyse von *HAVCR2* eignet sich der Nachweis zumindest eines Teils der Sequenz von Transkripten, deren cDNA zumindest teilweise SEQ ID NO:291, SEQ ID NO:292, SEQ ID NO:293, SEQ ID NO:294, SEQ ID NO:295 und/oder SEQ ID NO:296 entspricht. Eine bevorzugte Verfahrensausführung beinhaltet die Anwendung der DNA-Methylierungsanalyse des *HAVCR2* Gens zur Bestimmung des Ansprechens auf einen Wirkstoff, welcher geeignet, ist die immunregulatorische Wirkung von *HAVCR2* zu verändern, beispielsweise zu inhibieren. Ein geeigneter Inhibitor ist Anti-TIM-3 Antibody TSR-022 (Tesaro, Inc).

Das Gen *BTNL2* beziehungsweise *butyrophilin like* 2 wird auch als *BTL-II, BTN7, HSBLMHC1* und *SS2* bezeichnet. Bevorzugte Bereiche für die DNA-Methylierungsanalyse von *BTNL2* umfassen den Bereich, welcher für die Transkripte codiert (6:32393963-32407128, SEQ ID NO:387) sowie den Bereich mit Promotor und regulatorischen Elementen (6:32387381-32413712, SEQ ID NO:387). Weitere bevorzugte Bereiche für die DNA-Methylierungsanalyse von *BTNL2* sind aus denjenigen ausgewählt, welche für diesen Zweck im Ausführungsbeispiel 17 beschrieben sind. Für die erfindungsgemäße mRNA-Expressionsanalyse von *BTNL2* eignet sich der Nachweis zumindest eines Teils der Sequenz von Transkripten, deren cDNA zumindest teilweise SEQ ID NO:389 entspricht. Eine bevorzugte Ausgestaltung umfasst die Anwendung der DNA-Methylierungsanalyse des *BTNL2* Gens zur Bestimmung des Ansprechens auf einen Wirkstoff, welcher geeignet ist, die immunregulatorische Wirkung von *BTNL2* zu verändern. Dies beinhaltet insbesondere ein Antagonisten, welcher geeignet ist, den durch *BTNL2* codierten Immuncheckpoint zu inhibieren.

Das Gen *CD48* beziehungsweise *CD48 molecule* wird auch als *BCM1, BLAST, BLAST1, MEM-102, SLAMF2, hCD48* und *mCD48* bezeichnet. Bevorzugte Bereiche für die DNA-Methylierungsanalyse von *CD48* umfassen den Promotor und regulatorische Elemente (1:160676386-160722581) sowie den Promotor (1:160703189-160716911, SEQ ID NO:390). Weitere bevorzugte Bereiche für die DNA-Methylierungsanalyse von *CD48* sind aus denjenigen ausgewählt, welche für diesen Zweck im Ausführungsbeispiel 17 beschrieben sind. Für die erfindungsgemäße mRNA-Expressionsanalyse von *CD48* eignet sich der Nachweis zumindest eines Teils der Sequenz von Transkripten, deren cDNA zumindest teilweise SEQ ID NO:389 entspricht. Eine bevorzugte Ausgestaltung umfasst die Anwendung der DNA-Methylierungsanalyse des *CD48* Gens zur Bestimmung des Ansprechens auf einen Wirkstoff, welcher geeignet ist, die immunregulatorische Wirkung von *CD48* zu verändern.

*CD40, TNFRSF9, TNFRSF18, TNFRSF4* und *CD27* sind immunregulatorische Gene, die für die Proteine CD40, TNFRSF9, TNFRSF18, TNFRSF4 und CD27 codieren. CD40, TNFRSF9, TNFRSF18, TNFRSF4 und CD27 sind Rezeptoren der Tumornekrosefaktor Rezeptor-Superfamilie, welche Immun-Checkpoints im Sinne der Erfindung sind.

Das Gen *CD40* beziehungsweise *CD40 molecule, TNF receptor superfamily member* 5 wird auch als *TNFRSF5, p50, Bp50* und *CDW40* bezeichnet. Bevorzugte Bereiche für die DNA-Methylierungsanalyse von *CD40* sind vom Bereich, welcher für die Transkripte codiert (20:46118272-46129863, SEQ ID NO:297) sowie dem Promotor (20:46115599-46122305, SEQ ID NO:298), einem alternativen Promotor (20:46106713-46115878, SEQ ID NO:299) und/oder dem Enhancer (20:46121274-46143091, SEQ ID NO:300) umfasst. Weitere bevorzugte Bereiche für die DNA-Methylierungsanalyse von *CD40* sind aus denjenigen ausgewählt, welche für diesen Zweck in den Ausführungsbeispielen 4, 7, 8, 9 und 14 beschrieben sind. Für die erfindungsgemäße mRNA-Expressionsanalyse von *CD40* eignet sich der Nachweis zumindest eines Teils der Sequenz von Transkripten, deren cDNA zumindest teilweise SEQ ID NO:301, SEQ ID NO:302, SEQ ID NO:303, SEQ ID NO:304, SEQ ID NO:305, SEQ ID NO:306 und/oder SEQ ID NO:307 entspricht. Eine bevorzugte Ausgestaltung umfasst die Anwendung der DNA-Methylierungsanalyse des *CD40* Gens zur Bestimmung des Ansprechens auf einen Wirkstoff, welcher geeignet ist, die immunregulatorische Wirkung von *CD40* zu verändern. Dies beinhaltet insbesondere einen Agonisten, welcher geeignet ist, den durch *CD40* codierten Rezeptor zu aktivieren, wie beispielsweise CP-870,893 (Pfizer, VLST), Dacetuzumab (Seattle Genetics), Chi Lob 7/4 (University of Southampton). Insbesondere kann der Wirkstoff auch derart gewählt sein, dass er in der Lage ist, die immunregulatorische Wirkung von CD40 zu inhibieren. Ein geeigneter Wirkstoff ist beispielsweise Lucatumumab (Novartis).

Das Gen *TNFRSF9* beziehungsweise *tumor necrosis factor receptor superfamily member* 9 ist auch als *4-1BB, CDw137, CD137* und *ILA* bekannt. Bevorzugte Bereiche für die DNA-Methylierungsanalyse von *TNFRSF9* sind in dem Bereich, welcher für die Transkripte codiert (1:7915894-7943165, SEQ ID NO:308) sowie dem Promotor (1:7937419-7950341, SEQ ID NO:309), einem regulatorischen Element mit CTCF-Bindestelle, Enhancer und alternativem Promotor (1:7910016-7923298, SEQ ID NO:312), dem Enhancer (1:7922095-7939183, SEQ ID NO:310) und/oder einer CpG-Insel (1:7949284-7956816, SEQ ID NO:311) enthalten. Weitere bevorzugte Bereiche für die DNA-Methylierungsanalyse von *TNFRSF9* sind aus denjenigen ausgewählt, welche für diesen Zweck in den Ausführungsbeispielen 4, 7, 8, 11, 14, 15, 16 und 17 beschrieben sind. Für die erfindungsgemäße mRNA-Expressionsanalyse von *TNFRSF9* eignet sich der Nachweis zumindest eines Teils der Sequenz von Transkripten, deren cDNA zumindest teilweise SEQ ID NO:313, SEQ ID NO:314, SEQ ID NO:315 und/oder SEQ ID NO:316 entspricht. Eine bevorzugte Verfahrensausführung umfasst die Anwendung der DNA-Methylierungsanalyse des *TNFRSF9* Gens zur Bestimmung des Ansprechens auf einen Wirkstoff, welcher geeignet ist, die immunregulatorische Wirkung von *TNFRSF9* zu verändern, insbesondere einen Agonisten, welcher geeignet ist, den durch *TNFRSF9* codierten Rezeptor zu aktivieren. Geeignete Wirkstoffe sind PF-05082566 (PF-566, Pfizer) und Urelumab (BMS-663513, Bristol-Myers Squibb).

Das Gen *TNFRSF25* beziehungsweise *tumor necrosis factor receptor superfamily member* 25 wird auch als *WSL-LR, WSL, APO3, TNFRSF12, TR3, LARD, DDR3, TRAMP, WSL-1, DR3, WSL1* und *APO-3* bezeichnet. Bevorzugte Bereiche für die DNA-Methylierungsanalyse von *TNFRSF25* sind vom Bereich, welcher für die Transkripte codiert (1:6461151-6466195, SEQ ID NO:317) sowie einem alternativen Promotor (1:6458470-6463974, SEQ ID NO:318), zwei CpG-Inseln (1:6459713-6463353, SEQ ID NO:319; 1:6465602-6466846, SEQ ID NO:320) sowie einem weiteren Promotor (1:6464008-6468536, SEQ ID NO:321) und/oder einem Enhancer (1:6468908-6478190, SEQ ID NO:322) umfasst. Weitere bevorzugte Bereiche für die DNA-Methylierungsanalyse von *TNFRSF25* sind aus denjenigen ausgewählt, welche für diesen Zweck in den Ausführungsbeispielen 14 und 17 beschrieben sind. Für die erfindungsgemäße mRNA-Expressionsanalyse von *TNFRSF25* eignet sich der Nachweis zumindest eines Teils der Sequenz von Transkripten, deren cDNA zumindest teilweise SEQ ID NO:323, SEQ ID NO:324, SEQ ID NO:325, SEQ ID NO:326, SEQ ID NO:327 und/oder SEQ ID NO:328 entspricht. Eine bevorzugte Verfahrensausführung umfasst die Anwendung der DNA-Methylierungsanalyse des *TNFRSF25* Gens zur Bestimmung des Ansprechens auf einen Wirkstoff, welcher geeignet ist, die immunregulatorische Wirkung von *TNFRSF25* zu verändern. Besonders bevorzugt handelt es sich hierbei um einen Agonisten, welcher geeignet ist, den durch *TNFRSF25* codierten Rezeptor zu aktivieren.

Das Gen *TNFRSF18* beziehungsweise *tumor necrosis factor receptor superfamily member 18* ist auch als *GITR, AITR, CD357* und *GITR-D* bekannt. Bevorzugte Bereiche für die DNA-Methylierungsanalyse von *TNFRSF18* sind in dem Bereich, welcher für die Transkripte codiert (1:1203508-1206691, SEQ ID NO:329) und/oder dem Promotor (1:1195867-1210392, SEQ ID NO:330) enthalten. Für die erfindungsgemäße mRNA-Expressionsanalyse von *TNFRSF18* eignet sich der Nachweis zumindest eines Teils der Sequenz von Transkripten, deren cDNA zumindest teilweise SEQ ID NO:331, SEQ ID NO:332, SEQ ID NO:333 und/oder SEQ ID NO:334 entspricht. Weitere bevorzugte Bereiche für die Methylierungsanalyse von *TNFRSF18* sind aus denjenigen ausgewählt, welche für diesen Zweck in dem Ausführungsbeispiel 17 beschrieben sind. Eine bevorzugte Verfahrensausführung beinhaltet die Anwendung der DNA-Methylierungsanalyse des *TNFRSF18* Gens zur Bestimmung des Ansprechens auf einen Wirkstoff, welcher geeignet ist, die immunregulatorische Wirkung von *TNFRSF18* zu verändern. Dies betrifft insbesondere einen Agonisten, welcher geeignet ist, den durch *TNFRSF18* codierten Rezeptor zu aktivieren, beispielsweise TRX518 (GITR Inc.).

Das Gen *TNFRSF4* beziehungsweise *tumor necrosis factor receptor superfamily member 4* ist auch als *IMD16, OX40, TXGP1L, CD134* und *ACT35* bekannt. Bevorzugte Bereiche für die DNA-Methylierungsanalyse von *TNFRSF4* sind beispielsweise in dem Bereich, welcher für die Transkripte codiert (1:1211326-1214138, SEQ ID NO:335), dem Promotor (1:1209894-1215938, SEQ ID NO:336) und/oder einer CpG-Insel (1:1213505-1214288, SEQ ID NO:337) enthalten. Für die erfindungsgemäße mRNA-Expressionsanalyse von *TNFRSF4* eignet sich der Nachweis zumindest eines Teils der Sequenz von Transkripten, deren cDNA zumindest teilweise SEQ ID NO:338, SEQ ID NO:339 und/oder SEQ ID NO:340 entspricht. Weitere bevorzugte Bereiche für die Methylierungsanalyse von *TNFRSF4* sind aus denjenigen ausgewählt, welche für diesen Zweck in dem Ausführungsbeispiel 17 beschrieben sind. Eine bevorzugte Verfahrensausführung umfasst die Anwendung der DNA-Methylierungsanalyse des *TNFRSF4* Gens zur Bestimmung des Ansprechens auf einen Wirkstoff, welcher geeignet ist, die immunregulatorische Wirkung von *TNFRSF4* zu verändern. Besonders bevorzugt ist dabei ein Agonist, welcher geeignet ist, den durch *TNFRSF4* codierten Rezeptor zu aktivieren, wie beispielsweise MEDI6469 (9B12, MedImmune LLC, Astra Zeneca, AgonOx), Hu106-22 und Hu 119-122 (UTMDACC).

Das Gen *CD27* beziehungsweise *CD27 molecule* wird auch als *S152, T14, TNFRSF7, S152, LPFS2 oder Tp55* bezeichnet. Bevorzugte Bereiche für die DNA-Methylierungsanalyse von *CD27* sind beispielsweise vom Bereich, der für die Transkripte codiert (12:6444867-6451718, SEQ ID NO:341), zwei Promotoren (12:6442585-6448021, SEQ ID NO:342 und 12:6448982-6453895, SEQ ID NO:343) und/oder drei Enhancern (12:6453736-6456010, SEQ ID NO:344; 12:6447474-6449430, SEQ ID NO:345 und 12:6439173-6442795, SEQ ID NO:346) umfasst. Weitere bevorzugte Bereiche für die DNA-Methylierungsanalyse von *CD27* sind aus denjenigen ausgewählt, welche für diesen Zweck in den Beispielen 6 und 17 beschrieben sind. Für die mRNA-Expressionsanalyse von *CD27* eignet sich der Nachweis zumindest eines Teils der Sequenz von Transkripten, deren cDNA zumindest teilweise SEQ ID NO:347 und/oder SEQ ID NO:348 entspricht. Eine bevorzugte Verfahrensausführung beinhaltet die Anwendung der DNA-Methylierungsanalyse des *CD27* Gens zur Bestimmung des Ansprechens auf einen Wirkstoff, welcher geeignet ist, die immunregulatorische Wirkung von *CD27* zu verändern. Hierbei kann es sich insbesondere um einen Agonisten handeln, welcher geeignet ist, den durch *CD27* codierten Rezeptor zu aktivieren, beispielsweise Varlilumab (Celldex Therapeutics).

Das Gen *ADORA2A* ist ein immunregulatorisches Gen, welches für den Adenosin 2A-Rezeptor codiert. Der Adenosin 2A-Rezeptor ist ein Immun-Checkpoint im Sinne der Erfindung, der über eine Bindung des Metaboliten Adenosin immunregulatorisch wirkt. Das Gen *ADORA2A* beziehungsweise *adenosine A2a receptor* wird auch als *A2aR, ADORA2* oder *RDC8* bezeichnet. Bevorzugte Bereiche für die DNA-Methylierungsanalyse von *ADORA2A* sind beispielsweise in dem Bereich, welcher für die Transkripte codiert (22:24417879-24442360, SEQ ID NO:380), im Promotor mit einer CpG-Insel (22:24422358-24434469, SEQ ID NO:381), in einem Enhancer (22:24418577-24423493, SEQ ID NO:382) und in dem Bereich der für *ADORA2A* und dessen antisense RNA codiert (22:24420336-24501503) enthalten. Weitere bevorzugte Bereiche für die DNA-Methylierungsanalyse von *ADORA2A* sind aus denjenigen ausgewählt, die für diesen Zweck in dem Ausführungsbeispiel 17 beschrieben sind. Für die erfindungsgemäße mRNA-Expressionsanalyse von *ADORA2A* eignet sich der Nachweis zumindest eines Teils der Sequenz von Transkripten, deren cDNA zumindest teilweise SEQ ID NO:383, SEQ ID NO:384, SEQ ID NO:385 und/oder SEQ ID NO:386 entspricht. Eine bevorzugte Verfahrensausführung umfasst die Anwendung der DNA-Methylierungsanalyse des *ADORA2A* Gens zur Bestimmung des Ansprechens auf einen Wirkstoff, welcher geeignet ist, die immunregulatorische Wirkung des Adenosin 2A-Rezeptor zu verändern. Besonders bevorzugt ist dabei ein Antagonist, welcher geeignet ist, den durch *ADORA2A* codierten Rezeptor zu inhibieren, wie beispielsweise PBF-509, Istradefylline, ST1535, ST4206, Tozadenant, V81444, CPI-444, Preladenant, Vipadenant, SCH58261, ATL801.

Alternativ ist es möglich, die erfindungsgemäße DNA-Methylierungsanalyse eines immunregulatorischen Gens in Kombination mit anderen prognostischen und/oder prädiktiven Biomarkern durchzuführen. Insbesondere eignen sich hierfür Mutationen der Gene *BRAF* und *EGFR,* anhand derer ein Ansprechen auf Inhibitoren von BRAF und EGFR vorhergesagt werden kann. Es ist auch möglich die Methylierungsanalyse der immunregulatorischen Gene in Kombination mit einer Methylierungsanalyse und/oder einer Mutationsanalyse von Chemokinen und Chemokinrezeptoren, beispielsweise der Chemokinfamilien CXC und CX3C, durchzuführen. Beispielsweise kann dadurch eine Vorhersage des Ansprechens auf eine Therapie mit Ulocuplumab getroffen werden. Die erfindungsgemäße Methylierungsanalyse eines immunregulatorischen Gens kann auch mit einer Methylierungsanalyse der Gene *IL2RB, CXCL12, CXCR4* und *CXCR7* kombiniert werden.

Grundsätzlich kann die Abschätzung der Prognose auch vor, während oder nach einer immuntherapeutischen Behandlung des Patienten mit mindestens einem Wirkstoff durchgeführt werden, welcher dazu ausgelegt ist, die immunregulatorische Wirkung des Immun-Checkpoints zu verändern. Auch die Prädiktion kann das Ansprechen der malignen Erkrankung des Patienten auf eine Immuntherapie mit mindestens einem derartigen Wirkstoff umfassen. Die Veränderung der immunregulatorischen Wirkung kann dadurch zustande kommen, dass der Wirkstoff mit dem analysierten Immun-Checkpoint selbst oder dessen Rezeptor beziehungsweise Liganden wechselwirkt. Beispielsweise kann der Immun-Checkpoint den Liganden CD274 und/oder PDCD1LG2 umfassen und der Wirkstoff dazu ausgelegt sein, durch Wechselwirkung mit dem entsprechenden Rezeptor PDCD1 die immunregulatorische Wirkung von CD274 und/oder PDCD1LG2 zu verändern, inbesondere zu inhibieren, zum Beispiel indem der Wirkstoff die Bindung von CD274 beziehungsweise PDCD1LG2 an den PDCD1 Rezeptor blockiert. Insbesondere kommen als Wirkstoff sogenannte Immun-Checkpoint-Inhibitoren in Frage, welche in der Lage sind, die immunregulatorische Wirkung eines ko-inhibitorischen beziehungsweise anti-inflammatorischen Immun-Checkpoints zu reduzieren oder zu unterbinden. Beispiele für solche Immun-Checkpoint-Inhibitoren sind Nivolumab, Ipilimumab, Pembrolizumab und die weiteren bereits oben genannten Wirkstoffe. Eine andere Möglichkeit sind Wirkstoffe, welche die immunregulatorische Wirkung der erfindungsgegenständlichen Immun-Checkpoints aktivieren. Geeignete aktivierende Wirkstoffe umfassen die bereits oben genannten Wirkstoffe. Es ist auch möglich, anhand der DNA-Methylierungsanalyse verschiedener erfindungsgegenständlicher immunregulatorischer Gene eine Auswahl von Wirkstoffen zu treffen, für welche jeweils ein gutes Ansprechverhalten in der Immuntherapie angezeigt wird. Solche Kombinationstherapien sind häufig besonders wirksam, bedürfen aber aufgrund der mit ihnen verbundenen hohen Kosten einer sehr sorgfältigen Abwägung und daher einer besonders zuverlässigen Vorhersage des Ansprechverhaltens auf die einzelnen Wirkstoffe. Dies kann das erfindungsgemäße Verfahren nun erstmals leisten.

Hier knüpft als Referenz auch der zweite Aspekt der Erfindung an, nach dem ein Verfahren zur individualisierten Auswahl eines Wirkstoffs zur immuntherapeutischen Behandlung eines Patienten mit einer malignen Erkrankung offenbart wird. Das Verfahren ist wiederum dadurch charakterisiert, dass eine DNA-Methylierungsanalyse mindestens eines immunregulatorischen Gens von Zellen der malignen Erkrankung und/oder von mit den Zellen der malignen Erkrankung wechselwirkenden T-Lymphozyten durchgeführt wird, wobei das immunregulatorische Gen für einen Immun-Checkpoint ausgewählt aus B7-Proteinen und deren Rezeptoren (das heißt Rezeptoren, welche B7-Proteine binden), MHC:Peptid-Komplex bindenden Co-Rezeptoren, den Mitgliedern der Tumornekrosefaktor-Rezeptor-Superfamilie TNFRSF9, CD40, TNFRSF4, TNFRSF18 und CD27, den Mitgliedern der Immunglobulin-Superfamilie TIGIT, BTLA, HAVCR2, BTNL2 und CD48 und dem Adenosin-bindenden Adenosin 2A-Rezeptor codiert und auf der Grundlage des Ergebnisses der DNA-Methylierungsanalyse der Wirkstoff ausgewählt wird.

Der Wirkstoff kann wiederum so gewählt werden, dass er die immunregulatorische Wirkung des Immun-Checkpoints verändern kann, also beispielsweise als Immun-Checkpoint-Inhibitor in der Lage ist, die immunregulatorische Wirkung eines ko-inhibitorischen beziehungsweise anti-inflammatorischen Immun-Checkpoints zu reduzieren oder zu unterbinden oder als Immun-Checkpoint-Agonist in der Lage ist, die immunregulatorische Wirkung eines ko-stimulatorischen beziehungsweise proinflammatorischen Immun-Checkpoints zu verstärken.

Insbesondere wird der Wirkstoff ausgewählt, wenn die DNA-Methylierungsanalyse eine Expression des entsprechenden Immun-Checkpoints in den Zellen der malignen Erkrankung und/oder in den T-Lymphozyten anzeigt, da dessen Expression den Angriffspunkt für die immuntherapeutische Behandlung mit dem Wirkstoff liefert und folglich auch das Ansprechen auf eine entsprechende Immuntherapie hinreichend wahrscheinlich ist.

Der dritte Aspekt der Erfindung betrifft mithin die Verwendung einer DNA-Methylierungsanalyse mindestens eines immunregulatorischen Gens von Zellen einer malignen Erkrankung eines Patienten und/oder von mit den Zellen der malignen Erkrankung wechselwirkenden T-Lymphozyten, wobei die maligne Erkrankung eine Krebserkrankung ist, zur Prädiktion des Ansprechens des Patienten auf eine Immuntherapie und/oder zur individualisierten Auswahl eines Wirkstoffs zur immuntherapeutischen Behandlung des Patienten, wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern. Auch hier codiert das immunregulatorische Gen für einen Immun-Checkpoint ausgewählt aus B7-Proteinen deren Rezeptoren (das heißt Rezeptoren, welche B7-Proteine binden) und ist ausgewählt aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2,* sowie beliebigen Kombinationen davon, wobei der Wirkstoff ausgewählt wird, wenn die DNA-Methylierungsanalyse eine Expression des Immun-Checkpoints in den Zellen der malignen Erkrankung und/oder in den T-Lymphozyten anzeigt.

Der vierte Aspekt der Erfindung betrifft die Verwendung von Anwesenheit, Abwesenheit oder Ausmaß einer Methylierung von mindestens einem CpG-Dinukleotid eines immunregulatorischen Gens von Zellen einer malignen Erkrankung und/oder von mit den Zellen der malignen Erkrankung wechselwirkenden T-Lymphozyten, wobei die maligne Erkrankung eine Krebserkrankung ist, als prädiktiver Biomarker zur Vorhersage des Ansprechens der malignen Erkrankung auf eine Immuntherapie beziehungsweise als Biomarker zur individualisierten Auswahl eines Wirkstoffs zur immuntherapeutischen Behandlung des Patienten, wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern, wobei das immunregulatorische Gen wiederum für einen Immun-Checkpoint ausgewählt aus B7-Proteinen und deren Rezeptoren (also Rezeptoren, welche B7-Proteine binden) codiert und ausgewählt ist aus der Gruppe bestehend aus den für B7-Proteine und deren Rezeptor codierenden Genen *CD274, PDCD1LG2* und *PDCD1,* den für das B7-Protein und dessen Rezeptor codierenden Genen *CD80* und *CTLA4,* dem für den Rezeptor eines B7-Proteins codierenden Gen *ICOS* und den für die B7-Proteine codierenden Genen *CD276, C10orf54* und *HHLA2,* sowie beliebigen Kombinationen davon, wobei der Wirkstoff ausgewählt wird, wenn die Methylierung des mindestens einen CpG-Dinukleotids eine Expression des Immun-Checkpoints in den Zellen der malignen Erkrankung und/oder in den T-Lymphozyten anzeigt.

Im Übrigen entsprechen die weiteren Merkmale und bevorzugten Ausgestaltungen des zweiten, dritten und vierten Aspektes, soweit anwendbar, denen des ersten Aspektes.

Gemäß dem fünften Aspekt der Erfindung wird eine Verwendung eines Kits zur Durchführung des Verfahrens nach dem ersten oder zweiten Aspekt bereitgestellt. Das Kit umfasst mindestens ein Oligonukleotid-Paar für die DNA-Methylierungsanalyse, wobei das Oligonukleotid-Paar dazu ausgelegt ist, an eine Sequenz des immunregulatorischen Gens in DNA von den Zellen der malignen Erkrankung und/oder von den T-Lymphozyten zu hybridisieren, nachdem in der DNA enthaltene Cytosine in Uracil oder eine andere Base mit einem von Cytosin unterscheidbaren Basenpaarungsverhalten und/oder Molekulargewicht umgewandelt wurden, um die Sequenz zu amplifizieren und/oder zu detektieren.

Das Kit kann auch ein oder mehrere weitere Oligonukleotid-Paare enthalten, welche jeweils dazu ausgelegt sind, an weitere Sequenzen desselben immunregulatorischen Gens oder anderer immunregulatorischer Gene in der umgewandelten DNA zu hybridisieren, um die weiteren Sequenzen für die DNA-Methylierungsanalyse zu amplifizieren und/oder zu detektieren.

Weiterhin kann das Kit mindestens ein Oligonukleotid-Paar für die mRNA-Expressionsanalyse umfassen, wobei das Oligonukleotid-Paar dazu ausgelegt ist, an eine Sequenz der mRNA oder einer aus der mRNA generierten cDNA von mindestens einer Transkriptvariante des immunregulatorischen Gens von den Zellen der malignen Erkrankung und/oder von den T-Lymphozyten zu hybridisieren, um die Sequenz zu amplifizieren und/oder zu detektieren.

Bevorzugte Bereiche und Sequenzen des immunregulatorischen Gens, welche mithilfe der Oligonukleotid-Paare zu amplifizieren und/oder zu detektieren sind, entsprechen denen aus dem ersten Aspekt.

Das Kit umfasst vorzugsweise eine Gebrauchsanweisung für die Durchführung des Verfahrens nach dem ersten und/oder zweiten Aspekt und/oder für die Verwendung nach dem dritten oder vierten Aspekt.

### Detaillierte Beschreibung von Ausführungsbeispielen

Nachfolgend wird die Erfindung anhand von Ausführungsbeispielen und experimentellen Ergebnissen näher beschrieben. Diese Ausführungsbeispiele dienen der Erläuterung und nicht der Beschränkung auf spezifische Details.

### Beispiel 1: Bestimmung der Prognose eines Patienten mit einem Prostatakarzinom nach radikaler Ektomie anhand der DNA-Methylierungsanalyse des immunregulatorischen Gens PDCD1

Im Rahmen der erfindungsgemäßen Verfahrensausführung kann die Bestimmung der Prognose eines Patienten mit einem lokal begrenzten Prostatakarzinom erfolgen, zum Beispiel nachdem eine radikale Entfernung (radikale Ektomie) der Prostata und des in der Prostata befindlichen Tumors durchgeführt wurde.

Zunächst wurde die DNA aus dem Tumor gewonnen. Hierzu eignete sich zum Beispiel eine Lyse von Tumorgewebe mittels Proteinase K mit anschließender Extraktion der DNA mit Silika-Zentrifugationssäulen. Für die Gewinnung von DNA aus Tumoren bieten sich kommerziell erhältliche Kits an, wie beispielsweise der QIAamp DNA Mini Kit (Qiagen N.V., Hilden, Deutschland). Die extrahierte DNA wurde im Anschluß mittels Bisulfit konvertiert, so dass im Wesentlichen alle Cytosine zu Uracil desaminiert wurden, während methylierte Cytosine unverändert blieben. Anschließend wurde die DNA-Methylierungsanalyse genomweit durchgeführt, wofür sich zum Beispiel der Infinium HumanMethylation450 BeadChip (Illumina, Inc., San Diego, CA, USA) entsprechend der Herstellerangaben eignete. Die Generierung der HumanMethylation450 BeadChip Rohdaten erfolgte wie vom TCGA Research Network (http://cancergenome.nih.gov/) beschrieben. Insgesamt wurden Rohdaten von Tumoren von 417 Prostatakrebspatienten mit bekannter Prognose generiert und retrospektiv analysiert.

Für die erfindungsgemäße DNA-Methylierungsanalyse des *PDCD1* Gens wurde zunächst anhand der HumanMethylation450 BeadChip Rohdaten eine relative Methylierung errechnet. Dazu wurden die CpG-Dinukleotide im *PDCD1* Gen verwendet, welche durch die Bead-Paare cg00795812, cg27051683, cg03889044, cg17322655 und cg20805133 des Infinium HumanMethylation450 BeadChip abgedeckt werden. Diese Bead-Paare binden an die Bisulfit-konvertierte DNA, die vor Konversion die Sequenzen SEQ ID NO:20, SEQ ID NO:21, SEQ ID NO:22, SEQ ID NO:23 und SEQ ID NO:24 hatten. Zunächst wurde anhand der Rohdaten für jedes der genannten Bead-Paare und für jede Patientenprobe ein Methylierungswert berechnet. Dies erfolgte, indem das Signal des Beads eines Paares, das an die methylierte Variante bindet (S_M), zu dem Signal des Beads eines Paares, das an die unmethylierte DNA bindet (S_U), zueinander ins Verhältnis gesetzt wurde. Ein Bead umfasst ein gebundenes Oligonukleotid und wird hier auch als Sonde bezeichnet. Die Berechnung der DNA-Methylierung anhand der Bildung des Verhältnisses erfolgte nach der Formel Methylierung = (Intensität Sonde S_M) / ((Intensität Sonde S_M) + (Intensität Sonde S_U)). Anschließend wurden die so für jeden der fünf Loci mit den Sequenzen SEQ ID NO:20, SEQ ID NO:21, SEQ ID NO:22, SEQ ID NO:23 und SEQ ID NO:24 bestimmten Methylierungswerte arithmetisch gemittelt. Die Patienten wurden anschließend anhand des so ermittelten Methylierungswertes in Abhängigkeit von ihrer bekannten Prognose retrospektiv in zwei Gruppen unterteilt, wodurch sich ein Schwellenwert für Methylierung am *PDCD1* Genlokus von 57,5% ergab. Eine Gruppe hatte eine Methylierung am *PDCD1* Genlokus unterhalb des Schwellenwerts von 57,5% und wird im Folgenden als die Gruppe mit geringer *PDCD1* Methylierung bezeichnet. Entsprechend wird die Gruppe, welche am *PDCD1* Genlokus eine Methylierung von über 57,5% zeigte, als Gruppe mit hoher *PDCD1* Methylierung bezeichnet.

Figur 1 zeigt, dass es durch erfindungsgemäße Anwendung des Verfahrens möglich ist, die Patienten in zwei Gruppen aufzuteilen, von der die Patienten der Gruppe mit geringer *PDCD1* Methylierung eine signifikant besser Prognose aufweisen als die Patienten in der Gruppe mit der hohen *PDCD1* Methylierung, wobei die Prognose hier beispielhaft anhand der voraussichtlichen Zeit bis zum Eintreten eines biochemischen Rezidivs, das heißt einem Anstieg des für Prostatakrebs spezifischen Blutparameters PSA, definiert ist. Die Gruppe mit geringer *PDCD1* Methylierung zeigte ein mittleres PSA-rückfallfreies Überleben von 92 Monaten, während die Gruppe von Patienten mit hoher *PDCD1* Methylierung mit durchschnittlich 79 Monaten ein 13 Monate kürzeres PSA-rückfallfreies Überleben zeigte. Die erfindungsgemäße Anwendung des Verfahrens ermöglichte also die Einteilung der Patienten in unterschiedliche Risikogruppen für einen PSA-Rückfall.

Es ist demzufolge möglich, die Prognose eines Patienten, dessen Tumor gerade erst entfernt wurde und dessen Krankheitsverlauf in der Zukunft noch nicht bekannt ist, zu bestimmen. Hierzu kann die DNA-Methylierung des *PDCD1* Gens in dem Tumor dieses Patienten beispielsweise mittels Infinium HumanMethylation450 BeadChip bestimmt werden. Liegt diese Methylierung beispielsweise unter dem Schwellenwert von 57,5%, dann kann dieser Patient der Gruppe von Patienten mit der besseren Prognose mit durchschnittlich 92 Monaten rückfallfreiem Überleben zugeordnet werden. Liegt die Methylierung des *PDCD1* Gens im Tumor eines Patienten beispielsweise über dem Schwellenwert, dann tritt bei diesem Patienten der PSA-Rückfall wahrscheinlich früher ein. Dieser Patient könnte dann von einer zusätzlichen adjuvanten Therapie profitieren. Beispielsweise könnte man diesen Patienten in der Nachsorge auch engmaschiger verlaufskontrollieren, sodass der eintretende PSA-Rückfall auch frühzeitig diagnostiziert wird, sodass geeignete Behandlungsmaßnahmen eingeleitet werden können.

### Beispiel 2: Bestimmung der Prognose eines Patienten mit einem Prostatakarzinom nach radikaler Ektomie anhand der DNA-Methylierungsanalyse des immunregulatorischen Gens CD274

Durch die erfindungsgemäßen Ausführung des Verfahrens ist es auch möglich, die Bestimmung der Prognose eines Patienten mit maligner Erkrankung anhand der DNA-Methylierungsanalyse von CpG-Dinukleotiden innerhalb des *CD274* Gens durchzuführen. Beispielsweise kann die Bestimmung der Prognose eines Patienten mit einem lokal begrenzten Prostatakarzinom anhand der Methylierung des *CD274* Gens im Tumor erfolgen, nachdem der Patient durch eine radikale Entfernung (radikale Ektomie) der Prostata und des in der Prostata befindlichen Tumors behandelt wurde.

Es wurden Tumorproben von insgesamt 417 Patienten mit bekannter Prognose retrospektiv untersucht. Die DNA-Methylierungsanalyse erfolgte wie in Beispiel 1 beschrieben. Für die Berechnung der relativen Methylierung des *CD274* Genlokus anhand der Rohdaten des Infinium HumanMethylation450 BeadChip wurde wurden die fünf Bead-Paare cg15837913, cg02823866, cg14305799, cg13474877 und cg19724470 verwendet, die im Genom die CpG-Dinukleotid Methylierungsanalyse in den Sequenzen SEQ ID NO:8, SEQ ID NO:9, SEQ ID NO:10, SEQ ID NO:11 und SEQ ID NO:12 ermöglichen. Wie in Beispiel 1 analog für das Gen *PDCD1* beschrieben, wurde für die fünf Bead-Paare jeweils eine relative Methylierung für jeden Patienten berechnet, indem die für den unmethylierten und den methylierten Zustand spezifischen Beads eines Paares wie in Beispiel 1 beschrieben zueinander ins Verhältnis gesetzt wurden. Anschließend wurden diese fünf Methylierungswerte gemittelt, so dass ein relativer Methylierungswert pro Patientenprobe resultierte. Die Patienten wurden anschließend anhand des so ermittelten Methylierungswertes in Abhängigkeit von ihrer retrospektiv bekannten Prognose in zwei Gruppen unterteilt, wodurch sich ein Schwellenwert von 52,75% für die relative Methylierung am *CD274* Genlokus ergab. Eine Gruppe von 337 Patienten hatte eine Methylierung am *CD274* Genlokus unter dem Schwellenwert von 52,75% und wird im Folgenden als die Gruppe mit geringer *CD274* Methylierung benannt. Entsprechend wird die Gruppe, die am *CD274* Genlokus eine Methylierung von über 52,75% zeigte, als Gruppe mit hoher *CD274* Methylierung benannt. Diese Gruppe bestand aus 80 Patienten.

Figur 2A zeigt, dass es durch die erfindungsgemäße Anwendung des Verfahrens möglich ist, die Patienten in zwei Gruppen aufzuteilen, von der die Gruppe mit geringer *CD274* Methylierung eine signifikant besser Prognose aufweist als die Gruppe mit der hohen *CD274* Methylierung. In dieser beispielhaften Anwendung ist als Prognose die voraussichtliche Zeit bis zum Eintreten eines biochemischen Rezidivs, das heißt einem Anstieg des für Prostatakrebs spezifischen Blutparameters PSA, definiert. Die Gruppe mit geringer *CD274* Methylierung zeigte ein mittleres PSA-rückfallfreies Überleben von 2822 Tagen, während die Gruppe von Patienten mit hoher *PDCD1* Methylierung mit durchschnittlich 1578 Tagen ein 1244 Tage kürzeres PSA-rückfallfreies Überleben zeigte. Die erfindungsgemäße Anwendung des Verfahrens ermöglichte also die Einteilung der Patienten in unterschiedliche Risikogruppen für einen PSA-Rückfall.

Es ist auch möglich, für die erfindungsgemäße Bestimmung der Prognose von Patienten mit malignen Erkrankungen die DNA-Methylierungsanalyse mittels Echtzeit-PCR durchzuführen. In diesem Beispiel wurden 259 Patienten mit Prostatatumoren und bekannter Prognose retrospektiv untersucht, wobei es sich um eine andere Patientenkohorte als die vorgehend beschriebenen 417 Patienten handelte.

Für die DNA-Methylierungsanalyse mittels Echtzeit-PCR wurden Gewebedünnschnitte mit einer Dicke von 10 µm angefertigt und auf Glasobjektträger aufgezogen. Anhand eines HE-Schnitts wurden die Tumorbereiche durch pathologische Begutachtung identifiziert. Diese Tumorareale wurden anschließend mittels eines Skalpells von den Glasobjekträgern heruntergekratzt. Bisulfit-konvertierte DNA aus den Tumorarealen wurde mit dem innuCONVERT Bisulfit All-In-One Kit (Analytik Jena, Jena, Deutschland) nach Herstellerangaben bereitgestellt. Anschließend wurde die Menge konvertierter DNA mithilfe eines NanoDrop ND-1000 Spektrophotometers (Thermo Fisher Scientific, Waltham, MA, USA) quantifiziert. Die DNA-Methylierungsanalyse der Prostatatumoren wurde durchgeführt, indem der *CD274* Genlokus methylierungsspezifisch mithilfe einer quantitativen Echtzeit-PCR (qPCR) amplifiziert und gleichzeitig quantifiziert wurde. Dabei kam eine Duplex- oder Multiplex-PCR zum Einsatz, bei welcher innerhalb der gleichen Reaktion neben der Methylierung auch die Gesamt-DNA bestimmt wurde. Die Bestimmung der Gesamt-DNA kann beispielsweise durch die Verwendung von Primern und Sonden durchgeführt werden, deren Zielsequenz keine CpG Dinukleotide enthält und deren Zielsequenz somit methylierungsunabhängig amplifiziert wird. Vorliegend wurde ein Lokus im *ACTB* Gen für die Bestimmung der Gesamt-DNA amplifiziert. Dieser Lokus im Genom hat die Sequenz der SEQ ID NO:32 und hat nach der Konversion durch Bisulfit die Sequenz mit der SEQ ID NO:7. Diese Sequenz wurde unter Verwendung der Primer mit den Sequenzen SEQ ID NO:4 und SEQ ID NO:5 amplifiziert. Die sequenzspezifische Detektion des Amplifikats erfolgte mit der Sonde der Sequenz SEQ ID NO:6, welche an 5' den Fluoreszenzfarbstoff Atto 647N und an 3' den Quencher BHQ-2 trug. Die methylierungsspezifische Amplifikation des *CD274* Lokus erfolgte mit Primern mit den Sequenzen SEQ ID NO:13 und SEQ ID NO:14. Diese Primer amplifizieren die Sequenz, die nach der Bisulfit-Konversion der Sequenz SEQ ID NO:33 entsteht. Im Fall von vollständiger Methylierung hat diese umgewandelte Region im Genom die Sequenz SEQ ID NO:16. Die methylierungsspezifische Detektion erfolgte mit einer Sonde der Sequenz SEQ ID NO:15, welche an 5' den Fluoreszenzfarbstoff 6-FAM und an 3' den Quencher BHQ-1 trug. Der Methylierungszustand in der konvertierten DNA der Prostatatumoren wurde mittels der DeltaDelta-CT Methode berechnet und prozentual gegenüber einer Standard-DNA mit 100% Methylierung ausgedrückt. Als Standard-DNA wurde künstlich methylierte DNA (CpGenomeTM Universal Methylated DNA; Merck Millipore, Darmstadt, Germany) verwendet, welche zuvor mit dem innuCONVERT Bisulfite All-In-One Kit entsprechend der Herstellerangaben umgewandelt wurde. Im vorliegenden Beispiel wurde die Echtzeit-PCR Quantifizierung der *CD274* Methylierung in 20 µl PCR Reaktionen in jeweils drei unabhängigen Messungen durchgeführt, wobei sich die folgende Reaktionszusammensetzung eignete: 35 mM Tris-HCl, pH 8,4, 6 mM MgCl₂, 50 mM KCl, 4% Glycerin, 0,25 mM jedes dNTP (dTTP, dATP, dGTP, dCTP), 2 U FastStart *Taq* DNA-Polymerase (Roche Applied Science, Penzberg, Deutschland), 0,4 µM jedes Primers und 0,2 µM jeder Detektionssonde. Die qPCR wurde zum Beispiel mittels eines AB 7500 Fast Real-Time PCR System (Life Technologies Corporation, Carlsbad, CA, USA) durchgeführt. Ein geeignetes Temperaturprofils umfasste beispielweise folgende Schritte: 20 min bei 95 °C gefolgt von 45 Zyklen je 45 s bei 56 °C und 15 s bei 95 °C.

Figur 2B zeigt, dass die erfindungsgemäße Anwendung des Verfahrens geeignet ist, anhand der mittels Echtzeit-PCR ermittelten Methylierung des *CD274* Genlokus die Prognose von Patienten mit Prostatakarzinomen zu bestimmen. Die 259 Patienten konnten anhand der DNA-Methylierungsanalyse in zwei Gruppen mit unterschiedlicher Prognose eingeteilt werden. Patienten, deren Tumoren eine Methylierung über 0,975% zeigten, hatten ein durchschnittliches PSA-rückfallfreies Überleben von 93 Monaten, während Patienten, deren Tumor eine Methylierung unter 0,975% zeigten, ein um 21 Monate längeres durchschnittliches PSA-rückfallfreies Überleben zeigten (114 Monate).

Es ist beispielsweise möglich, die Methylierung des *CD274* Lokus in der Tumorprobe eines Patienten mit unbekannter Prognose analog zu der oben beschriebenen Echtzeit-PCR zu bestimmen. Liegt diese Methylierung zum Beispiel über 0,975%, dann kann der Patient eine ungünstige Prognose haben, welche durchschnittlich bei 93 Monaten rückfallfreiem Überleben liegen kann. Liegt die Methylierung im Tumor dagegen unter diesem Schwellenwert, dann hat der Patient wahrscheinlich eine günstigere Prognose mit durchschnittlich 114 Monaten rückfallfreiem Überleben.

In diesem Beispiel wurde das erfindungsgemäße Verfahren mithilfe von zwei unterschiedlichen Technologien, Echtzeit-PCR und Infinium HumanMethylation450 BeadChip, durchgeführt. Der Methylierungs-Schwellenwert, anhand dessen die Patienten in eine Gruppe mit guter und in eine Gruppe mit schlechter Prognose eingeteilt werden konnten, lag bei 52,75% (Infinium HumanMethylation450 BeadChip) und 0,975% (Echtzeit-PCR). Es ist für den Fachmann ersichtlich, dass geeignete Schwellenwerte für die Einteilung in Prognosegruppen in Abhängigkeit von der verwendeten Methode für die DNA-Methylierungsanalyse ermittelt werden können. Eine andere Möglichkeit besteht darin, verschiedene Methoden gegeneinander zu kalibrieren, so dass die Ergebnisse der jeweiligen DNA-Methylierungsanalyse direkt miteinander verglichen werden können. Beispielsweise kann zur Kalibrierung eine DNA-Methylierungsanalyse einer oder mehrerer Standard-DNA Proben mit definierter prozentualer Methylierung mit verschiedenen Methoden durchgeführt werden. Indem für jede Methode die Ergebnisse der DNA-Methylierungsanalyse von den zu untersuchenden immunregulatorischen Genen zu den Ergebnissen der DNA-Methylierungsanalyse der Standard-DNA Proben in Bezug gesetzt werden, können die Messergebnisse kalibriert werden, so dass sich eine Vergleichbarkeit der kalibrierten Messergebnisse aus verschiedenen Methoden ergibt.

### Beispiel 3: Bestimmung der Prognose von Patienten mit malignen Melanomen anhand der DNA-Methylierungsanalyse der immunregulatorischen Gene PDCD1, PDCD1LG2 und CD274

Dieses Beispiel zeigt die erfindungsgemäße Bestimmung der Prognose von 470 Patienten mit malignen Melanomen. Die Ergebnisse der DNA-Methylierungsanalyse wurden mittels des Infinium HumanMethylation450 BeadChip wie in Beispiel 1 beschrieben generiert. Für die Bestimmung der relativen Methylierung des jeweiligen Gens wurden zunächst wie in Beispiel 1 beschrieben für jedes ausgewählte Bead-Paar des Infinium HumanMethylation450 BeadChip eine relative Methylierung ermittelt. Diese relative Methylierung von bis zu sechs Bead-Paaren pro Gen wurde anschliessend gemittelt, um einen repräsentativen Methylierungswert für das jeweilige Gen zu erhalten. Für die Methylierungsanalyse von *CD274* wurden die Bead-Paare cg15837913 (SEQ ID NO:8), cg02823866 (SEQ ID NO:9), cg14305799 (SEQ ID NO:10), cg13474877 (SEQ ID NO:11) und cg19724470 (SEQ ID NO:12) verwendet. Diese Beads ermöglichen beispielsweise die Bestimmung der Methylierung von CpG-Dinukleotiden in des Sequenzbereichen SEQ ID NO:1, SEQ ID NO:33 und SEQ ID NO:76 des *CD274* Gens. Für die Methylierungsanalyse von *PDCD1* wurden die Bead-Paare cg00795812 (SEQ ID NO:20), cg03889044 (SEQ ID NO:22), cg17322655 (SEQ ID NO:23), cg20805133 (SEQ ID NO:24) und cg27051683 (SEQ ID NO:369) verwendet. Diese Beads ermöglichen beispielsweise die Bestimmung der Methylierung von CpG-Dinukleotiden in des Sequenzbereichen SEQ ID NO:3 und SEQ ID NO:29 des *PDCD1* Gens. Für die Methylierungsanalyse von *PDCD1LG2* wurde das Bead-Paar cg07211259 (SEQ ID NO:31) verwendet. Dieser Bead ermöglicht beispielsweise die Bestimmung der Methylierung von CpG-Dinukleotiden des Sequenzbereichs SEQ ID NO:84 des *PDCD1LG2* Gens.

Figur 3 zeigt die retrospektive Einteilung der 470 Patienten mit malignen Melanomen in Prognosegruppen anhand der DNA-Methylierungsanalysen. Dargestellt sind die Kaplan-Meier Überlebensanalysen der Patienten, die anhand der Methylierung der Gene *PDCD1* (Figur 3A), *CD274* (Figur 3B) und *PDCD1LG2* (Figur 3C) im Tumor in Abhängigkeit von ihrer Prognose in Gruppen eingeteilt wurden. Gruppe I umfasst Patienten, deren Tumor eine geringe Methylierung unterhalb eines Schwellenwertes am entsprechenden Genlokus aufwies. Gruppe II enthält diejenigen Patienten, deren Tumor eine erhöhte Methylierung oberhalb des Schwellenwertes des jeweiligen Gens aufweist. Als Schwellenwerte für die Gruppierung der Patienten wurden beispielsweise folgende Methylierungswerte festgesetzt: 12,84% für *PDCD1LG2,* 13,11% für *CD274* und 20,54% für *PDCD1.* Für alle drei analysierten Gene konnte mithilfe der DNA-Methylierungsanalyse gezeigt werden, dass die Patienten mit einer erhöhten Methylierung im Tumor einen ungünstigeren Verlauf der Erkrankung zeigten. Die erfindungsgemäße Anwendung des Verfahrens ermöglichte somit eine Bestimmung von Prognosegruppen von Patienten mit malignen Melanomen anhand der DNA-Methylierung des Immun-Checkpoint Gens *PDCD1* sowie des für den dazugehörigen Liganden codierenden Gens *CD274.* Außerdem konnte gezeigt werden, dass mit *PDCD1LG2* ein weiteres für einen Liganden des Immun-Checkpoints PDCD1 codierendes Gen zur erfindungsgemäßen Bestimmung der Prognose verwendet werden konnte.

Dieses Beispiel zeigt, dass das erfindungsgemäße Verfahren nicht nur wie in Beispiel 1 und Beispiel 2 nur auf eine einzige maligne Erkrankung, dem Prostatakarzinom, beschränkt ist, sondern dass auch bei anderen maligne Erkrankungen, wie beispielsweise beim malignen Melanom die Bestimmung der Prognose möglich ist. Die Bestimmung der Prognose anhand der DNA-Methylierungsanalyse dieser Gene ist demzufolge nicht auf einzelne Entitäten beschränkt, sondern auf andere Malignitäten übertragbar. Somit löst die vorliegende Erfindung das Problem der begrenzten Anwendbarkeit auf einzelne Entitäten von herkömmlichen Prognoseverfahren.

Des Weiteren konnte in diesem Beispiel gezeigt werden, dass mittels der erfindungsgemäßen Ausführung des Verfahrens die Bestimmung der Prognose hinsichtlich verschiedener in der Zukunft liegenden Gesundheitszustände möglich ist. In Beispiel 1 und Beispiel 2 konnte das Auftreten eines PSA-Rückfalls richtig prognostiziert werden. Ein PSA-Rückfall ist eine auf Prostatatumoren beschränkte Eigenschaft. In dem vorliegenden Beispiel 3 konnte dagegen der Eintritt des Todes des Patienten prognostiziert werden.

### Beispiel 4: Bestimmung der Prognose von Patienten mit malignen Melanomen anhand der DNA-Methylierungsanalyse der immunregulatorischen Gene TIGIT, TNFRSF9, LAG3, BTLA, CTLA4 und CD40

Das erfindungsgemäße Verfahren ermöglicht auch die Abschätzung der Prognose von Patienten mit malignen Melanomen anhand der Methylierung der Immun-Checkpoint-Gene *TIGIT, TNFRSF9, LAG3, BTLA, CTLA4* und *CD40.* Im vorliegenden Beispiel wurden die Ergebnisse der DNA-Methylierungsanalyse für die Bestimmung der relativen Methylierung der Gene mittels des Infinium HumanMethylation450 BeadChip (Illumina, Inc., San Diego, CA, USA) wie in Beispiel 1 beschrieben generiert. Anschließend wurde für die Gene *TIGIT, TNFRSF9, LAG3, BTLA, CTLA4* und *CD40* je eine relative Methylierung ermittelt. Hierzu wurde wie in Beispiel 1 beschrieben ein Verhältnis aus der für den methylierten und der für den unmethylierten Zustand spezifischen Beads eines Bead-Paares gebildet. Für das Gen *BTLA* wurden die Bead-Paare cg24157392 und cg19281794 verwendet. Diese Beads binden an die Sequenz der humanen DNA, welche vor der Bisulfit-Konversion die Sequenz mit der SEQ ID NO:37 beziehungsweise SEQ ID NO:37 hatte und ermöglichen somit beispielsweise die Bestimmung der Methylierung für den Sequenzbereich SEQ ID NO:276. Beide Bead-Paare bestehen jeweils aus einem Paar Beads, von der eines an die vor der Bisulfit-Konversion unmethylierte und eines vor der Konversion methylierte Sequenz bindet. Aus diesen beiden Bead-Paaren wurde das Verhältnis gebildet, wie beispielsweise für das Bead-Paar cg24157392 im Folgenden gezeigt: Methylierung = (Intensität cg24157392_M) / ((Intensität cg24157392_M) + (Intensität cg24157392_U)), wobei cg24157392_M das Bead ist, das an die methylierte Variante bindet und cg24157392_U das Bead, das an die unmethylierte Variante bindet. Das gleiche Verfahren wurde für das Bead-Paar cg19281794 angewendet. Anschließend wurde der relative Methylierungswert für *BTLA* berechnet, indem die relative Methylierung der Bead-Paare cg24157392 und cg19281794 arithmetisch gemittelt wurde.

Entsprechend wurde die relative Methylierung der Gene *TIGIT, TNFRSF9, LAG3, BTLA, CTLA4* und *CD40* bestimmt. Für die Bestimmung der relativen Methylierung von *TNFRSF9* wurden die Bead-Paare cg14614416 (SEQ ID NO:51), cg14153654 (SEQ ID NO:54), cg17123655 (SEQ ID NO:55), cg18025409 (SEQ ID NO:57), cg06956444 (SEQ ID NO:58) und cg08840010 (SEQ ID NO:59) verwendet, welche beispielsweise die Bestimmung der Methylierung der Sequenzbereiche SEQ ID NO:308, SEQ ID NO:309 und SEQ ID NO:321 im Genom ermöglichen. Bei der Bestimmung der Methylierung von *CD40* wurden die Bead-Paare cg19785066 (SEQ ID NO:47), cg09053081 (SEQ ID NO:48) und cg21601405 (SEQ ID NO:49) verwendet, die beispielsweise zur Bestimmung der Methylierung der Sequenzbereiche SEQ ID NO:297 und SEQ ID NO:298 geeignet sind. Für die Bestimmung der Methylierung von *TIGIT* wurden die Bead-Paare cg02771886 (SEQ ID NO:61), cg08723913 (SEQ ID NO:62), cg17164827 (SEQ ID NO:63), cg04885775 (SEQ ID NO:64), cg23637607 (SEQ ID NO:65) und cg16358924 (SEQ ID NO:66) verwendet, die geeignet sind die Methylierung des Sequenzbereichs SEQ ID NO:267 zu bestimmen. Für die Bestimmung der Methylierung des Sequenzbereichs SEQ ID NO:162 des *CTLA4* Gens wurde das Bead-Paar cg08460026 (SEQ ID NO:39) verwendet. Für die Bestimmung der relativen Methylierung von *LAG3* wurden die Bead-Paare cg04153135 (SEQ ID NO:40), cg20652042 (SEQ ID NO:41) und cg01820374 (SEQ ID NO:42) verwendet, die geignet sind, die Methylierung in den Sequenzbereichen SEQ ID NO:161, SEQ ID NO:349 und SEQ ID NO:351 zu bestimmen.

Figur 4 zeigt die retrospektive Einteilung der 470 Patienten mit malignen Melanomen in Prognosegruppen anhand der erfindungsgegenständlichen DNA-Methylierungsanalysen. Dargestellt sind die Kaplan-Meier Überlebensanalysen der Patienten, die anhand der Methylierung der Gene *TIGIT* (Figur 4A), *TNFRSF9* (Figur 4B), *LAG3* (Figur 4C), *BTLA* (Figur 4D), *CTLA4* (Figur 4E) und *CD40* (Figur 4F) im Tumor in Gruppen eingeteilt wurden. Gruppe I umfasst Patienten, deren Tumor eine geringe Methylierung unterhalb eines Schwellenwertes am entsprechenden Genlokus aufwies. Gruppe II enthält diejenigen Patienten, deren Tumor eine erhöhte Methylierung oberhalb des Schwellenwertes des jeweiligen Gens aufweist. Als Schwellenwerte für die Gruppierung der Patienten wurden beispielsweise folgende Methylierungswerte festgesetzt: 86,41% für *TIGIT,* 57,80% für *TNFRSF9,* 66,94% für *LAG3,* 91,31% für *BTLA,* 13,10% für *CTLA4* und 17,16% für *CD40.* Für alle in diesem Beispiel untersuchten immunregulatorischen Gene konnte gezeigt werden, dass die Patienten, deren Tumoren eine erhöhte Methylierung oberhalb des Schwellenwerts aufwiesen, einen signifikant schlechteren Krankheitsverlauf zeigten, als die Patienten, deren Tumoren eine Methylierung unterhalb des Schwellenwerts zeigten.

### Beispiel 5: Bestimmung der Prognose von Patienten mit Plattenepithelkarzinomen des Kopf- und Halsbereichs anhand der DNA-Methylierungsanalyse des immunregulatorischen Gens PDCD1

Figur 5 zeigt die retrospektive Einteilung von 528 Patienten mit Plattenepitehlkarzinomen des Kopf- und Halsbereichs in Prognosegruppen anhand des erfindungsgemäßen Verfahrens. Als klinischer Endpunkt ist der Tod definiert. Die DNA-Methlierungsanalyse des Immun-Checkpoint Gens *PDCD1* wurde in der DNA aus dem Tumorgewebe gemessen. Die Patienten mit hoher *PDCD1* Methylierung zeigen einen schlechteren Verlauf als die Patienten mit niedriger Methylierung des *PDCD1* Genlokus. Die Gruppierung der Patienten erfolgte mittels Dichotomisierung unter Verwendung des Medians als Schwellenwert, das heißt der Schwellenwert wurde derart gewählt, dass die eine Hälfte (n = 264 Patienten) der Patientenproben einen Methylierungswert oberhalb des Schwellenwertes und die andere Hälfte (n = 264 Patienten) unterhalb des Schwellenwertes aufwiesen.

Es war ebenfalls möglich, die DNA-Methylierungsanalyse von *PDCD1* mittels Echtzeit-PCR durchzuführen. Geeignete Bedingungen und Zusammensetzungen für die Echtzeit-PCR entsprechen denen aus Beispiel 2. Die folgenden Oligonukleotide wurden für die PCR verwendet: SEQ ID NO:25 (Vorwärtsprimer), SEQ ID NO:26 (Rückwärtsprimer) und SEQ ID NO:27 Detektionssonde. Die Detektionssonde war mit FAM und BHQ-1 markiert. Die verwendeten Primer ermöglichen die Amplifikation der Bisulfit-konvertierten DNA mit der Sequenz SEQ ID NO:368. Diese Sequenz hatte vor der Bisulfit-Umwandlung die Sequenz SEQ ID NO:367. Es wurden Tumorproben von insgesamt 120 Patienten mit Plattenepithelkarzinomen des Kopf und Halsbereichs und bekannter Prognose retrospektiv untersucht. Die Patienten konnten anhand der DNA-Methylierungsanalyse und in Abhängigkeit von ihrer Prognose in eine Gruppe von 40 Patienten mit niedriger und eine Gruppe von 80 Patienten mit hoher Methylierung aufgeteilt werden. Die Gruppe der Patienten mit hoher Methylierung zeigte ein signifikant schlechteres Gesamtüberleben im Vergleich zu der Gruppe mit der niedrigen Methylierung (p-Wert < 0,05).

Im Beispiel 1 konnte gezeigt werden, dass die erfindungsgemäße Verfahrensausführung die Bestimmung der Prognose bei Patienten mit Prostatakarzinomen anhand der Methylierung des Gens *PDCD1* ermöglicht. Bei diesen Prostatakarzinomen handelte es sich um Karzinome, die von dem Drüsenepithel der Prostata hervorging. Diese Karzinome gehören damit zu den Adenokarzinomen. In Beispiel 3 konnte auch gezeigt werden, dass die erfindungsgemäße Verfahrensauführung die Prognose bei Patienten mit malignen Melanomen ermöglicht. Melanome gehören nicht zu den Karzinomen, da sie nicht aus Epithelzellen, sondern aus Melanozyten hervorgehen. Melanome stellen somit eine grundsätzlich andere maligne Erkrankung im Vergleich zu Karzinomen dar. In dem vorliegenden Beispiel 5 konnte zusätzlich gezeigt werden, dass die erfindungsgemäße Bestimmung der Prognose auch bei Patienten mit Plattenepithelkarzinomen des Kopf- und Halsbereichs möglich ist. Diese Plattenepitelkarzinome gehören wie Adenokarzinome zu den Karzinomen, das heißt sie gehen aus Epithelzellen hervor. Im Gegensatz zu Adenokarzinomen gehen Plattenepithelkarzinome nicht aus den Drüsenepithelien hervor, sondern vom Plattenepithel. Es konnte also gezeigt werden, dass die erfindungsgemäße Verfahrensausführung nicht nur die Bestimmung der Prognose bei Karzinomen unterschiedlicher Herkunft, sondern auch von malignen Erkrankungen, die keine Karzinome sind, möglich ist. Diese Ergebnisse belegen zusätzlich die universelle Anwendbarkeit des erfindungsgemäßen Verfahrens zur Prognose und/oder Pradiktion bei verschiedensten malignen Erkrankungen.

### Beispiel 6: Bestimmung der mRNA-Expression der Immun-Checkpoint Gene anhand der DNA-Methylierungsanalyse

Die erfindungsgemäße Ausführung des Verfahrens ermöglicht auch die Bestimmung der Expression der von den immunregulatorischen Genen codierten Immun-Checkpoints, wie nachfolgend anhand der mRNA-Expression der Gene *BTLA* und *CD27* beispielhaft gezeigt wird.

Die Bestimmung der mRNA kann beispielsweise nach Extraktion der mRNA, Präparation einer cDNA Bibliothek der gesamt RNA und letztlich einer Next Generation Sequencing basierten Analyse bestimmt werden. Beispielsweise kann die RNA mittels des RNeasy Mini Kit (Qiagen, Hilden) entsprechend der Herstellerangaben aus dem Tumorgewebe gewonnen werden. Anschliessend kann beispielsweise entsprechend der Herstellerangaben unter Verwendung des TruSeq RNA Library Preparation Kit v2 (Illumina) eine Bibliothek hergestellt und mittels HiSeq PE (Paired-End) Cluster Kit v4 cBot (Illumina) auf einem HiSeq 2500 Next Generation Sequencer (Illumina) analysiert werden. Die Messung der mRNA beziehungsweise die Bestimmung der Anzahl der mRNA Moleküle einer bestimmten Sequenz im Verhältnis zur Anzahl der gesamten mRNA Moleküle erfolgte wie vom TCGA Research Network (http://cancergenome.nih.gov/) beschrieben.

Figur 6 zeigt die Korrelation der DNA-Methylierung im Gewebe von maligenen Melanomen von 470 Patienten mit der mRNA-Expression für *BTLA* (Figur 6A) und *CD27* (Figur 6C). Es ist ersichtlich, dass alle Tumoren, die eine Methylierung der jeweiligen Loci von unter 50% aufwiesen, eine Expression der mRNA von über 10 (*BTLA,* Figur 6A) beziehungsweise von über 100 (*CD27,* Figur 6C) zeigten. Figur 6B zeigt, dass Melanom-Patienten, deren Tumoren eine *BTLA* mRNA Expression von >10 aufwiesen, eine deutlich bessere Prognose haben, als Patienten, deren Tumoren eine geringere mRNA-Expression aufwiesen. Analog zu *BTLA* ist in Figur 6D gezeigt, dass Melanom-Patienten, deren Tumoren eine *CD27* mRNA-Expression von >100 hatten, eine deutlich bessere Prognose haben, als Patienten, deren Tumoren eine geringere mRNA-Expression aufwiesen.

Die Expression der Gene *BTLA* und *CD27* kann nicht nur für die Bestimmung der Prognose des Patienten herangezogen werden, sondern ist auch indikativ für das wahrscheinliche Ansprechen auf eine Therapie, beispielsweise wenn die Therapie eine gegen das entsprechende Genprodukt gerichtete Therapie ist. Dieses Beispiel zeigt, dass anhand der Methylierung der Genloci im Tumor eine Bestimmung der mRNA-Expression möglich ist und für die Bestimmung der Prognose des Patienten herangezogen werden kann. Dadurch wird zum einen das Problem gelöst, dass eine direkte Bestimmung der mRNA selbst aufgrund der geringen Stabilität der mRNA oftmals in der klinischen Routine nur eingeschränkt möglich ist. Des Weiteren zeigt dieses Beispiel, dass durch die Bestimmung der Prognose des Patienten anhand der DNA-Methylierungsanalyse auch gleichzeitig eine Abschätzung der Expression des analysierten immunregulatorischen Gens möglich ist. Dadurch kann der Patient für eine Therapie qualifiziert werden, die in Abhängigkeit der Expression des Genprodukts, also des Immun-Checkpoints, wahrscheinlich wirkt beziehungsweise wahrscheinlich nicht wirkt. Besonders sind hierfür Therapien geeignet, welche direkt gegen den Immun-Checkpoint oder dessen Interaktionspartner gerichtet sind. Insbesondere sind hierfür Immuntherapien mit monoklonalen Antikörpern geeignet, die gegen diese Genprodukte gerichtet sind.

### Beispiel 7: Bestimmung der Prognose eines Patienten mit einem malignen Melanom anhand der kombinierten DNA-Methylierungsanalyse und mRNA-Expressionsanalyse

Es wurde eine DNA-Methylierungsanalyse und eine mRNA-Expressionsanalyse des *CD274* Gens in Tumoren von 470 Patienten mit malignen Melanomen und bekannter Prognose durchgeführt. Die Bestimmung der mRNA-Expression und der DNA-Methylierung erfolgte wie in den Beispielen 1, 3 und 6 beschrieben.

Figur 7A zeigt ein Streudiagramm der ermittelten mRNA-Expression und DNA-Methylierung des *CD274* Gens mit eingezeichneten Schwellenwerten, anhand derer die retrospektive Einteilung in Prognosegruppen mit hoher und niedriger mRNA-Expression beziehungsweise DNA-Methylierung vorgenommen wurde. Figur 7A zeigt die signifikant negative Korrelation (Spearman's ρ = -0,546, p < 0,001 ) von DNA-Methylierung und mRNA-Expression. Anhand des eingeführten Schwellenwerts für die Methylierung bei 13,11% lassen sich die Patienten erfindungsgemäß in eine Gruppe mit höherer (Gruppe II+III) und mit niedriger DNA-Methylierung (Gruppe I+IV) einteilen. Analog ermöglicht die in Figur 7A dargestellte Einführung eines senkrechten Schwellenwertes für die mRNA-Expression bei 18,23 die Bildung von zwei Gruppen mit hoher (Gruppe III+IV) und niedriger (Gruppe I+II) mRNA-Expression. Figur 7B zeigt eine Kaplan-Meier Analyse des Gesamtüberlebens der Patienten mit hoher *CD274* Methylierung im Tumor (Gruppe II+III) im Vergleich zu den Patienten mit niedriger Methylierung (Gruppe I+IV). Die Patienten in Gruppe II+III zeigen eine ungünstigere Prognose im Vergleich zu den Patienten in Gruppe I+IV. Figur 7C illustriert die Kaplan-Meier Analyse des Gesamtüberlebens der Patienten mit hoher *CD274* mRNA-Expression im Tumor (Gruppe III+IV) im Vergleich zu den Patienten mit niedriger Methylierung (Gruppe I+II). Patienten, deren Tumoren eine erhöhte mRNA-Expression des Gens *CD274* im Tumor zeigen (Gruppe III+IV) haben einen günstigeren klinischen Verlauf im Vergleich zu den Patienten aus Gruppe I+II, deren Tumoren eine niedrigere mRNA-Expression von *CD274* aufwiesen. Figur 7D zeigt die Kaplan-Meier Analyse des Gesamtüberlebens der Patienten mit hoher *CD274* mRNA-Expression und niedriger Methylierung im Tumor (Gruppe IV) im Vergleich zu den Patienten mit hoher Methylierung und niedriger mRNA-Expression (Gruppe II) sowie den Patienten mit hoher Methylierung und hoher mRNA-Expression und den Patienten mit niedriger Methylierung und niedriger mRNA-Expression (Gruppe I+III). Es ist ersichtlich, dass Patienten, welche sowohl anhand der mRNA-Expressionsanalyse als auch der DNA-Methylierungsanalyse jeweils der Gruppe mit der guten Prognose zugewiesen sind (Gruppe IV: hohe mRNA Expression und niedriger Methylierung), einen deutlich günstigeren Verlauf haben im Vergleich zu den Patienten, deren Tumoren anhand der mRNA-Expressionsanalyse und der DNA-Methylierungsanalyse der Gruppe mit der schlechten Prognose zugewiesen sind (Gruppe II: hohe Methylierung und niedrige Expression). Alle anderen Patienten (Gruppe I+III) zeigen eine Prognose, die zwischen der Prognose der Gruppen II und IV liegt (Figur 7D).

Figur 7 beschreibt detailliert die erfindungsgemäße Verfahrensausführung zur Kombination der mRNA-Expressionsanalyse und der DNA-Methylierungsanalyse zur verbesserten Bestimmung der Prognose eines Patienten mit einem malignen Melanom am Beispiel des Gens *CD274.* Bei Anwendung dieses erfindungsgemäßen Verfahrens auf weitere Gene ist ebenfalls eine verbesserte Bestimmung der Prognose ermöglicht. Im Vergleich zu *CD274* (Figur 8A und Figur 7D) konnte dies ebenfalls für die Gene *PDCD1LG2* (Figur 8B), *PDCD1* (Figur 8C), *BTLA* (Figur 8D), *LAG3* (Figur 8E), *TIGIT* (Figur 8F), *CD40* (Figur 8G), *CTLA4* (Figur 8H) und *TNFRSF9* (Figur 8I) gezeigt werden. Figur 8 zeigt die Einteilung von 470 Patienten mit malignen Melanomen und bekannter Prognose retrospektiv anhand der kombinierten Analyse der mRNA-Expression und der DNA-Methylierung verschiedener immunregulatorischer Gene der vorliegenden Erfindung. Die Gruppierung der Patienten erfolgte analog zu Figur 7 entsprechend für die Gene individuell angepasster Schwellenwerte. Der Krankheitsverlauf der Patienten mit hoher DNA-Methylierung und niedriger mRNA-Expression im Tumor (Gruppe II), der Patienten mit niedriger DNA-Methylierung und hoher mRNA-Expression im Tumor (Gruppe IV), sowie den verbleibenden Patienten (Gruppe I+III) wurde verglichen. Für alle untersuchten Gene konnte gezeigt werden, dass die Gruppe II ein ungünstigen Krankheitsverlauf und die Gruppe IV einen günstigen Krankheitsverlauf hatte, während die verbleibenden Patienten (Gruppe I+III) ein mittleres Risiko hatten zu versterben. Gruppe II stellt damit eine Hochrisikogruppe und Gruppe IV eine Niedrigrisikogruppe dar.

### Beispiel 8: Bestimmung der Prognose eines Patienten mit einem malignen Melanom anhand der kombinierten DNA-Methylierungsanalyse und mRNA-Expressionsanalyse der immunregulatorischen Gene PDCD1LG2, CD274, PDCD1, BTLA, LAG3, TTGTT, CD40, CTLA4 und TNFRSF9

In den Beispielen 3 und 4 wurde die erfindungsgemäße Bestimmung der Prognose von Patienten mit malignen Melanomen anhand der DNA-Methylierungsanalyse von insgesamt neun verschiedenen Immun-Checkpoint Genen gezeigt (*PDCD1LG2, CD274, PDCD1, BTLA, LAG3, TIGIT, CD40, CTLA4* und *TNFRSF9*). Die Bestimmung der Prognose der Patienten mit maligner Erkrankung lässt sich weiter verbessern, indem DNA-Methylierungsanalysen und mRNA-Expressionsanalysen verschiedener Gene kombiniert werden. Die Bestimmung der mRNA-Expression und der DNA-Methylierung erfolgte wie in den Beispielen 1 und 6 beschrieben. Die Berechnung eines quantitativen Methylierungswertes erfolgte für jedes Gen wie in den Beispielen 3 und 4 beschrieben. Die Einteilung der Patienten in Prognosegruppen erfolgte zunächst anhand der mRNA-Expression der neun Gene. Gruppe I ist die Gruppe, der anhand der mRNA-Expression der neun Gene eine günstige Prognose zugeordnet werden konnte. Gruppe I umfasst Patienten, deren Tumor eine erhöhte mRNA-Expression über dem Schwellenwert von mindestens acht der neun Gene zeigte. Gruppe II ist die Gruppe, der anhand der mRNA-Expression der neun Gene eine ungünstige Prognose zugeordnet werden konnte. In Gruppe II befinden sich diejenigen Patienten, deren Tumoren bei weniger als acht der analysierten neun Gene eine mRNA-Expression oberhalb des Schwellenwertes zeigten. Anschliessend erfolgte die Einteilung der Patienten anhand der DNA-Methylierung der neun Gene. Gruppe III ist die Gruppe, der anhand der DNA-Methylierung der neun Gene eine günstige Prognose zugeordnet werden konnte. Gruppe III umfasst Patienten, deren Tumor eine erhöhte DNA-Methylierung über dem Schwellenwert bei maximal vier der neun Gene zeigt. Gruppe IV ist die Gruppe, der anhand der DNA-Methylierung der neun Gene eine ungünstige Prognose zugeordnet werden konnte. In Gruppe IV befinden sich diejenigen Patienten, deren Tumoren bei mindestens fünf der analysierten neun Gene eine Methylierung oberhalb des Schwellenwertes zeigten. Letztlich erfolgte die Einteilung der Patienten anhand der DNA-Methylierung und der mRNA-Expression der neun Gene. Gruppe V umfasste die Patienten, die sich sowohl hinsichtlich der mRNA-Expression als auch der DNA-Methylierung den Gruppen mit der günstigen Prognose zuordnen ließen. Gruppe V umfasste die Patienten, die sowohl in Gruppe I als auch in Gruppe III waren. Gruppe VI umfasste die Patienten, die sich sowohl hinsichtlich der mRNA-Expression als auch der DNA-Methylierung in den Gruppen mit der ungünstigen Prognose befanden. Gruppe VI umfasste die Patienten, die sowohl in Gruppe II als auch in Gruppe IV waren. Gruppe VII umfasste die Patienten, die sich entweder hinsichtlich der mRNA-Expression oder der DNA-Methylierung in der Gruppe mit der günstigen Prognose befanden. Gruppe VII umfasste die Patienten, die sowohl in Gruppe I als auch in Gruppe IV oder sowohl in Gruppe II als auch in Gruppe III waren. Figur 9B zeigt, dass diejenigen Patienten, welche anhand der DNA-Methylierung von maximal vier der analysierten neun Gene jeweils einer Gruppe mit schlechterer Prognose zugeorndet waren, also für das jeweilige Gen eine Methylierung oberhalb des Schwellenwertes aufwiesen, insgesamt eine sehr günstige Prognose aufwiesen (Gruppe III). Patienten, welche anhand der Methylierungsanalyse bei mindestens fünf der neun Gene einer Gruppe mit schlechterer Prognose zuzuordnen sind (Gruppe IV), hatten dagegegen auch insgesamt eine deutlich ungünstigere haben. Figur 9A zeigt, dass Patienten, welche anhand der mRNA-Expressionsanalyse bei mindestens acht der neun Gene der Gruppe mit der günstigeren Prognose zuzuordnen waren (Gruppe I), insgesamt eine deutlich günstigere Prognose aufwiesen, als die Patienten, welche sich anhand der mRNA-Expressionsanalyse bei weniger als acht der neun Gene in die Gruppe mit der guten Prognose zuordnen ließen (Gruppe II). Als Schwellenwerte für die Gruppierung der Patienten anhand der DNA-Methylierungsanalyse wurden beispielsweise folgende Methylierungswerte festgesetzt: 12,84% für *PDCD1LG2,* 13,11% für *CD274,* 20,54% für *PDCD1,* 86,41% für *TIGIT,* 57,80% für *TNFRSF9,* 66,94% für *LAG3,* 91,31% für *BTLA,* 13,10% für *CTLA4* und 17,16% für *CD40.* Als Schwellenwerte für die Gruppierung der Patienten anhand der mRNA-Expressionsanalyse wurde beispielsweise folgende mRNA-Expression festgesetzt: 5,61 für *PDCD1LG2,* 18,23 für *CD274,* 7,29 für *PDCD1,* 12,38 für *TIGIT,* 0,935 für *TNFRSF9,* 9,65 für *LAG3,* 3,33 für *BTLA,* 13,12 für *CTLA4* und 30,14 für *CD40.*

In Beispiel 7 (Figur 8) konnte bereits gezeigt werden, dass die erfindungsgemäße Kombination von DNA-Methylierungsanalyse und mRNA-Expressionsanalyse eine noch differenziertere Bestimmung der Prognose von Patienten mit maligenen Erkrankung ermöglicht, als die Bestimmung der Prognose entweder anhand der DNA-Methylierungsanalyse oder der mRNA-Expressionsanalyse allein. Dieser vorteilhafte Effekt der Erfindung lässt sich demnach wie hier gezeigt noch weiter verbessern, wenn die Kombination von DNA-Methylierungsanalyse und mRNA-Expressionsanalyse mehrere immunregulatorische Gene umfasst. Figur 9C zeigt, dass Patienten, deren Tumoren sich sowohl anhand der DNA-Methylierungsanalyse als auch anhand der mRNA-Expressionsanalyse der vorliegend untersuchten neun Gene in jeweils den Gruppen mit der guten Prognose befinden (Figur 9B, Gruppe III und Figur 9A, Gruppe I), eine insgesamt sehr gute Prognose aufweisen (Figur 9C, Gruppe V). Patienten, die sowohl anhand der DNA-Methylierungsanalyse als auch anhand der mRNA-Expressionsanalyse der vorliegend untersuchten neun Gene jeweils in der Gruppe mit der schlechten Prognose waren (Figur 9 C, Gruppe VI), wiesen eine ungünstige Prognose auf. Alle anderen Patienten (Figur 9C, Gruppe VII) zeigten einen Verlauf, welcher günstiger als der von Gruppe VI und ungünstiger als der von Gruppe V war.

### Beispiel 9: Bestimmung der Prognose eines Patienten mit einem malignen Melanom anhand der Promotor-Methylierung und der intragenischen Methylierung am Beispiel der immunregulatorischen Gene CD40 und LAG3

Es ist eine verbreitete Annahme, dass die DNA-Methylierung eines Gens invers mit der mRNA-Expression korreliert. Das heißt, dass eine hohe Methylierung des Gens mit einer geringen mRNA-Expression des Gens einhergeht. In diesem erfindungsgemäßen Ausführungsbeispiel konnte gezeigt werden, dass es in intragenischen Bereichen auch zu einer signifikanten positiven Korrelation kommen kann. Dies wurde am Beispiel der Gene *LAG3* und *CD40* gezeigt. Die Bestimmung der mRNA-Expression und der DNA-Methylierung wurde wie in den Beispielen 1 und 6 beschrieben durchgeführt. Die Berechnung der relativen Methylierung der Promotoren der beiden Gene *LAG3* und *CD40* erfolgte wie in Beispiel 4 beschrieben. Für die Bestimmung der intragenischen Bereiche wurden die im Folgenden genannten Bead-Paare verwendet und wie in Beispiel 1 beschrieben zueinander ins Verhältnis gesetzt, um einen relativen Methylierungswert für jeden Genlokus zu erhalten. Für die intragenische DNA-Methylierungsanalyse von *LAG3* wurden die Bead-Paare cg11429292 (SEQ ID NO:43) und cg14292870 (SEQ ID NO:44) verwendet, die beispielsweise die DNA-Methylierungsanalyse in den Bereichen SEQ ID NO:161 und SEQ ID NO:350 ermöglichen. Für die intragenische DNA-Methylierungsanalyse von *CD40* wurden das Bead-Paar cg06218285 (SEQ ID NO:50) verwendet, welches beispielsweise die DNA-Methylierungsanalyse in dem Bereich SEQ ID NO:297 ermöglicht. Figur 10 zeigt das Ergebnis erfindungsgemäßen Bestimmung der Prognose von 470 Patienten mit malignen Melanomen. Die Einteilung der Patienten erfolgte retrospektiv anhand der Analyse der Methylierung des Promotor- und des intragenischen Bereichs, jeweils für sich genommen und in Kombination. Figur 10A und Figur 10C zeigen, dass die relative Methylierung von CpG-Dinukleotiden, die sich im Bereich des Promoters befinden, negativ mit der mRNA-Expression des jeweiligen Gens korreliert ist. Dazu konnte gezeigt werden, dass die relative Methylierung der intragenischen Bereiche dieser Gene im Gegensatz zu den Promotorbereichen positiv und signifikant mit der mRNA-Expression korreliert ist (Figur 10B und Figur 10D). Figur 10E und 10H zeigen die bereits in Beispiel 4 dargestellten Ergebnisse für die DNA-Methylierungsanalyse der Gene *LAG3* und *CD40.*

Diejenigen Patienten mit erhöhter DNA-Methylierung (Gruppe II) im *LAG3* und *CD40* Promotor weisen einen ungünstigen Verlauf auf (Figur 10E und 10H). Diese Verhältnisse sind im intragenischen Bereich überraschenderweise umgedreht. Gruppe III in Figur 10F umfasst diejenigen Patienten, deren Tumoren eine intragenische Methylierung des *LAG3* Gens über 20,67% zeigten. Gruppe IV beinhaltet diejenigen Patienten, deren Tumoren eine Methylierung unter 20,67% zeigten. Diejenigen Patienten mit erniedrigter intragenischer Methylierung des *LAG3* Gens weisen einen ungünstigen Verlauf auf. Die Kaplan-Meier Überlebensanalyse des Verlaufs der Patienten in Abhängigkeit der kombinierten intragenischen und Promotor Methylierung des *LAG3* Gens zeigte eine verbesserte Bestimmung der Prognose (Figur 10G). Gruppe V umfasst diejenigen Patienten, die sich sowohl in Gruppe II als auch in Gruppe IV befinden. Gruppe VII umfasst diejenigen Patienten, die sich sowohl in Gruppe I als auch in Gruppe III befinden. Gruppe VI beinhaltet die Patienten, die entweder in den Gruppen II und III oder I und IV sind. Die Patienten, die sowohl anhand der intragenischen Methylierung als auch der Promotormethylierung des *LAG3* Gens eine ungünstige Prognose zeigten, bildeten eine Gruppe mit besonders ungünstigem Verlauf (Figur 10G, Gruppe V) .

Die Kaplan-Meier Überlebensanalyse des Verlaufs der Patienten in Abhängigkeit der Promotor- und der intragenischen Methylierung des *CD40* Gens sowie der kombinierten Analyse aus beiden Analysen (Figur 10H, 10I, 10J) führte zu vergleichbaren Ergebnissen wie diejenigen, welche mit *LAG3* erzielt wurden. Gruppe I (Figur 10H) umfasst diejenigen Patienten, deren Tumoren eine Methylierung unter 17,16% zeigten. Gruppe II beinhaltet diejenigen Patienten, deren Tumoren eine Methylierung über 17,16% zeigten (Figur 10H). Diejenigen Patienten mit erhöhter Methylierung im *CD40* Promotor wiesen einen ungünstigen Verlauf auf. Gruppe III (Figur 10I) umfasst diejenigen Patienten, deren Tumoren eine Methylierung über 35% zeigten. Gruppe IV (Figur 10I) beinhaltet diejenigen Patienten, deren Tumoren eine Methylierung unter 35% zeigten. Diejenigen Patienten mit erniedrigter intragenischer Methylierung des *CD40* Gens wiesen einen ungünstigen Verlauf auf. Gruppe V (Figur 10J) umfasst diejenigen Patienten, welche sich sowohl in Gruppe II als auch in Gruppe IV befinden. Gruppe VII (Figur 10J) umfasst diejenigen Patienten, welche sich sowohl in Gruppe I als auch in Gruppe III befinden. Gruppe VI beinhaltet diejenigen Patienten, welche entweder in den Gruppen II und III oder I und IV sind. Es konnte gezeigt werden, dass die Patienten, die sich sowohl anhand der DNA-Methylierungsanalyse des *CD40* Promotors als auch des intragenischen Bereichs in den Gruppen mit der guten Prognose befanden nun eine Gruppe mit einer besonders guten Prognose bildeten. Dadurch konnte durch die kombinierte DNA-Methylierungsanalyse des Promotors und des intragenischen Bereichs des *CD40* Gens die Bestimmung der Prognose gegenüber den einzelnen Analysen im Sinne einer differenzierteren Risikoeinschätzung verbessert werden.

### Beispiel 10: Bestimmung der Prognose von Patienten mit akuter myeloischer Leukämie anhand DNA-Methylierungsanalyse und mRNA-Expressionsanalyse von CD274, PDCD1 und PDCD1LG2

Das erfindungsgegenständliche Verfahren ermöglicht auch die Bestimmung der Prognose von Patienten mit malignen hämatologischen Erkrankungen. Die mRNA-Expressionsanalyse und die DNA-Methylierungsanalyse von malignen Zellen von 182 Patienten mit akuter myeloischer Leukämie wurde wie in den Beispielen 1 und 6 beschrieben durchgeführt. Die Berechnung der relativen DNA-Methylierung der Gene *CD274, PDCD1* und *PDCD1LG2* anhand der Ergebnisse der DNA-Methylierungsanalyse erfolgte wie in Beispiel 3 beschrieben.

Figur 11 zeigt Kaplan-Meier Analysen des Gesamtüberlebens der 182 Patienten mit akuter myeloischer Leukämie. Überraschenderweise zeigten die Patienten mit einer Methylierung der Gene *CD274, PDCD1* und *PDCD1LG2* unterhalb des Schwellenwerts (Gruppe I, Figur 11A, 11D, 11G) eine ungünstige Prognose während in den vorhergehenden Beispielen die Patienten mit einer erhöhten Methylierung dieser immunregulatorischen Gene eine ungünstigere Prognose aufwiesen. Es ist daher eine überraschende Erkenntnis, dass die erfindungsgemäße Verfahrensausführung auch die Bestimmung der Prognose von Patienten mit malignen hämatologischen Erkrankungen ermöglicht. Die Analyse der mRNA-Expression der Gene *CD274, PDCD1* und *PDCD1LG2* (Figur 11B, 11E, 11H) ermöglichte ebenfalls die Bestimmung der Prognose der Patienten. Es ist ebenfalls überraschend, dass auch bei der Analyse der mRNA-Expression diejenigen Patienten mit verminderter Expression (Gruppe IV) im Gegensatz zu den bisher beschriebenen Ergebnissen aus Beispiel 7 und 8 eine günstigere Prognose aufwiesen. Figur 11C, 11F und 11I zeigt, dass die kombinierte Analyse der DNA-Methylierung und der mRNA-Expression zu einer differenzierteren Risikoabschätzung gegenüber den jeweiligen Einzelanalysen von mRNA-Expression beziehungsweise DNA-Methylierung führt.

### Beispiel 11: Bestimmung der Prognose von Patienten mit akuter myeloischer Leukämie anhand der DNA-Methylierungsanalyse von TNFRSF9, TIGIT, BTLA, HAVCR2, CD80, CTLA4, ICOS, C10orf54, HHLA2, CD160, KIR2DL4 und KIR3DL1

Das erfindungsgemäße Verfahren ermöglicht die Bestimmung der Prognose von Patienten mit malignen hämatologischen Erkrankungen auch unter Anwendung weiterer immunregulatorischer Gene. Für die DNA-Methylierungsanalyse wurden die in Beispiel 10 erhobenen Daten verwendet. Die Bestimmung der relativen Methylierung von *TNFRSF9, TIGIT, BTLA* und *CTLA4* anhand der Daten für die Bead-Paare erfolgte wie in Beispiel 4 beschrieben. Für die Bestimmung der relativen Methylierung der Gene *HAVCR2, CD80, ICOS, C10orf54, HHLA2, CD160, KIR2DL4* und *KIR3DL1* wurden die im Folgenden genannten Bead-Paare verwendet und wie in Beispiel 1 beschrieben zur Berechnung der relativen Methylierung eingesetzt. Für *HAVCR2* wurde das Bead-Paar cg09574807 (SEQ ID NO:46) verwendet, welches die Bestimmung der Methylierung des Genbreichs SEQ ID NO:286 ermöglichte. Die Bestimmung der Methylierung von *CD80* erfolgte anhand der Bead-Paare cg12978275 (SEQ ID NO:126) und cg13458803 (SEQ ID NO:127), welche die Methylierungsanalyse des Sequenzbereichs SEQ ID NO:163 ermöglichten. Die DNA-Methylierungsanalyse des Gens *ICOS* basierte auf den Bead-Paaren cg18561976 (SEQ ID NO:112) und cg15344028 (SEQ ID NO:113), welche die Methylierungsanalyse der Sequenzbereiche SEQ ID NO:92, SEQ ID NO:93 und SEQ ID NO:95 erlauben. Die DNA-Methylierungsanalyse von *HHLA2* wurde anhand der vier Bead-Paare cg00915092 (SEQ ID NO:122), cg14703454 (SEQ ID NO:123), cg11326415 (SEQ ID NO:124) und cg22926869 (SEQ ID NO:125) durchgeführt, welche die Analyse der Sequenzbereiche SEQ ID NO:36, SEQ ID NO:195 und SEQ ID NO:196 ermöglichen. Für die DNA-Methylierungsanalyse der Sequenzbereiche SEQ ID NO:190 und SEQ ID NO:191 des Gens *C10orf54* wurde das Bead-Paar ch.10.1529706R (SEQ ID NO:35) verwendet. Die DNA-Methylierungsanalyse der Sequenzbereiche SEQ ID NO:361, SEQ ID NO:362 und SEQ ID NO:366 im *CD160* Gen erfolgte mittels der Bead-Paare cg12832565 (SEQ ID NO:150), cg10798745 (SEQ ID NO:151) und cg15892497 (SEQ ID NO:152). Für die DNA-Methylierungsanalyse der Sequenzbereiche SEQ ID NO:217 und SEQ ID NO:218 des Gens *KIR2DL4* wurde das Bead-Paar cg08326410 (SEQ ID NO:143) verwendet. Die DNA-Methylierungsanalyse von *KIR3DL1* wurde mittels der Bead-Paare cg15588997 (SEQ ID NO:137), cg08129658 (SEQ ID NO:138), cg02469067 (SEQ ID NO:139), cg19689800 (SEQ ID NO:140), cg06494497 (SEQ ID NO:141) und cg05720980 (SEQ ID NO:142) durchgeführt, welche die Methylierungsanalyse der Sequenzbereiche SEQ ID NO:230, SEQ ID NO:231 und SEQ ID NO:232 ermöglichen.

Figur 12 zeigt Kaplan-Meier Analyse des Gesamtüberlebens von 182 Patienten mit akuter myeloischer Leukämie. Die Patienten wurden stratifiziert anhand der DNA-Methylierungsanalyse der immunregulatorischen Gene *TNFRSF9* (Figur 12A), *TIGIT* (Figur 12B), *BTLA* (Figur 12C), *HAVCR2* (Figur 12D), *CD80* (Figur 12E), *CTLA4* (Figur 12F), *ICOS* (Figur 12G), *C10orf54* (Figur 12H), *HHLA2* (Figur 12I), *CD160* (Figur 12J), *KIR2DL4* (Figur 12K) und *KIR3DL1* (Figur 12L). Die Dichotomisierung erfolgte anhand von optimierten Schwellenwerten. Die Optimierung des Schwellenwerts wurde in der Art durchgeführt, dass eine optimale Trennung, das heißt ein möglichst niedriger p-Wert des Log-Rank Tests, von Patienten mit guter und schlechter Prognose möglich war. Für alle analysierten Gene konnte gezeigt werden, dass Patienten, deren maligne Erkrankung eine Methylierung unterhalb des Schwellenwerts aufwiesen (Gruppe I) einen ungünstigen Verlauf zeigen im Vergleich zu den Patienten mit einer Methylierung oberhalb des Schwellenwerts.

### Beispiel 12: Bestimmung der Prognose von Patienten mit niedriggradigen Gliomen anhand DNA-Methylierungsanalyse und mRNA-Expressionsanalyse von CD274, PDCD1 und PDCD1LG2

Das erfindungsgemäße Verfahren ermöglicht ebenfalls die Bestimmung der Prognose bei Patienten mit Gliomen. Beispielsweise wurde eine DNA-Methylierungsanalyse und mRNA-Expressionsanalyse der immunregulatorischen Gene *CD274, PDCD1* und *PDCD1LG2* bei 510 Patienten mit niedriggradigen Gliomen durchgeführt. Die Präparation von DNA und RNA, die mRNA-Expressionsanalyse und die DNA-Methylierungsanalyse sowie deren Auswertung erfolgte wie in den Ausführungsbeispielen 1 und 6 beschrieben. Die Berechnung der relativen Methylierung der Gene *CD274, PDCD1* und *PDCD1LG2* anhand der mittels dem Illumina HumanMethylation450 BeadChip generierten Daten erfolgte wie in Ausführungsbeispiel 3 beschrieben.

Wie bereits in Beispiel 11 für maligne hämatologische Erkrankungen gezeigt, kann auch bei Patienten mit Gliomen die Prognose anhand der DNA-Methylierungsanalyse, der mRNA-Expressionsanalyse und der Kombination aus beiden Analysen bestimmt werden. Figur 13 zeigt, dass diejenigen Patienten, welche eine Methylierung der immunregulatorischen Gene in den Zellen des Glioms aufweisen, die unterhalb des Schwellenwerts liegt (Gruppe I), eine deutlich schlechtere Prognose hatten als Patienten, deren Tumoren eine Methylierung oberhalb des Schwellenwerts aufwiesen (Gruppe II). Dies konnte für die drei Gene *CD274* (Figur 13A), *PDCD1* (Figur 13D) und *PDCD1LG2* (Figur 13G) gleichermaßen gezeigt werden. Bei der Analyse der entsprechenden mRNA-Expression der immunregulatorischen Gene konnten ebenfalls die Patienten mit einer schlechten Prognose anhand einer erhöhten Expression der Gene identifiziert werden (Gruppe III). Als besonders vorteilhaft erwies sich wiederum die erfindungsgemäße Kombination aus mRNA-Expressionsanalyse und DNA-Methylierungsanalyse, wodurch eine Bildung von drei Prognosegruppen ermöglicht wurde, welche ein hohes Risiko (Gruppe I+III), ein mittleres Risiko (Gruppe I+IV/II+III) und ein niedriges Risiko (Gruppe II+IV) für das Eintreten des Todes hatten. Wie schon für die malignen hämatologischen Erkrankungen in den Beispielen 10 und 11 gezeigt wurde, ist es auch bei den Gliomen ein überraschender Befund, dass im Gegensatz zu den vorhergehenden Analysen maligner Erkrankungen hier eine verminderte mRNA-Expression und eine erhöhte DNA-Methylierung mit einer guten Prognose assoziiert war. Dies unterstreicht die Leistungsfähigkeit der vorliegenden Erfindung in Bezug auf die Bestimmung der Prognose bei Patienten mit verschiedensten malignen Erkrankungen.

### Beispiel 13: Bestimmung der Prognose von Patienten mit niedriggradigen Gliomen anhand der DNA-Methylierungsanalyse und mRNA-Expressionsanalyse von CD80, CTLA4, ICOS und CD276

Die erfindungsgemäße Bestimmung der Prognose von Patienten mit Gliomen ist auch anhand der DNA-Methylierung und mRNA-Expression der immunregulatorischen Gene *CD80, CTLA4, ICOS* und *CD276* möglich. Die vorliegende Analyse basiert auf den Methylierungs- und Expressionsdaten, die wie in Beispiel 12 beschrieben erhoben wurden. Die Bestimmung der relativen DNA-Methylierung der Gene *CD80, CTLA4* und *ICOS* erfolgte wie in den Beispielen 11 und 4 beschrieben. Für die Bestimmung der relativen DNA-Methylierung des *CD276* Gens wurden die Bead-Paare cg24688248 (SEQ ID NO:119), cg14910296 (SEQ ID NO:120) und cg12524179 (SEQ ID NO:121) verwendet und wie in Beispiel 1 beschrieben rechnerisch ausgewertet. Diese Bead-Paare erlauben eine Bestimmung der DNA-Methylierung für die Sequenzbereiche SEQ ID NO:181, SEQ ID NO:182 und SEQ ID NO:183.

Figur 14 zeigt die Kaplan-Meier Analyse des Gesamtüberlebens der analysierten 510 Patienten mit niedriggradigen Gliomen. Die Patienten wurden stratifiziert anhand der DNA-Methylierungsanalyse (Figur 14A, 14D, 14G, 14J), der mRNA-Expressionsanalyse (Figur 14B, 14E, 14H, 14K) und der kombinierten DNA-Methylierungs- und mRNA-Expressionsanalyse (Figur 14C, 14F, 14I, 14L) der immunregulatorischen Gene *CD80* (Figur 14 A, B, C), *CTLA4* (Figur 14 D, E, F), *ICOS* (Figur 14G, 14H, 14I), *CD276* (14J, 14K, 14L). Für die Dichotomisierung anhand der DNA-Methylierung wurden folgende Schwellenwerte gewählt: 90,76% *(CD80),* 86,43% *(CTLA4),* 84,75% *(ICOS)* und 30,08% (*CD276*). Für die Dichotomisierung der mRNA-Expression wurde der Median als Schwellenwert angewendet. Gruppe I umfasst Patienten mit einer Methylierung unterhalb des Schwellenwerts, Gruppe II wird von Patienten mit einer Methylierung oberhalb des Schwellenwerts gebildet. Gruppe III umfasst Patienten, deren Tumoren eine mRNA-Expression oberhalb des Medians aufwiesen, während Patienten aus Gruppe IV eine mRNA-Expression im Tumor unterhalb des Medians zeigten. Wie bereits in Beispiel 12 und Figur 13 für *CD274, PDCD1* und *PDCD1LG2* gezeigt wurde, konnte auch anhand der DNA-Methylierung und der mRNA-Expression der Gene *CD80, CTLA4, ICOS* und *CD276* sowie der Kombination von beiden Analysen die Prognose der Patienten bestimmt werden. Auch in diesem Fall war es eine überraschende Erkenntnis, dass wieder eine hohe DNA-Methylierung und eine niedrige mRNA-Expression der erfindungsgegenständlichen immunregulatorischen Gene mit einem günstigeren Krankheitsverlauf einhergingen.

### Beispiel 14: Bestimmung der Prognose von Patienten mit niedriggradigen Gliomen anhand der DNA-Methylierungsanalyse von TNFRSF25, TNFRSF9, CD40, TIGIT, BTLA, HAVCR2, C10orf54, HHLA2, LAG3, CD160, KIR2DL4 und KIR3DL1

Es ist ebenfalls möglich, die Prognose von Patienten mit niedriggradigen Gliomen anhand der DNA-Methylierungsanalyse weiterer immunregulatorischer Gene zu bestimmen. Die in Figur 15 dargestellten Ergebnisse basieren auf einer Analyse der DNA-Methylierungsdaten, welche wie in Beispiel 12 beschrieben generiert wurden. Die für die Bestimmung der relativen DNA-Methylierung der Gene *TNFRSF9, CD40, TIGIT, BTLA, HAVCR2, C10orf54, HHLA2, LAG3, CD160, KIR2DL4* und *KIR3DL1* verwendeten Bead-Paare und die Berechnung der DNA-Methylierung ist in den Beispielen 1, 4 und 11 beschrieben. Für die Bestimmung der DNA-Methylierung des Gens *TNFRSF25* können beispielsweise die in diesem Beispiel verwendeten Bead-Paare cg27224823 (SEQ ID NO:153), cg13331246 (SEQ ID NO:154), cg23588699 (SEQ ID NO:155) und cg10982045 (SEQ ID NO:156) verwendet werden, mit denen die DNA-Methylierungsanalyse der Sequenzbereiche SEQ ID NO:317, SEQ ID NO:318 und SEQ ID NO:319 ermöglicht ist. Es können ebenfalls die Bead-Paare cg00087884 (SEQ ID NO:157), cg10059687 (SEQ ID NO:158) und cg11756870 (SEQ ID NO:159) für die DNA-Methylierungsanalyse der Sequenzbereiche SEQ ID NO:317, SEQ ID NO:320 und SEQ ID NO:321 herangezogen werden.

Figur 15 zeigt die Kaplan-Meier Analyse des Gesamtüberleben von 510 Patienten mit niedriggradigen Gliomen, die anhand anhand der DNA-Methylierungsanalyse der Gene *TNFRSF25* (A), *TNFRSF9* (B), *CD40* (C), *TIGIT* (D), *BTLA* (E), *HAVCR2* (F), *C10orf54* (G), *HHLA2* (H), *LAG3* (I), *CD160* (J), *KIR2DL4* (K) und *KIR3DL1* (L) retrospektiv stratifiziert wurden. Die Stratifizierung der Patienten erfolgte anhand einer dichotomisierten relativen Methylierung der Gene, wobei folgende Schwellenwerte zugrunde gelegt wurden: 61,88% *(TNFRSF25),* 79,24% *(TNFRSF9),* 43,24% *(CD40),* 80,66% *(TIGIT),* 84,64% (*BTLA*), 2,495% *(HAVCR2),* 66,52% *(C10orf54),* 77,14% *(HHLA2),* 74,34% *(LAG3),* 92,89% *(CD160),* 90,85% *(KIR2DL4)* und 52,28% *(KIR3DL1).* Gruppe I umfasst Patienten mit einer DNA-Methylierung unterhalb des Schwellenwerts; Gruppe II bildet diejenigen Patienten ab, deren Tumor eine DNA-Methylierung oberhalb des Schwellenwerts zeigte. Für alle immunregulatorischen Gene konnte gezeigt werden, dass eine niedrige DNA-Methylierung mit einer schlechten Prognose der Patienten assoziiert ist.

### Beispiel 15: Bestimmung der Prognose von Patienten mit klarzelligen Nierenzellkarzinomen anhand der DNA-Methylierungsanalyse von CD274, TNFRSF9, TIGIT, CD80, CTLA4, CD276 und HHLA2

In den vorangegangenen Ausführungsbeispielen konnte bereits gezeigt werden, dass das erfindungsgemäße Verfahren die Bestimmung der Prognose von Patienten mit verschiedenen malignen Erkrankungen, beispielsweise Adenokarzinomen, Plattenepithelkarzinomen, Melanomen, Leukämien und Gliomen ermöglicht. In diesem Beispiel wurde das erfindungsgemäße Verfahren bei Patienten mit klarzelligen Nierenzellkarzinomen angewendet. Die DNA-Methylierungsdaten wurden wie in Beispiel 1 beschrieben generiert. Die Berechnung der relativen DNA-Methylierung anhand der Daten aus den Bead-Paaren für die Gene *CD274, TNFRSF9, TIGIT, CD80, CTLA4, CD276* und *HHLA2* erfolgte wie in den Beispielen 3, 4, 11 und 13 beschrieben.

Figur 16 zeigt die Kaplan-Meier Analysen des Gesamtüberlebens von 318 Patienten mit klarzelligen Nierenzellkarzinomen, welche anhand der DNA-Methylierung der immunregulatorischen Gene *TIGIT* (A), *TNFRSF9* (B), *CD274* (C), *CD80* (D), *CTLA4* (E), *CD276* (F) und *HHLA2* (G) retrospektiv stratifiziert wurden. Die Dichotomisierung der relativen DNA-Methylierungswerte erfolgte für jedes Gen anhand des jeweiligen Medians der DNA-Methylierung von allen Patienten. Gruppe I umfasst die Patienten, deren Tumoren eine DNA-Methylierung unterhalb des Medians hatten, während in Gruppe II diejenigen Patienten sind, deren Tumoren eine DNA-Methylierung oberhalb des Medians aufwiesen. Figur 16 zeigt, dass bei sechs der sieben analysierten Gene eine erhöhte DNA-Methylierung (Gruppe I) signifikant mit einer ungünstigeren Prognose korrelierte. Für das Gen *HHLA2* konnte überraschenderweise gezeigt werden, dass die Prognose von Patienten mit klarzelligen Nierenzellkarzinomen ungünstig ist, wenn das immunregulatorische Gen eine Methylierung unterhalb des Schwellenwertes aufwies (Figur 16G). Die Ergebnisse demonstrieren, dass das erfindungsgegenständliche Verfahren für weitere Karzinome neben den in den vorangegangenen Beispielen beschriebenen Adeno- und Plattenepithelkarziomen anwendbar ist.

### Beispiel 16: Bestimmung der Prognose von Patienten mit klarzelligen Nierenzellkarzinomen anhand der DNA-Methylierungsanalyse in Kombination mit der mRNA-Expressionsanalyse von TNFRSF9, TIGIT, CD80, CTLA4, CD276 und HHLA2

Die universelle Anwendbarkeit des erfindungsgemäßen Verfahrens bei verschiedensten malignen Erkrankungen wurde weiterhin im Rahmen einer kombinierten Analyse der mRNA-Expression und der DNA-Methylierung für die Gene *TNFRSF9, TIGIT, CD80, CTLA4, CD276* und *HHLA2* bei 318 Patienten mit klarzelligen Nierenzellkarzinomen verifiziert. Die DNA-Methylierungsanalyse und mRNA-Expressionsanalyse wurde wie in Beispiel 1 und 6 beschrieben durchgeführt und ausgewertet. Die Berechnung der relativen Methylierung der Gene anhand der verwendeten Bead-Paare ist in den Beispielen 4, 11 und 13 beschrieben.

Figur 17 zeigt die Kaplan-Meier Analyse des Gesamtüberlebens der 318 Patienten mit klarzelligen Nierenzellkarzinomen, welche anhand der kombinierten Analyse von DNA-Methylierung und mRNA-Expression der Gene *TIGIT* (A), *TNFRSF9* (B), *CD80* (C), *CTLA4* (D), *CD276* (E) und *HHLA2* (F) stratifiziert wurden. Die Dichotomisierung erfolgte anhand des Medians der Methylierung beziehungsweise der mRNA-Expression aller Patienten. Gruppe I: Methylierung unterhalb und mRNA oberhalb des Medians; Gruppe III: Methylierung oberhalb und mRNA unterhalb des Medians; Gruppe II: Methylierung oberhalb und mRNA oberhalb des Medians oder Methylierung unterhalb und mRNA unterhalb des Medians. Für die sechs in diesem Beispiel analysierten Gene konnte gezeigt werden, dass eine Einteilung der Patienten in drei Gruppen mit je hohem, mittleren und niedrigem Risiko für das Eintreten des Todes möglich war.

Während bei den Genen *TIGIT* (A), *TNFRSF9* (B), *CD80* (C), *CTLA4* (D) und *CD276* (E) eine hohe DNA-Methylierung und eine niedrigere mRNA-Expression (Gruppe I) mit einer besonders schlechten Prognose assoziiert war, ergibt sich für das Gen *HHLA2* (F) ein genau umgekehrter Zusammenhang, welcher auch das bereits in Beispiel 15 für die alleinige DNA-Methylierungsanalyse dieses Gens beschriebene überraschende Ergebnis bestätigt.

Wie bereits in den Ausführungsbeispielen 13, 12, 10, 8 und 7 gezeigt werden konnte, stellt die kombinierte Analyse der DNA-Methylierung und der mRNA-Expression von erfindungsgegenständlichen immunregulatorischen Genen eine besonders vorteilhafte Verfahrensausgestaltung dar, indem eine differenziertere Prognosestellung gegenüber den Einzelanalysen möglich wird.

### Beispiel 17: Bestimmung des Ansprechens von Patienten mit malignen Melanomen auf eine Immuntherapie anhand der DNA-Methylierungsanalyse von PDCD1 und CD274

Die erfindungsgemäße Anwendung der DNA-Methylierungsanalyse eines immunregulatorischen Gens zur Vorhersage des Ansprechens auf eine Immuntherapie wurde anhand einer Kohorte von 23 Patienten mit malignem Melanom überprüft. Die Patienten erhielten eine Immuntherapie mit Pembrolizumab. Dieser Wirkstoff ist ein monoklonaler Antikörper, der gegen den durch das immunregulatorische Gen *PDCD1* codierten Rezeptor PDCD1 gerichtet ist. Der Antikörper verhindert durch Wechselwirkung mit dem Rezeptor PDCD1 die Bindung der entsprechenden durch *CD274* und *PDCD1LG2* codierten Liganden und verändert auf diese Weise die immunregulatorische Wirkung sowohl von PDCD1 als auch von CD274 und/oder PDCD1LG2. Von den 23 Patienten zeigten 11 ein Fortschreiten der Erkrankung (Patienten 13 bis 23), bei 11 Patienten (Patienten 2 bis 12) blieb die Erkrankung auf einem stabilen Niveau oder die Patienten zeigten einen Rückgang der Tumormasse. Bei einem Patienten (Patient 1) ging der Tumor soweit zurück, dass er nicht mehr nachweisbar war.

Für die DNA-Methylierungsanalyse der immunregulatorischen Gene *PDCD1* und *CD274* wurde DNA aus Zellen von kutanen Metastasen, Lymphknotenmetastasen und Fernmetastasen der Tumorpatienten gewonnen. Die Metastasen wurden vor Beginn der Immuntherapie chirugisch entnommen. Die Umwandlung in Bisulfit-konvertierte DNA wurde wie in Beispiel 2 beschrieben durchgeführt. Die erfindungsgemäße DNA-Methylierungsanalyse der immunregulatorischen Gene erfolgte mittels quantitativer Echtzeit-PCR wie in den Beispielen 2 und 5 für jeweils *CD274* und *PDCD1* beschrieben.

Figur 18 zeigt die patientenabhängige DNA-Methylierung des immunregulatorischen Gens *CD274,* welches für den Liganden CD274 codiert, der an den durch das immunregulatorische Gen *PDCD1* codierten Rezeptor PDCD1 bindet. Die Patienten 13 bis 23, welche kein Ansprechen auf die Therapie mit Pembrolizumab zeigen, weisen durchschnittlich eine DNA-Methylierung von 18,5% im Tumor auf, während die Gruppe der Patienten, die teilweise oder komplett anspricht (Patienten 1 bis 12), durchschnittlich nur nur 6,2% DNA-Methylierung im Tumor aufwies.

Die Ergebnisse zeigen am Beispiel der DNA-Methylierungsanalyse des *CD274* Gens und des Wirkstoffs Pembrolizumab, dass mit Hilfe des erfindungsgemäßen Verfahren das Ansprechen auf eine Immuntherapie vorhergesagt werden kann. Insbesondere zeigen die Ergebnisse, dass es möglich ist, die Patienten anhand der DNA-Methylierung zum Beispiel derart auszuwählen, dass nur diejenigen Patienten eine Therapie mit Pembrolizumab bekommen, die eine niedrige DNA-Methylierung, beispielsweise unter 10%, des *CD274* Gens aufweisen, da bei den Patienten mit hoher DNA-Methylierung die Wahrscheinlichkeit des Ansprechens auf die Therapie im vorliegenden Beispiel geringer ist. Von den Patienten 1 bis 12 mit teilweisem oder kompletten Ansprechen weisen zehn (83%) eine DNA-Methylierung von *CD274* unter 10% aus und konnten so in diesem Beispiel zutreffend als Patienten mit voraussichtlichem Ansprechen auf die Therapie identifiziert werden. Insgesamt zeigten acht Patienten eine *CD274* Methylierung von über 10%. Für sechs dieser acht Patienten (75%) konnten richtigerweise ein Ausbleiben des Ansprechens auf die Immuntherapie vorausgesagt werden.

Dieses Beispiel zeigt, dass die erfindungsgemäße Bestimmung der DNA-Methylierung eines immunregulatorischen Gens grundsätzlich geeignet ist, das Ansprechen auf eine Immuntherapie vorherzusagen. Dies ist demzufolge auch dann möglich, wenn das immunregulatorische Gen für einen Liganden als Immun-Checkpoint codiert und ein Wirkstoff eingesetzt wird, welcher dazu bestimmt ist, die immunregulatorische Wirkung des Liganden zu verändern, indem er den entsprechenden Rezeptor des Liganden inhibiert. Damit konnte auch gezeigt werden, dass die erfindungsgemäße DNA-Methylierungsanalyse eines immunregulatorischen Gens auch dafür geeignet ist, das Ansprechen auf einen Wirkstoff vorherzusagen, der antagonistisch mit einem Immun-Checkpoint wechselwirkt, der durch ein anderes immunregulatorisches Gen codiert ist.

Figur 19 zeigt die entsprechende erfindungsgemäße Bestimmung der DNA-Methylierung des immunregulatorischen Gens *PDCD1.* Bei der Gruppe der Patienten 1 bis 12, welche ein Ansprechen zeigte, wurde durchschnittlich 59% Methylierung des *PDCD1* Gens in den Tumorproben gefunden. Diese Methylierung war mit einem p-Wert eines t-Tests von 0,008 signifikant höher als die Methylierung in den Tumoren der Patienten, die ein Fortschreiten der Erkrankung zeigten und bei denen die mittlere Methylierung bei 22% lag. Die Höhe der DNA-Methylierung des *PDCD1* Gens korreliert somit ebenfalls mit dem Ansprechen auf die Immuntherapie und ermöglicht, anhand der DNA-Methylierung von *PDCD1* vor Start der Therapie diejenigen Patienten zu identifizieren, die vorraussichtlich auf die Therapie ansprechen. Beispielsweise zeigten die Tumoren von neun der zwölf (75%) ansprechenden Patienten (Patienten 1 bis 12) eine DNA-Methylierung des *PDCD1* Gens von über 25% und konnten somit richtigerweise als anprechende Patienten identifiziert werden. Der Patient mit komplettem Rückgang des Tumor durch die Immuntherapie (Patient 1) wies vorliegend mit 98% die von allen untersuchten Proben höchste Methylierung auf und konnte somit besonders zuverlässig identifiziert werden. Dagegen zeigten neun der elf (82%) Patienten (Patienten 13-23), die nicht auf die Therapie ansprachen, eine *PDCD1* Methylierung von unter 25% und konnten richtigerweise als Patienten identifiziert werden, welche nicht auf die ausgewählte Immuntherapie mit Pembrolizumab ansprechen. Solchen Patienten könnte die Anwendung des erfindungsgemäßen Verfahrens zukünftig beispielsweise eine Immuntherapie mit Pembrolizumab und dessen Nebenwirkungen ersparen. Gleichzeitig könnte wertvolle Zeit gewonnen werden, indem bei diesen Patienten frühzeitiger eine andere Therapie angewendet wird, die mit größerer Wahrscheinlichkeit Wirkung zeigt. Beispielsweise hätte eine individualisierte Auswahl der alternativen Therapie durch die erfindungsgemäße Anwendung des Verfahrens zur Bestimmung der DNA-Methylierung weiterer für Immun-Checkpoints codierender immunregulatorischer Gene vorgenommen werden können, um das Ansprechen auf eine Therapie mit Wirkstoffen, die in der Lage sind die immunregulatorische Wirkung dieser weiteren Immun-Checkpoints zu verändern, vorauszusagen.

Die Figur 19 zeigt auch beispielhaft, dass die Berücksichtigung der quantitativen Methylierung eines immunregulatorischen Gens die Vorhersage des Therapieansprechens ermöglichen kann. Dadurch lässt sich für einen Patienten auch ohne Anwendung eines Schwellenwerts ein Therapieansprechen vorhersagen. Im vorliegenden Beispiel zeigt der Patient 1, bei dem der Tumor komplett zurück ging, eine höhere Methylierung des *PDCD1* Gens als die Patienten 2 bis 12, die nur teilweise auf die Therapie ansprachen.

Es ist auch möglich, die erfindungsgemäße Anwendung der DNA-Methylierungsanalyse von verschiedenen immunregulatorischen Genen zu kombinieren, um so das Ansprechen auf eine Immuntherapie noch präziser voraussagen zu können. Aus den Figuren 18 und 19 ist ersichtlich, dass der Patient 1, welcher einen kompletten Rückgang des Tumor zeigte, sowohl eine geringe *CD274* DNA-Methylierung (3,6%), als auch eine hohe Methylierung von *PDCD1* (98%) im Tumor aufwies. *CD274* codiert für den Liganden, der an den durch *PDCD1* codierten Rezeptor bindet. Pembrolizumab ist ein Wirkstoff, der die immunregulatorische Wirkung der beiden durch diese Gene codierten Immun-Checkpoints durch eine Unterbrechung oder Inhibition dieser Liganden-Rezeptor-Bindung verändert. Dieser Befund zeigt, dass die erfindungsgemäße Analyse mehrerer Immun-Checkpoints, insbesondere solcher, die an derselben Liganden-Rezeptor-Bindung beteiligt sind, die Vorhersage des Ansprechens auf eine Immuntherapie weiter verbessern kann. Dies lässt sich im vorliegenden Beispiel auch daran zeigen, dass von den 12 Patienten (Patienten 1 bis 12), die zumindest teilweise auf die Therapie ansprachen, alle (100%) entweder durch eine *CD274* Methylierung unter 10% oder durch eine *PDCD1* Methylierung über 25% charakterisiert waren. Somit konnte für diese Patienten richtigerweise das Ansprechen auf die Immuntherapie vorhergesagt werden. Von den 11 Patienten mit ausbleibendem Therapieansprechen (Patienten 13-23) zeigten 5 (45%) eine *CD274* Methylierung über 10% und eine *PDCD1* Methylierung kleiner als 25% (Patienten 14 bis 16, 19 und 21). Für diese Patienten konnte das Ausbleiben des Therapieansprechens anhand der kombinierte DNA-Methylierungsanalyse von *PDCD1* und *CD274* besonders zuverlässig vorhergesagt werden.

### Beispiel 18: Bestimmung der Prognose von Patienten mit Plattenepithelkarzinomen des Kopf- und Halsbereichs anhand der DNA-Methylierungsanalyse und der mRNA-Expressionsanalyse verschiedener immunregulatorischer Gene

Die DNA-Methylierungsanalyse erfolgte wie in Beispiel 1 beschrieben. Für die Berechnung der relativen Methylierung der einzelnen in Tabelle 1 beschriebenen immunregulatorischen Gene wurden die in Tabelle 1 aufgeführten Bead-Paare des Infinium HumanMethylation450 BeadChip (Illumina, Inc., San Diego, CA, USA) verwendet. Die Zielsequenz dieser Bead-Paare im Genom ist ebenfalls in Tabelle 1 durch eine entsprechende SEQ ID NO gekennzeichnet. Für die Bead-Paare wurde jeweils eine relative Methylierung für jeden Patienten berechnet, indem die für den unmethylierten und den methylierten Zustand spezifischen Beads eines Paares wie in Beispiel 1 analog beschrieben zueinander ins Verhältnis gesetzt wurden. Untersucht wurde wie in Beispiel 5 das Gesamtüberleben von 528 Patienten mit Plattenepithelkarzinomen des Kopf- und Halsbereichs.

Die Bestimmung der mRNA-Expression erfolgte wie in Beispiel 6 beschrieben. Untersucht wurden die in Tabelle 2 aufgeführten mRNAs.

Die in Tabelle 1 zusammengefassten Ergebnisse zeigen, dass die erfindungsgemäße Bestimmung der DNA-Methylierung der immunregulatorischen Gene signifikant mit dem Überleben der Patienten assoziiert ist und damit die Bestimmung der Prognose der Patienten erlaubt. Die Hazard Ratio 1 wurde mittels des Cox Proportional Hazard Modells bestimmt und beschreibt, inwiefern sich das Risiko des Patienten zu versterben mit dem Anstieg des DNA-Methylierungswertes des entsprechenden immunregulatorischen Gens erhöht. Hazard Ratios größer als eins bedeuten, dass das Risiko des Patienten zu versterben mit höherem DNA-Methylierungswert größer wird. Bei einer Hazard Ratio kleiner als eins verringert sich dieses Risiko.

Dieses Beispiel zeigt zum einen, dass die erfindungsgemäße DNA-Methylierungsanalyse der in Tabelle 1 aufgelisteten immunregulatorischen Gene die Bestimmung der Prognose der Patienten erlaubt. Des Weiteren zeigt dieses Beispiel, dass die Bestimmung der Prognose nicht nur durch Einteilung von Patienten anhand von Methylierungsschwellenwerten in Gruppen mit unterschiedlicher Prognose möglich ist, sondern auch anhand von statistischen Modellen, wie beispielsweise dem Cox Proportional Hazards Modell, mit denen bestimmt werden kann, inwiefern sich die Prognose eines Patienten in Abhängigkeit von dem jeweiligen Methylierungswert ändert.

Anhand der Genbeispiele *ADORA2A, BTNL2, C10orf54, CD160, CD276, CD48, CD80, CD86, PDCD1, TIGIT* und *TNFRSF18* ist in Tabelle 1 außerdem gezeigt, dass verschiedene Bereiche innerhalb eines Gens für die erfindungsgemäße Verfahrensanwendung geeignet sein können. Dies geht daraus hervor, dass bei diesen Genen die DNA-Methylierungsanalyse einer Vielzahl von Bereichen, die mittels der verschiedenen Bead-Paare untersucht wurden, jeweils eine erfolgreiche Bestimmung der Prognose ermöglicht.

Hazard Ratio 2 in Tabelle 1 beschreibt, inwiefern das Risiko der Patienten mit Tumoren, deren Methylierungswert oberhalb eines Schwellenwertes liegt, höher ist zu versterben im Vergleich zu den Patienten mit Werten unterhalb des Schwellenwertes. Beispielsweise konnte im Fall des immunregulatorischen Gens *CD274* überraschenderweise für die beiden Bereiche mit den SEQ ID NO:425 und SEQ ID NO:426 gezeigt werden, dass eine erhöhte Methylierung mit einem niedrigen Risiko zu versterben einhergeht, während in Beispiel 3 gezeigt wurde, dass die erhöhte Methylierung anderer Bereiche des *CD274* Gens beim malignen Melanom mit einem schlechteren Überleben assoziiert ist. Ein ähnlicher Befund ergab sich für *CD276.* Die DNA-Methylierungsanalyse der Bereiche *von CD276,* die durch die SEQ ID NO:427, SEQ ID NO:428 und SEQ ID NO:429 abgedeckt sind, korreliert invers mit dem Überleben, das heißt eine erhöhte Methylierung dieser Bereiche geht mit einem geringeren Versterbensrisiko einher. Dagegen ist die Methylierung des Bereichs mit der SEQ ID NO:430 positiv mit dem Risiko zu versterben korreliert. Es stellt somit einen besonderen Vorteil der erfindungsgemäßen DNA-Methylierungsanalyse dar, dass über die Wahl eines oder mehrerer untersuchter Bereiche im Gen eine noch differenziertere Prognose erreicht werden kann, als dies beispielsweise bei einer alleinigen Betrachtung der mRNA-Expression oder Proteinexpression möglich ist.

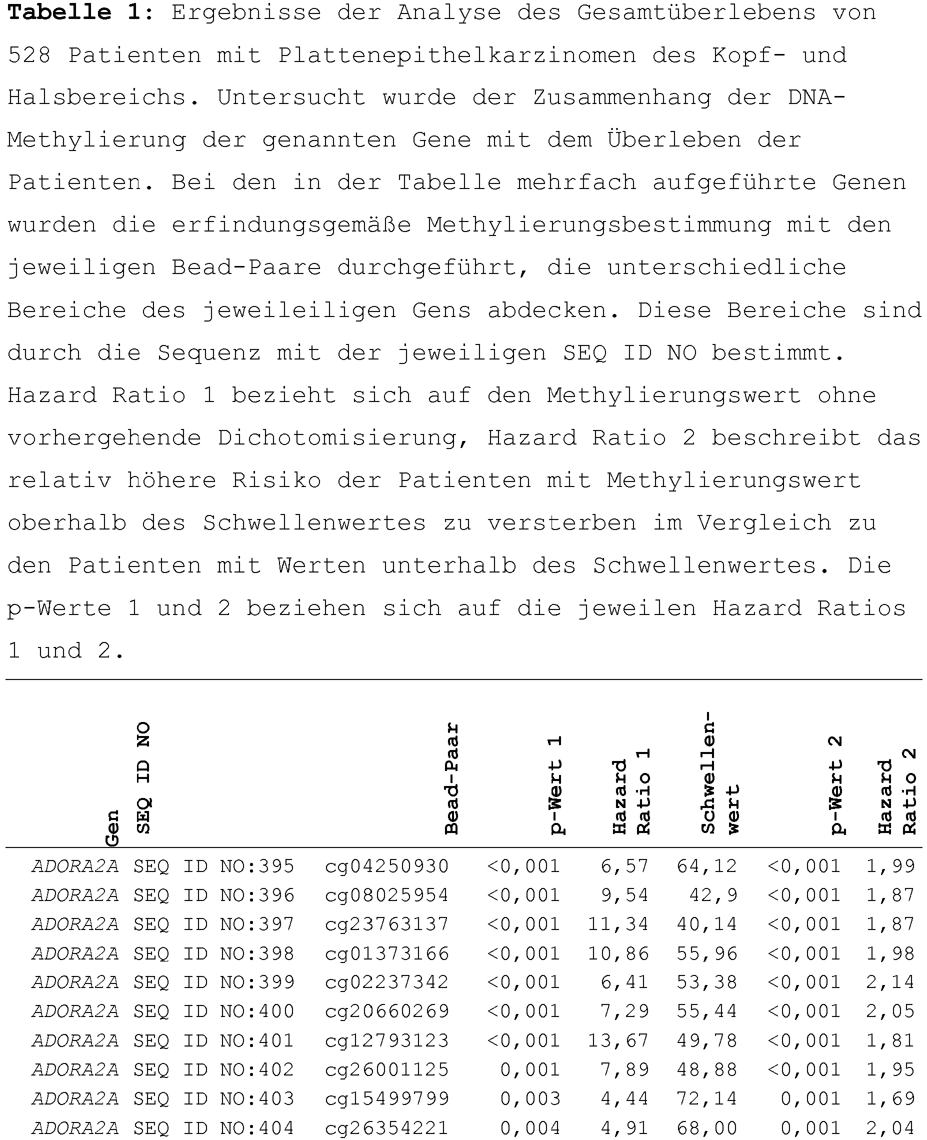

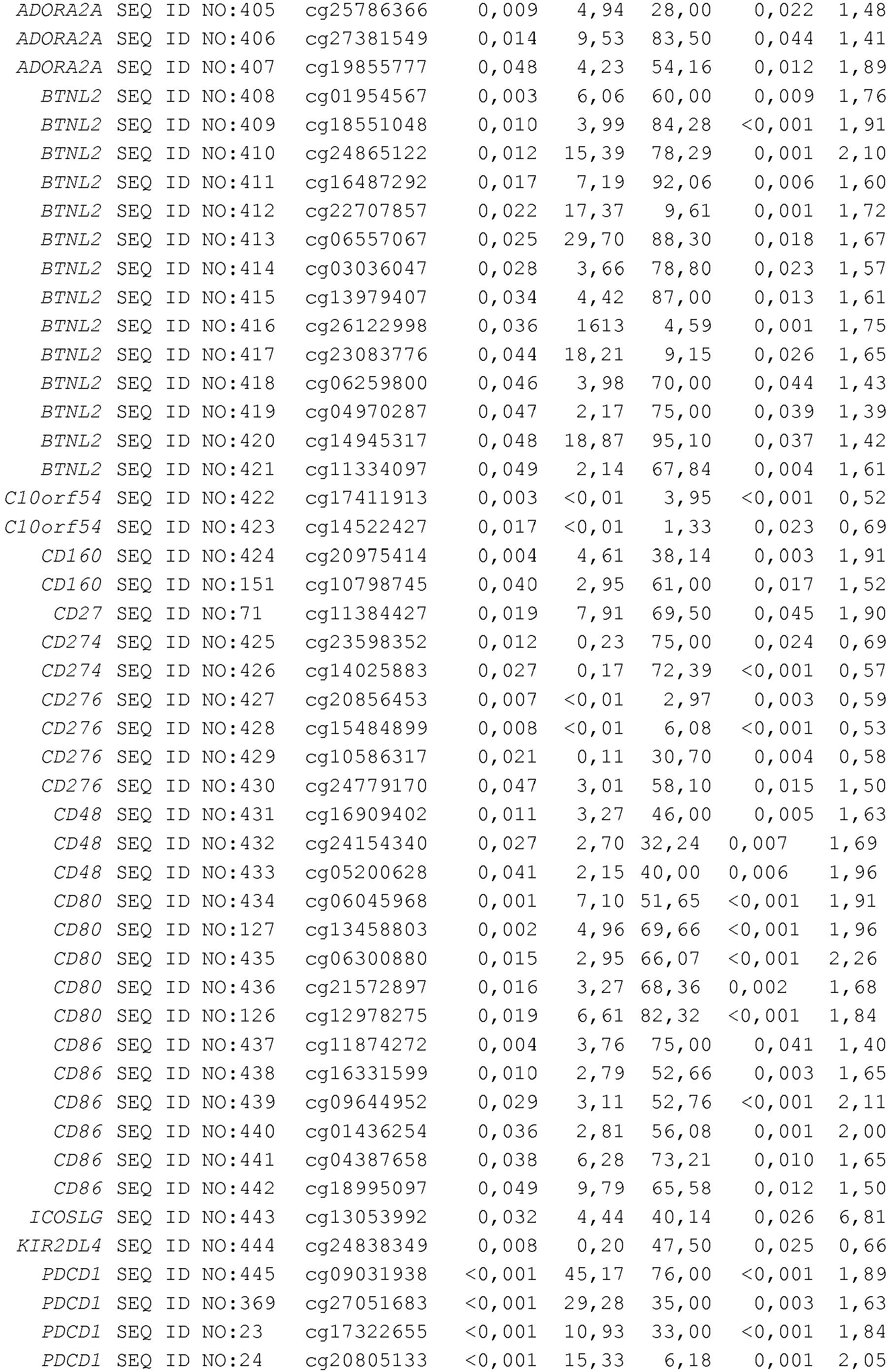

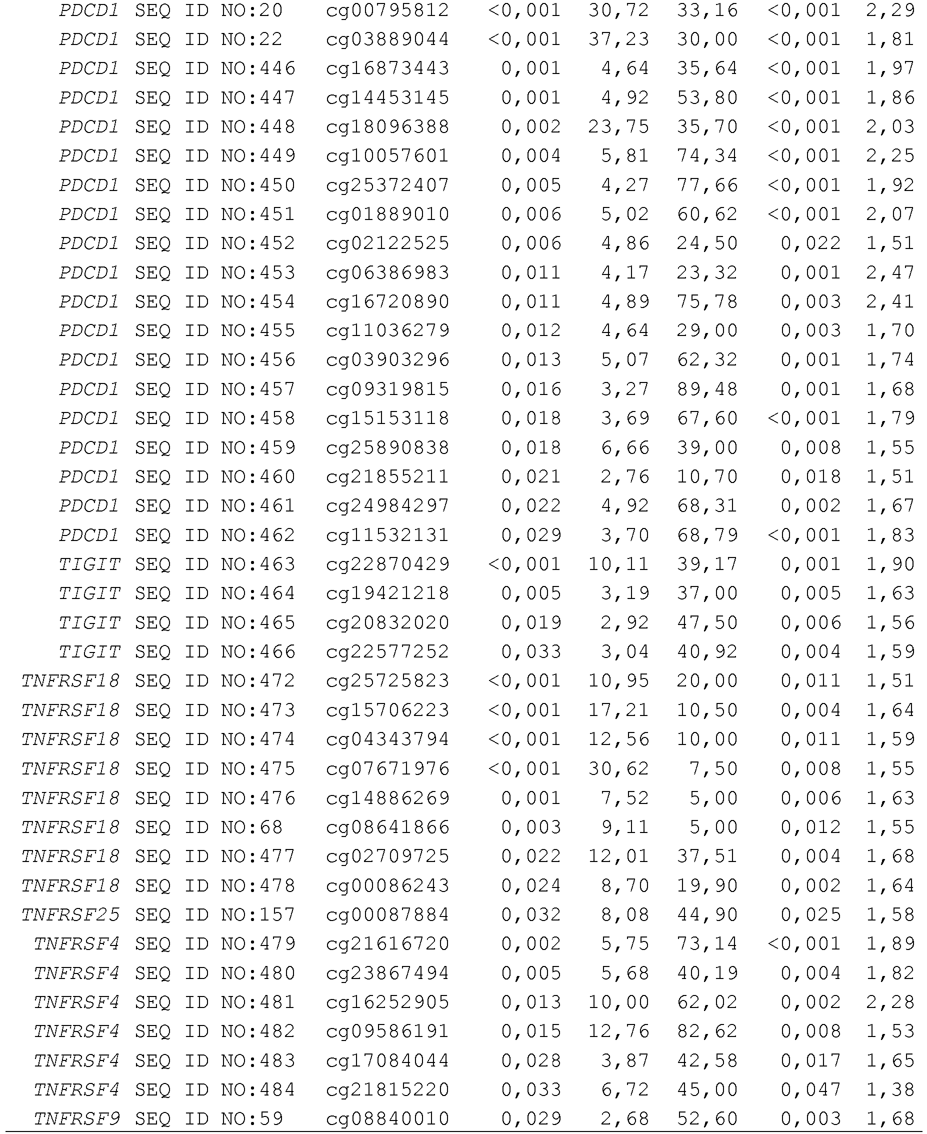

Tabelle 2 zeigt am Beispiel der genannten immunregulatorischen Gene, dass deren Analyse der mRNA-Expression ebenfalls die Vorhersage des Überlebens der Patienten ermöglicht und somit erfindungsgemäß die DNA-Methylierungsanalyse komplementieren kann. Die Patienten wurden anhand eines beispielhaften Schwellenwertes gruppiert. Dieser Schwellenwert ist die Anzahl von mRNA Molekülen des Immun-Checkpoints im Verhältnis zu allen mittels RNA-Seq analysierten mRNA Molekülen (*normalized count*), anhand dessen sich die Patienten besonders gut in eine Gruppe mit guter und eine Gruppe mit schlechter Prognose einteilen ließen. Patienten, die im Tumor eine mRNA-Expression von *CD274* oder *CD276* oberhalb des jeweiligen Schwellenwertes zeigten, wiesen einen signifikant ungünstigeren Verlauf der Erkrankung auf als Patienten, deren Tumoren diese Gene unterhalb des jeweiligen Schwellenwertes exprimierten. Bei den Genen *ADORA2a, BTNL2, C10orf54, CD160, CD27, CD48, PDCD1, TIGIT, TNFRSF18, TNFRSF25* und *TNFRSF4* hatten die Patienten, deren Tumoren das Gen oberhalb des jeweiligen Schwellenwertes exprimierten, dagegen eine günstigere Prognose. An diesem Beispiel konnte demnach demonstriert werden, dass die mRNA-Expressionsanalyse zuverlässig als weiterer Indikator für die erfindungsgemäße Bestimmung der Prognose hinzugezogen werden kann.

**Tabelle 2: Ergebnisse der Analyse des Gesamtüberlebens von 528 Patienten mit Plattenepithelkarzinomen des Kopf- und Halsbereichs. Untersucht wurde der Zusammenhang der mRNA-Expression der genannten immunregulatorischen Gene mit dem Überleben der Patienten. Die Hazard Ratio wurde mittels des anhand eines Schwellenwertes dichotomisierten mRNA-Expressionswertes bestimmt.**

| Gen | Schwellenwert | p-Wert | Hazard Ratio |
|---|---|---|---|
| *ADORA2A* | 87,2 | 0,003 | 0,60 |
| *BTNL2* | 0,45 | 0,049 | 0,58 |
| *C10orf54* | 1015 | 0,096 | 0,73 |
| *CD160* | 11,78 | 0,002 | 0,42 |
| *CD27* | 108,1 | <0,001 | 0,54 |
| *CD274* | 127,0 | 0,015 | 1,50 |
| *CD276* | 3944 | 0,004 | 1,59 |
| *CD48* | 270 | 0,018 | 0,63 |
| *PDCD1* | 48,2 | 0,021 | 0,68 |
| *TIGIT* | 100 | 0,010 | 0,65 |
| *TNFRSF18* | 250 | 0,018 | 0,68 |
| *TNFRSF25* | 250 | 0,041 | 0,69 |
| *TNFRSF4* | 57,32 | <0,001 | 0,48 |

### Beispiel 19: Bestimmung der DNA-Methylierung und mRNA-Expression anhand der Co-Methylierung und Co-Expression immunregulatorischer Gene

Wie bereits in Beispiel 8 gezeigt wurde, sind immunregulatorische Gene oftmals co-exprimiert, das heißt Zellen einer malignen Erkrankung und/oder Immunzellen wie T-Lymphozyten exprimieren nicht nur ein einzelnes immunregulatorisches Gen, sondern es liegt eine gleichzeitige Expression mehrerer immunregulatorischer Gene vor. In diesem Versuch wurde die mRNA-Expression und die DNA-Methylierung verschiedener immunregulatorischer Gene wie in den Beispielen 1 und 6 beschrieben bestimmt. Die mRNA-Expressionsdaten konnten für 520 und die DNA-Methylierungsdaten für 528 Kopf- und Halstumor-Patientenproben generiert werden.

Tabelle 3 zeigt, dass es für die beispielhaft aufgeführten immunregulatorischen Gene eine statistisch signifikante und positive Korrelation der mRNA-Expression von verschiedenen immunregulatorischen Genen in den Tumoren gibt. Somit lässt sich anhand der Bestimmung der mRNA-Expression einzelner oder mehrerer immunregulatorischer Gene auf die mRNA-Expression anderer immunregulatorischer Gene zurückschliessen.

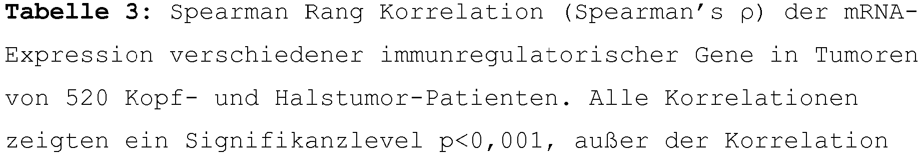

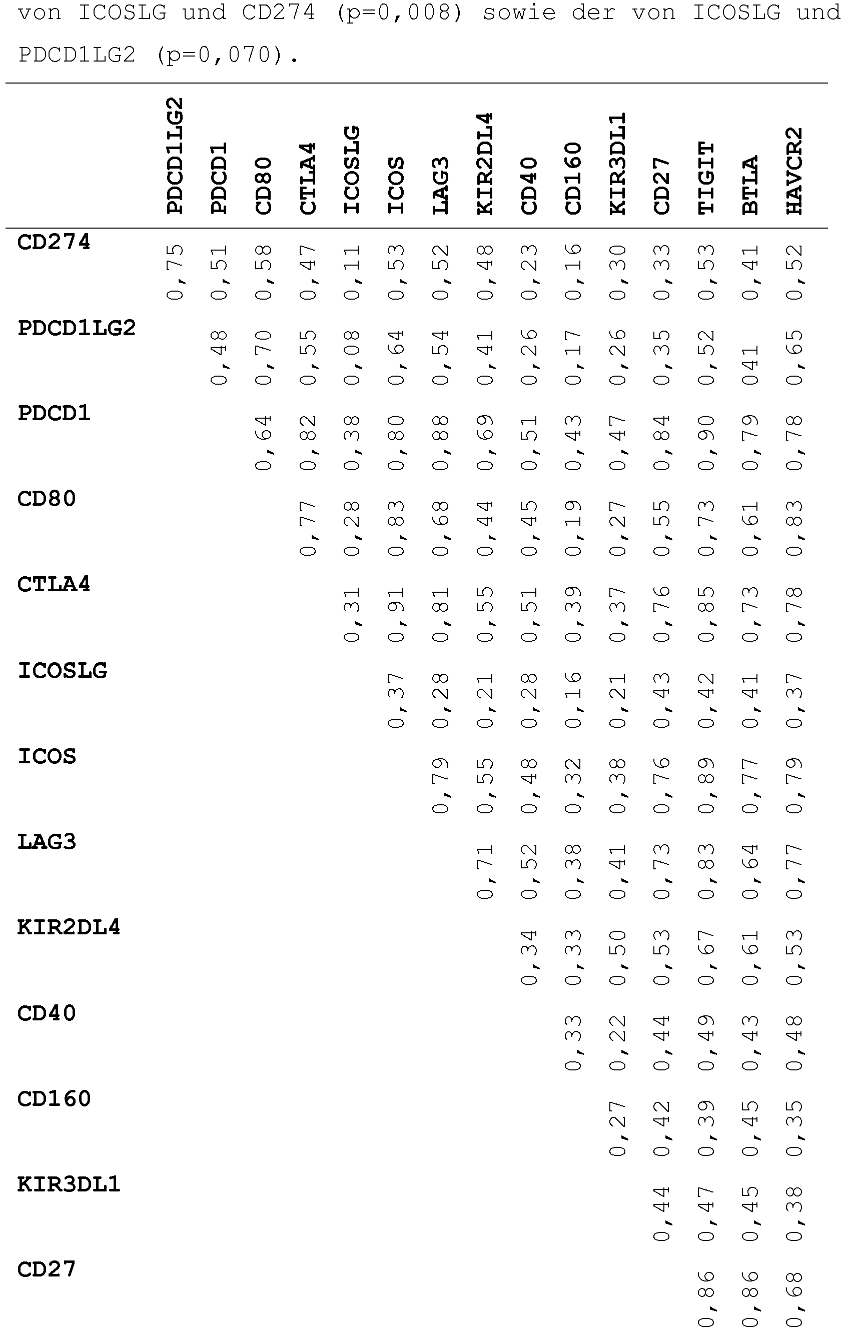

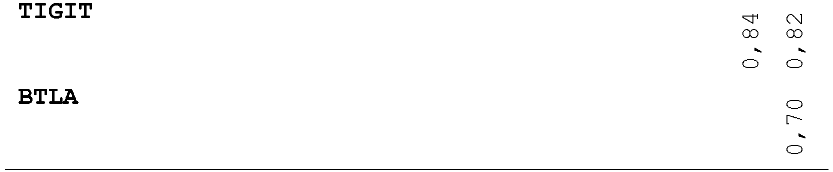

*PDCD1LG2* und *CD274* codieren für Liganden, die beide an den Rezeptor binden, der durch das Gen *PDCD1* codiert wird. Für diese Gene wurde die Korrelation der DNA-Methylierung miteinander untersucht. Für die DNA-Methylierungsanalyse von *PDCD1LG2* wurde das Bead-Paar cg07211259 (SEQ ID NO:31) und für *CD274* die Bead-Paare cg15837913 (SEQ ID NO:8), cg13474877 (SEQ ID NO:11) und cg19724470 (SEQ ID NO:12) verwendet. Die Korrelation wurde mittels einer Spearman Rang Korrelation bestimmt und der Korrelationskoeffizient als Spearman's ρ dargestellt. Die statistische Signifikanz ist als p-Wert wiedergegeben. Das Ergebnis von Bead-Paar cg07211259 korrelierte mit Bead-Paar cg15837913 (ρ=0,33, p<0,001), cg07211259 mit cg13474877 (ρ=0,24, p<0,001), und cg07211259 mit cg19724470 (ρ=0,42, p<0,001). Dieses zeigt, dass beispielsweise über die DNA-Methylierungsanalyse von *PDCD1LG2* aufgrund der festgestellten Korrelation eine Aussage über die Methylierung von *CD274* getroffen werden kann. Auf diese Weise kann zum Beispiel anhand einer DNA-Methylierungsanalyse von *PDCD1LG2* die Prädiktion des Ansprechens auf eine Therapie mit einem Wirkstoff getroffen werden, der den durch *CD274* oder *PDCD1* codierten Immun-Checkpoint inhibiert, beispielsweise wenn die DNA-Methylierungsanalyse von *PDCD1LG2* aufgrund der gezeigten Korrelation stellvertretend eine bestimmte DNA-Methylierung für *CD274* anzeigt, welche das Ansprechen auf die Therapie mit dem betreffenden Wirkstoff wahrscheinlich macht.

## Patentansprüche

1. Ein Verfahren zur Prädiktion des Ansprechens eines Patienten mit einer malignen Erkrankung auf eine Immuntherapie,
wobei die maligne Erkrankung eine Krebserkrankung ist,
wobei eine DNA-Methylierungsanalyse mindestens eines immunregulatorischen Gens von Zellen der malignen Erkrankung und/oder von mit den Zellen der malignen Erkrankung wechselwirkenden T-Lymphozyten durchgeführt wird,
wobei das immunregulatorische Gen für einen Immun-Checkpoint ausgewählt aus B7-Proteinen und deren Rezeptoren kodiert und ausgewählt ist aus der Gruppe bestehend aus den für B7-Proteine und deren Rezeptor kodierenden Genen *CD274, PDCD1LG2* und *PDCD1,* den für das B7-Protein und dessen Rezeptor kodierenden Genen *CD80* und *CTLA4,* dem für den Rezeptor eines B7-Proteins kodierenden Gen *ICOS* und den für die B7-Proteine kodierenden Genen *CD276, C10orf54* und *HHLA2,* sowie beliebigen Kombinationen davon,
wobei die Immuntherapie mindestens einen Wirkstoff umfasst, welcher ein Immun-Checkpoint-Inhibitor ist, der dazu ausgelegt ist, die immunregulatorische Wirkung des Immun-Checkpoints zu verändern,
und das Ergebnis der DNA-Methylierungsanalyse mit einem Referenzwert verglichen wird, um das Ansprechen des Patienten auf die Immuntherapie zu bestimmen.

2. Das Verfahren nach Anspruch 1, wobei die maligne Erkrankung ein Karzinom, insbesondere ein Plattenepithelkarzinom oder ein Adenokarzinom, ein Melanom, eine Leukämie, ein Gliom, ein Sarkom und/oder ein Lymphom umfasst.

3. Das Verfahren nach Anspruch 1, wobei der Immun-Checkpoint CD274 und/oder PDCD1LG2 umfasst und der Wirkstoff dazu ausgelegt ist, durch Wechselwirkung mit PDCD1 die immunregulatorische Wirkung von CD274 und/oder PDCD1LG2 zu verändern, inbesondere zu inhibieren.

4. Das Verfahren nach einem der Ansprüche 1 bis 3, wobei die DNA-Methylierungsanalyse ein Bestimmen von Anwesenheit, Abwesenheit oder Ausmaß einer Methylierung von mindestens einem in dem immunregulatorischen Gen enthaltenen CpG-Dinukleotid umfasst.

5. Das Verfahren nach Anspruch 4, wobei die Methylierung von mindestens je einem CpG-Dinukleotid von mindestens zwei verschiedenen immunregulatorischen Genen bestimmt wird.

6. Das Verfahren nach Anspruch 4 oder 5, wobei die DNA-Methylierungsanalyse unter Bedingungen durchgeführt wird, welche eine quantitative Bestimmung der Methylierung des mindestens einen CpG-Dinukleotids erlauben.

7. Das Verfahren nach einem der Ansprüche 1 bis 6, wobei zusätzlich eine mRNA-Expressionsanalyse des immunregulatorischen Gens durchgeführt wird.

8. Verwendung einer DNA-Methylierungsanalyse mindestens eines immunregulatorischen Gens von Zellen einer malignen Erkrankung eines Patienten und/oder von mit den Zellen der malignen Erkrankung wechselwirkenden T-Lymphozyten,
wobei die maligne Erkrankung eine Krebserkrankung ist,
wobei das immunregulatorische Gen für einen Immun-Checkpoint ausgewählt aus B7-Proteinen und deren Rezeptoren kodiert und ausgewählt ist aus der Gruppe bestehend aus den für B7-Proteine und deren Rezeptor kodierenden Genen *CD274, PDCD1LG2* und *PDCD1,* den für das B7-Protein und dessen Rezeptor kodierenden Genen *CD80* und *CTLA4,* dem für den Rezeptor eines B7-Proteins kodierenden Gen *ICOS* und den für die B7-Proteine kodierenden Genen *CD276, C10orf54* und *HHLA2,* sowie beliebigen Kombinationen davon,
zur Prädiktion des Ansprechens des Patienten auf eine Immuntherapie und/oder zur individualisierten Auswahl eines Wirkstoffs zur immuntherapeutischen Behandlung des Patienten,
wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern,
wobei der Wirkstoff ausgewählt wird, wenn die DNA-Methylierungsanalyse eine Expression des Immun-Checkpoints in den Zellen der malignen Erkrankung und/oder in den T-Lymphozyten anzeigt.

9. Verwendung von Anwesenheit, Abwesenheit oder Ausmaß einer Methylierung eines immunregulatorischen Gens von Zellen einer malignen Erkrankung und/oder von mit den Zellen der malignen Erkrankung wechselwirkenden T-Lymphozyten,
wobei die maligne Erkrankung eine Krebserkrankung ist,
wobei das immunregulatorische Gen für einen Immun-Checkpoint ausgewählt aus B7-Proteinen und deren Rezeptoren kodiert und ausgewählt ist aus der Gruppe bestehend aus den für B7-Proteine und deren Rezeptor kodierenden Genen *CD274, PDCD1LG2* und *PDCD1,* den für das B7-Protein und dessen Rezeptor kodierenden Genen *CD80* und *CTLA4,* dem für den Rezeptor eines B7-Proteins kodierenden Gen *ICOS* und den für die B7-Proteine kodierenden Genen *CD276, C10orf54* und *HHLA2,* sowie beliebigen Kombinationen davon,
als prädiktiver Biomarker zur Vorhersage des Ansprechens der malignen Erkrankung auf eine Immuntherapie und/oder als Biomarker zur individualisierten Auswahl eines Wirkstoffs zur immuntherapeutischen Behandlung eines Patienten mit der malignen Erkrankung,
wobei die Immuntherapie und/oder die immuntherapeutische Behandlung mindestens einen Wirkstoff umfasst, der ein Immun-Checkpoint-Inhibitor ist, welcher dazu ausgelegt ist, die immunregulatorische Wirkung des Immun-Checkpoints zu verändern,
wobei der Wirkstoff ausgewählt wird, wenn die Methylierung eine Expression des Immun-Checkpoints in den Zellen der malignen Erkrankung und/oder in den T-Lymphozyten anzeigt.

10. Eine Verwendung eines Kits zur Durchführung des Verfahrens nach einem der Ansprüche 1 bis 7, das Kit umfassend
mindestens ein Oligonukleotid-Paar für die DNA-Methylierungsanalyse, wobei das Oligonukleotid-Paar dazu ausgelegt ist, an eine Sequenz des immunregulatorischen Gens in DNA von den Zellen der malignen Erkrankung und/oder von den T-Lymphozyten zu hybridisieren, nachdem in der DNA enthaltene Cytosine in Uracil oder eine andere Base mit einem von Cytosin unterscheidbaren Basenpaarungsverhalten und/oder Molekulargewicht umgewandelt wurden, um die Sequenz zu amplifizieren und/oder zu detektieren.

## Claims

1. A method for predicting response of a patient with a malignant disease to immunotherapy,
wherein said malignant disease is a cancer disease,
wherein a DNA methylation analysis of at least one immunoregulatory gene of cells of the malignant disease and/or of T lymphocytes interacting with said cells of the malignant disease is performed,
wherein said immunoregulatory gene encodes an immune checkpoint selected from B7 proteins and their receptors and selected from the group consisting of the B7 proteins and their receptor encoding genes *CD274, PDCD1LG2* and *PDCD1,* the B7 protein and its receptor encoding genes *CD80* and *CTLA4,* the gene *ICOS* encoding the receptor of a B7 protein and the B7 proteins encoding genes *CD276, C10orf54* and *HHLA2,* and any combinations thereof,
wherein said immunotherapy comprises at least one pharmaceutical compound which is an immune checkpoint inhibitor intended to alter the immunoregulatory effect of said immune checkpoint,
and the result of the DNA methylation analysis is compared to a reference value to determine the patient's response to the immunotherapy.

2. The method according to claim 1, wherein the malignant disease comprises a carcinoma, in particular a squamous cell carcinoma or an adenocarcinoma, a melanoma, a leukemia, a glioma, a sarcoma and/or a lymphoma.

3. The method according to claim 1, wherein said immune checkpoint comprises CD274 and/or PDCD1LG2 and said pharmaceutical compound is designed to alter, in particular inhibit, the immunoregulatory effect of CD274 and/or PDCD1LG2 by interaction with PDCD1.

4. The method according to any one of claims 1 to 3, wherein the DNA methylation analysis comprises determining the presence, absence or level of methylation of at least one CpG dinucleotide contained in said immunoregulatory gene.

5. The method according to claim 4, wherein the methylation of at least one CpG dinucleotide from each of at least two different immunoregulatory genes is determined.

6. The method according to claim 4 or 5, wherein the DNA methylation analysis is performed under conditions which allow for a quantitative determination of said methylation of the at least one CpG dinucleotide.

7. The method according to one of claims 1 to 6, wherein an additional mRNA expression analysis of said immunoregulatory gene is performed.

8. Use of a DNA methylation analysis of at least one immunoregulatory gene of cells of a malignant disease of a patient and/or of T lymphocytes interacting with the cells of the malignant disease,
wherein said malignant disease is a cancer disease,
wherein said immunoregulatory gene encodes an immune checkpoint consisting of B7 proteins and their receptors and is selected from the group consisting of the B7 proteins and their receptor encoding genes *CD274, PDCD1LG2* and *PDCD1,* the B7 protein and its receptor encoding genes *CD80* and *CTLA4,* the gene *ICOS* encoding the receptor of a B7 protein and the B7 proteins encoding genes *CD276, C10orf54* and *HHLA2,* and any combinations thereof,
for predicting the patient's response to an immunotherapy and/or for the individualised selection of a pharmaceutical compound for immunotherapeutic treatment of the patient,
wherein said immunotherapy and/or said immunotherapeutic treatment comprises at least one pharmaceutical compound which is an immune checkpoint inhibitor designed to alter an immunoregulatory effect of said immune checkpoint,
wherein said pharmaceutical compound is selected when said DNA methylation analysis indicates expression of said immune checkpoint in said cells of the malignant disease and/or in said T lymphocytes.

9. Use of presence, absence or level of methylation of an immunoregulatory gene of cells of a malignant disease and/or of T lymphocytes interacting with said cells of the malignant disease,
wherein said malignant disease is a cancer disease,
wherein said immunoregulatory gene encodes an immune checkpoint consisting of B7 proteins and their receptors and is selected from the group consisting of the B7 proteins and their receptor encoding genes *CD274, PDCD1LG2* and *PDCD1,* the B7 protein and its receptor encoding genes *CD80* and *CTLA4,* the gene *ICOS* encoding the receptor of a B7 protein and the B7 proteins encoding genes *CD276, C10orf54* and *HHLA2,* and any combinations thereof,
as predictive biomarker for predicting response of said malignant disease to an immunotherapy and/or for individualized selection of a pharmaceutical compound for immunotherapeutic treatment of a patient afflicted with said malignant disease,
wherein said immunotherapy and/or said immunotherapeutic treatment comprises at least one pharmaceutical compound which is an immune checkpoint inhibitor designed to alter an immunoregulatory effect of said immune checkpoint,
wherein said pharmaceutical compound is selected when said DNA methylation analysis indicates expression of said immune checkpoint in said cells of the malignant disease and/or in said T lymphocytes.

10. Use of a kit for carrying out the method according to any one of claims 1 to 7, the kit comprising
at least one pair of oligonucleotides for said DNA methylation analysis, said oligonucleotide pair being designed to hybridize to a sequence of the immunoregulatory gene in DNA from said cells of the malignant disease and/or from said T lymphocytes after cytosines contained in said DNA have been converted into uracil or another base having a base pairing behaviour and/or molecular weight distinguishable from that of cytosine in order to amplify and/or detect said sequence.

## Revendications

1. Procédé permettant de prédire la réponse d'un patient atteint d'une maladie maligne à une immunothérapie,
dans lequel la maladie maligne est un cancer,
dans lequel une analyse de méthylation de l'ADN d'au moins un gène immunorégulateur de cellules de la maladie maligne et/ou de lymphocytes T interagissant avec les cellules de la maladie maligne est réalisée,
dans lequel le gène immunorégulateur est choisi pour un point de contrôle immunitaire et est constitué de protéines B7 et de leurs récepteurs, choisis dans le groupe constitué des gènes *CD274, PDCD1LG2* et *PDCD1* codant pour les protéines B7 et leurs récepteurs, les gènes *CD80* et *CTLA4* codant pour la protéine B7 et son récepteur, le gène *ICOS* codant pour le récepteur d'une protéine B7 et les gènes *CD276, C10orf54* et *HHLA2* codant pour les protéines B7, ainsi que des combinaisons quelconques de ceux-ci,
dans lequel l'immunothérapie comprend au moins un principe actif qui est un inhibiteur de point de contrôle immunitaire conçu pour modifier l'action immunorégulatrice du point de contrôle immunitaire,
et le résultat de l'analyse de méthylation de l'ADN est comparé avec une valeur de référence afin de déterminer la réponse du patient à l'immunothérapie.

2. Procédé selon la revendication 1, dans lequel la maladie maligne comprend un carcinome, en particulier un carcinome épidermoïde ou un adénocarcinome, un mélanome, une leucémie, un gliome, un sarcome et/ou un lymphome.

3. Procédé selon la revendication 1, dans lequel le point de contrôle immunitaire comprend CD274 et/ou PDCD1LG2 et le principe actif est conçu pour modifier, en particulier pour inhiber, l'action immunorégulatrice de CD274 et/ou de PDCD1LG2 par interaction avec PDCD1.

4. Procédé selon l'une des revendications 1 à 3, dans lequel l'analyse de méthylation de l'ADN comprend une détermination de la présence, de l'absence ou du degré d'une méthylation d'au moins un dinucléotide CpG contenu dans le gène immunorégulateur.

5. Procédé selon la revendication 4, dans lequel la méthylation d'au moins respectivement un dinucléotide CpG d'au moins deux gènes immunorégulateurs différents est déterminée.

6. Procédé selon la revendication 4 ou 5, dans lequel l'analyse de méthylation de l'ADN est réalisée dans des conditions qui permettent une détermination quantitative de la méthylation de l'au moins un dinucléotide CpG.

7. Procédé selon l'une des revendications 1 à 6, dans lequel une analyse d'expression de l'ARNm du gène immunorégulateur est en outre réalisée.

8. Utilisation d'une analyse de méthylation de l'ADN d'au moins un gène immunorégulateur de cellules d'une maladie maligne d'un patient et/ou de lymphocytes T interagissant avec les cellules de la maladie maligne,
dans lequel la maladie maligne est un cancer,
dans lequel le gène immunorégulateur pour un point de contrôle immunitaire est choisi parmi les protéines B7 et leurs récepteurs, choisis dans le groupe constitué des gènes *CD274, PDCD1LG2* et *PDCD1* codant pour les protéines B7 et leurs récepteurs, les gènes *CD80* et *CTLA4* codant pour la protéine B7 et son récepteur, le gène *ICOS* codant pour le récepteur d'une protéine B7 et les gènes *CD276, C10orf54* et *HHLA2* codant pour les protéines B7, ainsi que des combinaisons quelconques de ceux-ci,
pour la prédiction de la réponse du patient à une immunothérapie et/ou pour la sélection individualisée d'un principe actif pour le traitement immunothérapeutique du patient,
dans lequel l'immunothérapie et/ou le traitement immunothérapeutique comprennent au moins un principe actif qui est un inhibiteur de point de contrôle immunitaire qui est conçu pour modifier l'action immunorégulatrice du point de contrôle immunitaire,
dans lequel le principe actif est choisi lorsque l'analyse de méthylation de l'ADN indique une expression du point de contrôle immunitaire dans les cellules de la maladie maligne et/ou dans les lymphocytes T.

9. Utilisation de la présence, de l'absence ou du degré d'une méthylation d'un gène immunorégulateur de cellules d'une maladie maligne et/ou de lymphocytes T interagissant avec les cellules de la maladie maligne,
dans lequel la maladie maligne est un cancer, dans lequel le gène immunorégulateur pour un point de contrôle immunitaire est choisi parmi les protéines B7 et leurs récepteurs, dans le groupe constitué des gènes *CD274, PDCD1LG2* et *PDCD1* codant pour les protéines B7 et leurs récepteurs, les gènes *CD80* et *CTLA4* codant pour la protéine B7 et son récepteur, le gène *ICOS* codant pour le récepteur d'une protéine B7 et les gènes *CD276, C10orf54* et *HHLA2* codant pour les protéines B7, ainsi que des combinaisons quelconques de ceux-ci, comme biomarqueur prédictif pour prédire la réponse de la maladie maligne à une immunothérapie et/ou comme biomarqueur pour la sélection individualisée d'un principe actif pour le traitement immunothérapeutique d'un patient atteint de la maladie maligne,
dans lequel l'immunothérapie et/ou le traitement immunothérapeutique comprennent au moins un principe actif qui est un inhibiteur de point de contrôle immunitaire qui est conçu pour modifier l'action immunorégulatrice du point de contrôle immunitaire,
dans lequel le principe actif est choisi lorsque la méthylation indique une expression du point de contrôle immunitaire dans les cellules de la maladie maligne et/ou dans les lymphocytes T.

10. Utilisation d'un kit pour la réalisation du procédé selon l'une des revendications 1 à 7, le kit comprenant
au moins une paire d'oligonucléotides pour l'analyse de méthylation de l'ADN, dans lequel la paire d'oligonucléotides est conçue pour s'hybrider à une séquence du gène immunorégulateur dans l'ADN des cellules de la maladie maligne et/ou des lymphocytes T, après que la cytosine contenue dans l'ADN a été convertie en uracile ou en une autre base comportant un comportement d'appariement de bases et/ou un poids moléculaire différents de ceux de la cytosine, afin d'amplifier et/ou de détecter la séquence.
